# EUROPEAN PATENT APPLICATION

(11) **EP 4 434 982 A1**
(43) Date of publication of application: **25.09.2024**
(21) Application number: 22912011.8
(22) Date of filing: 23.12.2022
(51) Int. Cl.: C07D 403/04, A61K 31/517, A61K 31/506, A61K 31/551, A61P 3/00, A61P 1/16, C07D 401/14, C07D 403/14, C07D 401/04, C07D 239/38

(54) **(S)-2-(2-METHYLAZETIDIN-1-YL)PYRIMIDINE DERIVATIVE AND PHARMACEUTICAL COMPOSITION COMPRISING SAME**

(30) Priority: 24.12.2021 KR 20210187602; 24.12.2021 KR 20210187603
(71) Applicant: LG Chem, Ltd., Yeongdeungpo-gu Seoul 07336 (KR)
(72) Inventor: LEE, Sung Bae, Daejeon 34122 (KR); KIM, Chang Hoon, Daejeon 34122 (KR); HUR, Jeong Mi, Daejeon 34122 (KR); MOON, Hee Jeong, Daejeon 34122 (KR)
(74) Representative: Hoffmann Eitle
(86) International application number: PCT/KR2022/021153
(87) International publication number: WO 2023/121378

(57) **Abstract**

The present invention relates to a novel (S)-2-(2-methylazetidin-1-yl)pyrimidine derivative compound having ketohexokinase (KHK) inhibitory activity, a pharmaceutically acceptable salt thereof or an isomer thereof, and a pharmaceutical composition comprising same as active ingredients, and provides a compound of chemical formula 1, a pharmaceutically acceptable salt or isomer thereof, and a pharmaceutical composition containing same as active ingredients.

## Description

### TECHNICAL FIELD

### [Cross-reference to Related Applications]

The present application claims the benefit of priority based on Korean Patent Application No. 10-2021-0187602, filed on December 24, 2021, and Korean Patent Application No. 10-2021-0187603, filed on December 24, 2021, the entire contents of which are incorporated herein by reference.

### [Technical Field]

The present invention relates to a novel (S)-2-(2-methylazetidin-1-yl)pyrimidine derivative compound having ketohexokinase (KHK) inhibitory activity, a pharmaceutically acceptable salt thereof, or an isomer thereof, and a pharmaceutical composition comprising same as an active ingredient.

### BACKGROUND ART

The causes of disease of nonalcoholic steatohepatitis that is one of metabolic diseases are known to be largely divided into fatty liver production, increased inflammation, apoptosis, or the like. The nonalcoholic steatohepatitis as a chronic disease may progress to hepatic fibrosis, hepatic cirrhosis, or hepatocellular cancer.

Meanwhile, ketohexokinase (KHK) is an enzyme involved in fructose metabolism and is a type of kinase responsible for the phosphorylation of fructose during fructose metabolism. Different from glucose metabolism, the fructose metabolism does not receive energy dependent inhibition and induces the rapid accumulation of fat in the liver to influence the production of a fat liver. Accordingly, if the ketohexokinase (KHK) is inhibited, effects of suppressing the production of a fatty liver which is a factor of inducing steatohepatitis, could be expected.

In animal experiment models, it was observed that overall indicators on metabolic diseases were improved in case of suppressing ketohexokinase (KHK) activity, and no major side effects are expected due to ketohexokinase (KHK) inhibition, because no difference was found in the phenotype of a ketohexokinase (KHK) knockout mouse from a normal mouse.

However, in the way that ketohexokinase (KHK) is responsible for phosphorylation using ATP, if ketohexokinase (KHK) is inhibited, there are possibilities of inhibiting other types of kinases, and it is true that there are many concerns in terms of safety as a cure for chronic diseases. Accordingly, it is important that a ketohexokinase (KHK) inhibitor is required to have selectivity with other kinases.

Study on a ketohexokinase (KHK) inhibitor is recently actively conducted, and the development of therapies effectively used for preventing or treating metabolic diseases such as diabetes, complications of diabetes, obesity, nonalcoholic steatohepatitis, and fatty hepatitis, is still needed.

### [Prior Art Document]

### [Patent Document]

(Patent Document 1) US Registration Patent No. 9,809,579

### DISCLOSURE OF THE INVENTION

### TECHNICAL PROBLEM

The present invention is made to solve the above-described problems of the conventional technique and an object is to provide a novel compound having inhibition activity on ketohexokinase (KHK), a pharmaceutically acceptable salt thereof or an isomer thereof.

In addition, another object of the present invention is to provide a pharmaceutical composition for inhibiting ketohexokinase, containing the compound as an active ingredient.

In addition, another object of the present invention is to provide a pharmaceutical composition for preventing or treating metabolic diseases, containing the compound as an active ingredient.

Also, another object of the present invention is to provide a method for treating metabolic diseases including nonalcoholic steatohepatitis in mammals, comprising a step of administering the compound.

### TECHNICAL SOLUTION

In order to solve the tasks, the present invention provides a compound of Formula 1, a pharmaceutically acceptable salt thereof, or an isomer thereof.

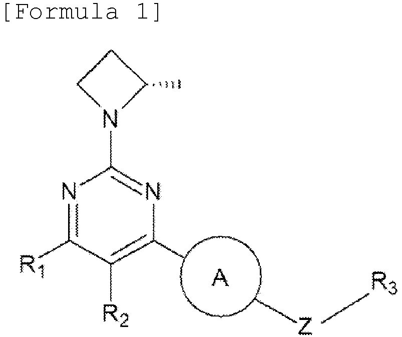

In Formula 1,
A is a C₃-C₈ heterocycloalkyl group, unsubstituted or substituted with a C₁-C₃ alkyl group or a carbonyl group, or 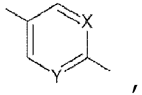 X and Y are each independently N or C-Rₐ, Rₐ is hydrogen or a halogen group, Z is a direct linkage, a C₁-C₃ alkylene group, -O-, or -C(O)-, R₁ and R₂ are each independently hydrogen, a halogen group, or a C₁-C₃ alkyl group, unsubstituted or substituted with 1 to 5 halogen groups, or R₁ and R₂ are connected with each other to form a C₃-C₆ aliphatic cyclic group, unsubstituted or substituted with 1 to 3 halogen groups, or a C₃-C₆ heterocycloalkyl group, unsubstituted or substituted with 1 to 3 halogen groups, R₃ is -(CR₄R₅)ₙ-B-R₆, R₄ and R₅ are each independently hydrogen or a C₁-C₃ alkyl group, n is an integer of 0 to 3, B is a direct linkage, -C(O)-, or -S(O)₂-, and R₆ is a hydroxyl group, or a C₃-C₇ heterocycloalkyl group containing N, unsubstituted or substituted with a C₁-C₃ alkyl group, a carbonyl group or a C₁-C₃ alkylcarbonyl group.

In addition, the present invention provides a pharmaceutical composition for inhibiting ketohexokinase (KHK), comprising the compound, the pharmaceutically acceptable salt thereof, or the isomer thereof, as an active ingredient.

In addition, the present invention provides a pharmaceutical composition for preventing or treating metabolic diseases, comprising the compound, the pharmaceutically acceptable salt thereof, or the isomer thereof, as an active ingredient.

In addition, the present invention provides a method for preparing a pharmaceutical composition for preventing or treating diabetes, complications of diabetes, obesity, nonalcoholic steatohepatitis, or fatty hepatitis, comprising a step of mixing the compound, the pharmaceutically acceptable salt thereof, or the isomer thereof, as an active ingredient with a pharmaceutically acceptable carrier.

In addition, the present invention provides a method for treating a metabolic disease comprising nonalcoholic steatohepatitis in a mammal, comprising administering the compound, the pharmaceutically acceptable salt thereof, or the isomer thereof.
(1) The present invention provides the compound of Formula 1, the pharmaceutically acceptable salt thereof, or the isomer thereof:
(2) The present invention provides the compound, the pharmaceutically acceptable salt thereof, or the isomer thereof according to (1), wherein, in Formula 1, in case where A is the heterocycloalkyl group, A is a substituent represented by the following Formula 1a or Formula 1b:

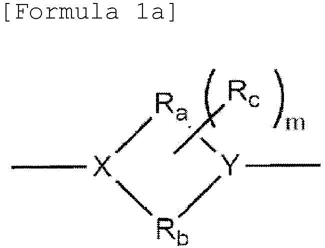

   in Formula 1a,
   X and Y are each independently CH or N,
   Rₐ and R_{b} are each independently a C₁-C₃ alkylene group,
   R_{c} is a C₁-C₃ alkyl group or a carbonyl group, and
   m is 0 or 1,

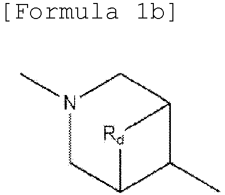

      in Formula 1b,
      R_{d} is a direct linkage or CH₂.
(3) The present invention provides the compound, the pharmaceutically acceptable salt thereof, or the isomer thereof according to (1), wherein, in case where A is the 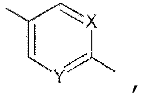 in Formula 1,
   X and Y are each independently N or C-Rₐ, Rₐ is hydrogen or a halogen group, Z is a C₁-C₃ alkylene group or - O-, R₁ and R₂ are connected with each other to form a C₃-C₆ aliphatic cyclic group, unsubstituted or substituted with 1 to 3 halogen group, R₃ is -(CR₄R₅)ₙ-A-R₆, R₄ and R₅ are hydrogen, n is an integer of 0 to 3, A is -C(O)-, and R₆ is a hydroxyl group, or a C₄-C₆ heterocycloalkyl group containing N, unsubstituted or substituted with a C₁-C₃ alkyl group.
(4) The present invention provides the compound, the pharmaceutically acceptable salt thereof, or the isomer thereof according to (1) or (3), wherein, in case where A is the 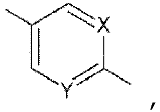 in Formula 1,
   X is N, Y is C-Rₐ, where Rₐ is hydrogen, Z is a C₁-C₃ alkylene group or -O-, R₁ is a C₁-C₃ alkyl group substituted with 1 to 3 halogen groups, R₂ is hydrogen or a C₁-C₃ alkyl group, R₃ is -(CR₄R₅)ₙ-A-R₆, R₄ and R₅ are hydrogen, n is an integer of 0 to 3, A is -C(O)-, and R₆ is a C₄-C₆ heterocycloalkyl group containing N, unsubstituted or substituted with a C₁-C₃ alkyl group.
(5) The present invention provides the compound, the pharmaceutically acceptable salt thereof, or the isomer thereof according to any one among (1) to (4), wherein the halogen group is F or Cl.
(6) The present invention provides the compound, the pharmaceutically acceptable salt thereof, or the isomer thereof according to any one among (1) to (5), wherein the compound of Formula 1 is one or more selected from the group consisting of the following group:
   (S)-2-((1-(2-(2-methylazetidin-2-yl)-6-(trifluoromethyl)pyrimidin-4-yl)azetidin-3-yl)oxy)-1-(piperazin-1-yl)ethan-1-one;
   (S)-2-((1-(5-chloro-2-(2-methylazetidin-1-yl)-6-(trifluoromethyl)pyrimidin-4-yl)azetidin-3-yl)oxy)-1-(piperazin-1-yl)ethyn-1-one;
   (S)-2-((1-(7,7-difluoro-2-(2-(methylazetidin-1-yl)-6,7-dihydro-5H-cyclopenta[d]pyrimidin-4-yl)azetidin-3-yl)oxy)-1-(piperazin-1-yl)ethan-1-one;
   (S)-2-((1-(8,8-difluoro-2-(2-methylazetidin-1-yl)-5,6,7,8-tetrahydroquinazolin-4-yl)azetidin-3-yl)oxy)-1-(piperazin-1-yl)ethan-1-one;
   2-(((2S,3R)-1-(7,7-difluoro-2-((S)-2-methylazetidin-1-yl)-6,7-dihydro-5H-cyclopenta[d]pyrimidin-4-yl)-2-methylazetidin-3-yl)oxy)-1-(piperazin-1-yl)ethan-1-one;
   2-(((S)-1-(2-((S)-2-methylazetidin-1-yl)-6-(trifluoromethyl)pyrimidin-4-yl)pyrrolidin-3-yl)oxy)-1-(piperazin-1-yl)ethan-1-one;
   2-(((S)-1-(7,7-difluoro-2-((S)-2-methylazetidin-1-yl)-6,7-dihydro-5H-cyclopenta[d]pyrimidin-4-yl)pyrrolidin-3-yl)oxy)-1-(piperazin-1-yl)ethan-1one;
   2-(((R)-1-(7,7-difluoro-2-((S)-2-methylazetidin-1-yl)-6,7-dihydro-5H-cyclopenta[d]pyrimidin-4-yl)pyrrolidin-3-yl)oxy)-1-(piperazin-1-yl)ethan-1one;
   2-((R)-1-(7,7-difluoro-2-((S)-2-methylazetidin-1-yl)-6,7-dihydro-5H-cyclopenta[d]pyrimidin-4-yl)pyrrolidin-3-yl)-1-(piperazin-1-yl)ethan-1one;
   (S)-2-((1-(7,7-difluoro-2-(2-methylazetidin-1-yl)-6,7-dihydro-5H-cyclopenta[d]pyrimidin-4-yl)piperizin-4-yl)oxy)-1-(piperazin-1-yl)ethan-1-one;
   (S)-2-(4-(2-(2-methylazetidin-1-yl)-6-(trifluoromethyl)pyrimidin-4-yl)piperazin-1-yl)-1-(piperazin-1-yl)ethan-1-one;
   (S)-2-(4-(5-chloro-2-(2-methylazetidin-1-yl)-6-(trifluoromethyl)pyrimidin-4-yl)piperazin-1-yl)-1-(piperazin-1-yl)ethan-1-one;
   (S)-2-(4-(2-(2-methylazetidin-1-yl)-6-(trifluoromethyl)pyrimidin-4-yl)-1-(2-oxo-2-(piperazin-1-yl)ethyl)piperazin-2-one;
   (S)-2-(4-(7,7-difluoro-2-(2-methylazetidin-1-yl)-6,7-dihydro-5H-cyclopenta[d]pyrimidin-4-yl)piperazin-1-yl)-1-(piperazin-1-yl)ethan-1-one;
   (S)-2-(4-(7,7-difluoro-2-(2-methylazetidin-1-yl)-6,7-dihydro-5H-cyclopenta[d]pyrimidin-4-yl)piperazin-1-yl)-1-morpholinoethan-1-one;
   (S)-1-(4-(7,7-difluoro-2-(2-methylazetidin-1-yl)-6,7-dihydro-5H-cyclopenta[d]pyrimidin-4-yl)piperazin-1-yl)-3-(piperazin-1-yl)propan-1,3-dione;
   (S)-(4-(7,7-difluoro-2-(2-methylazetidin-1-yl)-6,7-dihydro-5H-cyclopenta[d]pyrimidin-4-yl)-1-(2-oxo-2-(piperazin-1-yl)ethyl)piperazin-2-one;
   (S)-2(4-(7,7-difluoro-2-(2-methylazetidin-1-yl)-6,7-dihydro-5H-cyclopenta[d]-pyrimidin-4-yl)piperidin-1-yl)-1-(piperazin-1-yl)ethan-1-one;
   2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)-1-(piperazin-1-yl)ethan-1-one;
   2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)-1-(4-methylpiperazin-1-yl)ethan-1-one;
   4-(2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)acetyl)piperazin-2-one;
   2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)-1-(piperazin-1-yl)ethan-1-one;
   1-(4-acetylpiperazin-1-yl)-2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)ethan-1-one;
   2-((1R,5S,6S)-3-(8,8-difluoro-2-((S)-2-methylazetidin-1-yl)-5,6,7,8-tetrahydroquinazol-4-yl)-3-azabicyclo[3.1.1]heptan-6-yl)-1-(piperazin-1-yl)ethan-1-one;
   2-((1R,5S,6S)-3-(8,8-difluoro-2-((S)-2-methylazetidin-1-yl)-5,8-dihydro-6H-pyrazol[3,4-d]pyrimidin-4-yl)-3-azabicyclo[3.1.1]heptan-6-yl)-1-(piperazin-1-yl)ethan-1-one;
   7,7-difluoro-2-((S)-2-methylazetidin-1-yl)-4-((1R,5S,6S)-6-((piperazin-1-ylsulfonyl)methyl)-3-azabicyclo[3.1.0]hexan-3-yl)-6,7-dihydro-5H-cyclopenta[d]pyrimidine;
   (S)-2-((1-(5-methyl-2-(2-methylazetidin-1-yl)-6-(trifluoro)pyrimidin-4-yl)azetidin-3-yl)oxy)-1-(piperazin-1-yl)ethan-1-one;
   (S)-2-((1-(2-(2-methylazetidin-1-yl)-6-(trifluoromethyl)pyrimidin-4-yl)piperidin-4-yl)oxy)-1-(piperazin-1-yl)ethan-1-one;
   (S)-2-((1-(5-methyl-2-(2-methylazetidin-1-yl)-6-(trifluoro)pyrimidin-4-yl)piperidin-4-yl)oxy)-1-(piperazin-1-yl)ethan-1-one;
   (S)-2-(4-(7,7-difluoro-2-(2-methylazetidin-1-yl)-6,7-dihydro-5H-cyclopenta[d]-pyrimidin-4-yl)phenyl)acetic acid;
   (S)-2-(4-(7,7-difluoro-2-(2-methylazetidin-1-yl)-6,7-dihydro-5H-cyclopenta[d]-pyrimidin-4-yl)phenyl)-1-(piperazin-1-yl)ethan-1-one;
   (S)-2-(5-(2-(2-methylazetidin-1-yl)-6-(trifluoromethyl)pyrimidin-4-yl)pyridin-2-yl)-1-(piperazin-1-yl)ethan-1-one;
   (S)-2-(4-(7,7-difluoro-2-(2-methylazetidin-1-yl)-6,7-dihydro-5H-cyclopenta[d]pyrimidin-4-yl)phenoxy)-1-(piperazin-1-yl)ethan-1-one;
   (S)-2-(4-(7,7-difluoro-2-(2-methylazetidin-1-yl)-6,7-dihydro-5H-cyclopenta[d]pyrimidin-4-yl)-2-fluorophenoxy)-1-(piperazin-1-yl)ethan-1-one;
   (S)-2-(4-(7,7-difluoro-2-(2-methylazetidin-1-yl)-6,7-dihydro-5H-cyclopenta[d]pyrimidin-4-yl)-2,6-difluorophenoxy)-1-(piperazin-1-yl)ethan-1-one;
   (S)-2-(2-chloro-4-(7,7-difluoro-2-(2-methylazetidin-1-yl)-6,7-dihydro-5H-cyclopenta[d]pyrimidin-4-yl)phenoxy)-1-(piperazin-1-yl)ethan-1-one;
   (S)-2-(2-chloro-4-(7,7-difluoro-2-(2-methylazetidin-1-yl)-6,7-dihydro-5H-cyclopenta[d]pyrimidin-4-yl)phenoxy)-1-(1,4-diazepan-1-yl)ethan-1-one;
   (S)-2-((5-(7,7-difluoro-2-(2-methylazetidin-1-yl)-6,7-dihydro-5H-cyclopenta[d]pyrimidin-4-yl)pyridin-2-yl)oxy)-1-(piperazin-1-yl)ethan-1-one;
   (S)-2-((3-chloro-5-(7,7-difluoro-2-(2-methylazetidin-1-yl)-6,7-dihydro-5H-cyclopenta[d]pyrimidin-4-yl)pyridin-2-yl)oxy)-1-(piperazin-1-yl)ethan-1-one;
   (S)-2-((5-(2-(2-methylazetidin-1-yl)-6-(trifluoromethyl)pyrimidin-4-yl)pyridin-2-yl)oxy)-1-(piperazin-1-yl)ethan-1-one;
   (S)-2-((5-(5-methyl-2-(2-methylazetidin-1-yl)-6-(trifluoromethyl)pyrimidin-4-yl)pyridin-2-yl)oxy)-1-(piperazin-1-yl)ethan-1-one.
(7) The present invention provides the compound, the pharmaceutically acceptable salt thereof, or the isomer thereof according to any one among (1) to (6), being a ketohexokinase (KHK) inhibitor.
(8) The present invention provides the compound, the pharmaceutically acceptable salt thereof, or the isomer thereof according to any one among (1) to (6), being used for preventing or treating metabolic diseases.
(9) The present invention provides a pharmaceutical composition for inhibiting ketohexokinase (KHK), the composition comprising the compound, the pharmaceutically acceptable salt thereof, or the isomer thereof according to any one among (1) to (6), as an active ingredient.
(10) The present invention provides a pharmaceutical composition for preventing or treating metabolic diseases, the composition comprising the compound, the pharmaceutically acceptable salt thereof, or the isomer thereof according to any one among (1) to (6), as an active ingredient.
(11) The present invention provides the pharmaceutical composition for preventing or treating metabolic diseases according to (10), wherein the metabolic disease is diabetes, complications of diabetes, obesity, nonalcoholic steatohepatitis, or fatty hepatitis.
(12) The present invention provides the pharmaceutical composition for preventing or treating metabolic diseases according to (10) or (11), wherein the metabolic disease is nonalcoholic steatohepatitis.
(13) The present invention provides a method for preparing a pharmaceutical composition for preventing or treating diabetes, complications of diabetes, obesity, nonalcoholic steatohepatitis, or fatty hepatitis, the method comprising a step of mixing the compound, the pharmaceutically acceptable salt thereof, or the isomer thereof according to any one among (1) to (6), as an active ingredient with a pharmaceutically acceptable carrier.
(14) The present invention provides a method for treating a metabolic disease comprising nonalcoholic steatohepatitis in a mammal, the method comprising administering the compound, the pharmaceutically acceptable salt thereof, or the isomer thereof according to any one among (1) to (6).

### ADVANTAGEOUS EFFECTS

The (S)-2-(2-methylazetidin-1-yl)pyrimidine derivative compound of Formula 1 according to the present invention plays the role of a ketohexokinase (KHK) inhibitor, and may be effectively used for preventing or treating metabolic diseases such as diabetes, complications of diabetes, obesity, nonalcoholic steatohepatitis, and fatty hepatitis.

### BEST MODE FOR CARRYING OUT THE INVENTION

Hereinafter, embodiments will be described in more detail to assist the understanding of the present invention.

It will be understood that words or terms used in the present disclosure and claims shall not be interpreted as the meaning defined in commonly used dictionaries. It will be further understood that the words or terms should be interpreted as having a meaning that is consistent with their meaning in the context of the relevant art and the technical idea of the invention, based on the principle that an inventor may properly define the meaning of the words or terms to best explain the invention.

### Definition of terms

The term "substitution" in the present disclosure may mean the substitution of the hydrogen of a functional group, an atomic terminal or a compound with a specific substituent. In case where the hydrogen of a functional group, an atomic terminal or a compound is substituted with a specific substituent, there may be present multiple substituents of one or two or more according to the number of hydrogen, and in case where multiple substituents are present, the substituents may be the same or different from each other.

The term "alkyl group" in the present disclosure may mean a monovalent aliphatic saturated hydrocarbon and may mean including all of: a linear alkyl group such as methyl, ethyl, propyl and butyl; a branch type alkyl group such as isopropyl, sec-butyl, tert-butyl and neo-pentyl; and a cyclic saturated hydrocarbon, or a cyclic unsaturated hydrocarbon including one or two or more unsaturated bonds.

The term "alkylene group" in the present disclosure may mean a divalent aliphatic saturated hydrocarbon such as methylene, ethylene, propylene and butylene.

The term "heterocycloalkyl group" in the present disclosure means a partially or fully saturated hydrocarbon including N, S or O as a heteroatom as a ring-forming atom and forming a single or fused ring, and may mean a monocycle forming one ring and a polycyclic structure of two or more fused rings.

The term "direct linkage" in the present disclosure may mean a single covalent bond itself not including a separate atom or a molecular moiety.

In the present disclosure, the terms "comprising", and "having" and the derivatives thereof, though these terms are particularly disclosed or not, do not intended to preclude the presence of optional additional components, steps, or processes. In order to avoid any uncertainty, all compositions claimed by using the term "comprising" may include optional additional additives, auxiliaries, or compounds, including a polymer or any other materials, unless otherwise described to the contrary. In contrast, the term "consisting essentially of ~" excludes unnecessary ones for operation and precludes optional other components, steps or processes from the scope of optional continuous description. The term "consisting of ~" precludes optional components, steps or processes, which are not particularly described or illustrated.

The term "pharmaceutically acceptable salt" in the present disclosure includes acid-addition salts which are formed from inorganic acids such as hydrochloric acid, sulfuric acid, nitric acid, phosphoric acid, hydrobromic acid, and hydroiodic acid; organic acids such as tartaric acid, formic acid, citric acid, acetic acid, trichloroacetic acid, trifluoroacetic acid, gluconic acid, benzoic acid, lactic acid, fumaric acid, maleic acid and salicylic acid; or sulfonic acids such as methanesulfonic acid, ethansulfonic acid, benzenesulfonic acid, and p-toluenesulfonic acid, which form non-toxic acid-addition salts containing pharmaceutically acceptable anions. In addition, the "pharmaceutically acceptable salt" includes carboxylates, and the carboxylate includes, for example, alkali metal or alkaline earth metal salts formed by lithium, sodium, potassium, or magnesium, amino acid salts such as lysine, arginine and guanidine, and organic salts such as dicyclohexylamine, N-methyl-D-glucamine, tris(hydroxymethyl)methylamine, diethanolamine, choline, and triethylamine. Accordingly, the term "pharmaceutically acceptable salt" in the present invention means converted types of the compound of Formula 1 into pharmaceutically acceptable salts as described above.

The term "isomer" in the present disclosure means a molecule having the same molecular formula but different atomic arrangement or three-dimensional structure, and may have an achiral carbon center and an asymmetric axis or asymmetric plane, and accordingly, may include all of E or Z isomers, R or S isomers, racemates, stereoisomer mixtures and individual stereoisomers. Accordingly, "the isomer thereof" in the present disclosure may mean that the compound of Formula 1 includes such isomers and mixtures as described above.

The present invention provides the (S)-2-(2-methylazetidin-1-yl)pyrimidine derivative compound of Formula 1, a pharmaceutically acceptable salt thereof or an isomer thereof, which plays the role of a ketohexokinase (KHK) inhibitor and is effective for preventing or treating metabolic diseases such as diabetes, complications of diabetes, obesity, nonalcoholic steatohepatitis, and fatty hepatitis.

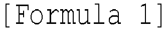

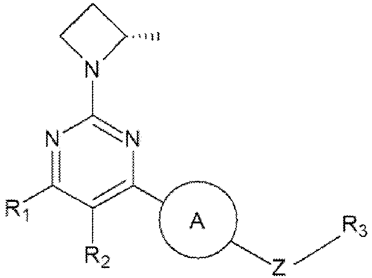

In Formula 1, R₁ and R₂ may be each independently any one selected from the group consisting of hydrogen, a halogen group, and a C₁-C₃ alkyl group, or connected with each other to form a C₃-C₆ aliphatic cyclic group or a C₃-C₆ heterocycloalkyl group. Particularly, R₁ and R₂ may be each independently hydrogen or a C₁-C₃ alkyl group, or connected with each other to form a C₃-C₆ aliphatic cyclic group. In this case, the alkyl group, the aliphatic cyclic group or the heterocycloalkyl group may be substituted with 1 to 5, particularly, 1 to 3 halogen groups, and the halogen group may be F or Cl.

In addition, in Formula 1, R₃ may be -(CR₄R₅)ₙ-B-R₆.

In this case, n may be an integer of 0 to 3, particularly, an integer of 0 to 2, for example, an integer of 0 to 1.

In addition, R₄ and R₅ may be each independently hydrogen or a C₁-C₃ alkyl group. Particularly, R₄ and R₅ may be each independently hydrogen or a C₁-C₂ alkyl group.

In addition, B may be a direct linkage, or any one among -C(O)- and -S(O)₂-.

In addition, R₆ may be hydrogen, a hydroxyl group, or a C₃-C₇ heterocycloalkyl group, and particularly, R₆ may be a hydroxyl group, or a C₃-C₇ heterocycloalkyl group. In this case, the C₃-C₇ heterocycloalkyl group may include N, O or S, particularly N. In addition, the C₃-C₇ heterocycloalkyl group may be substituted with a C₁-C₃ alkyl group, a carbonyl group or a C₁-C₃ alkylcarbonyl group. For example, R₆ may be a C₃-C₇ heterocycloalkyl group containing N, particularly, azetidinyl, pyrrolidinyl, piperidinyl, morpholinyl, imidazolinyl, piperazinyl, or the like.

In addition, in Formula 1, Z may be a direct linkage, a C₁-C₃ alkylene group, -O- or -C(O)-.

In addition, in Formula 1, A may be a C₃-C₈ heterocycloalkyl group. In this case, the C₃-C₈ heterocycloalkyl group may include N, O or S, particularly N. In addition, the C₃-C₈ heterocycloalkyl group may be substituted with a C₁-C₃ alkyl group or a carbonyl group.

Particularly, A may be a substituent represented by Formula 1a or Formula 1b.

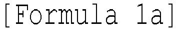

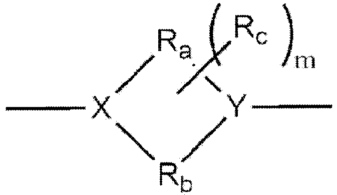

In Formula 1a, X and Y may be each independently CH or N, Rₐ and R_{b} may be each independently a C₁-C₃ alkylene group, R_{c} may be a C₁-C₃ alkyl group or a carbonyl group, and m may be 0 or 1,

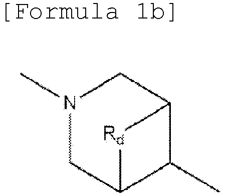

in Formula 1b, R_{d} may be a direct linkage or CH₂.

Meanwhile, in Formula 1, A may be

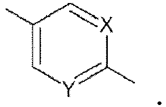

X and Y may be each independently N or C-Rₐ, Rₐ may be hydrogen or a halogen group, Z may be a C₁-C₃ alkylene group or -O-, R₁ and R₂ may be connected with each other to form a C₃-C₆ aliphatic cyclic group, unsubstituted or substituted with 1 to 3 halogen groups, R₃ may be -(CR₄R₅)ₙ-A-R₆, R₄ and R₅ may be hydrogen, n may be an integer of 0 to 3, A may be -C (O)-, and R₆ may be a hydroxyl group, or a C₄-C₆ heterocycloalkyl group containing N, unsubstituted or substituted with a C₁-C₃ alkyl group, wherein the halogen group may be F or Cl.

In another embodiment, in case where A is 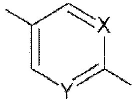 in the compound of Formula 1 of the present invention, in Formula 1, X may be N, Y may be C-Rₐ, where Rₐ may be hydrogen, Z may be a C₁-C₃ alkylene group or -O-, R₁ may be a C₁-C₃ alkyl group substituted with 1 to 3 halogen groups, R₂ may be hydrogen or a C₁-C₃ alkyl group, R₃ may be -(CR₄R₅)ₙ-A-R₆, R₄ and R₅ may be hydrogen, n may be an integer of 0 to 3, A may be -C(O)-, and R₆ may be a C₄-C₆ heterocycloalkyl group containing N, unsubstituted or substituted with a C₁-C₃ alkyl group.

In an embodiment, the compound of Formula 1 according to the present invention may be one or more selected from the following group:
(S)-2-((1-(2-(2-methylazetidin-2-yl)-6-(trifluoromethyl)pyrimidin-4-yl)azetidin-3-yl)oxy)-1-(piperazin-1-yl)ethan-1-one;
(S)-2-((1-(5-chloro-2-(2-methylazetidin-1-yl)-6-(trifluoromethyl)pyrimidin-4-yl)azetidin-3-yl)oxy)-1-(piperazin-1-yl)ethyn-1-one;
(S)-2-((1-(7,7-difluoro-2-(2-(methylazetidin-1-yl)-6,7-dihydro-5H-cyclopenta[d]pyrimidin-4-yl)azetidin-3-yl)oxy)-1-(piperazin-1-yl)ethan-1-one;
(S)-2-((1-(8,8-difluoro-2-(2-methylazetidin-1-yl)-5,6,7,8-tetrahydroquinazolin-4-yl)azetidin-3-yl)oxy)-1-(piperazin-1-yl)ethan-1-one;
2-(((2S,3R)-1-(7,7-difluoro-2-((S)-2-methylazetidin-1-yl)-6,7-dihydro-5H-cyclopenta[d]pyrimidin-4-yl)-2-methylazetidin-3-yl)oxy)-1-(piperazin-1-yl)ethan-1-one;
2-(((S)-1-(2-((S)-2-methylazetidin-1-yl)-6-(trifluoromethyl)pyrimidin-4-yl)pyrrolidin-3-yl)oxy)-1-(piperazin-1-yl)ethan-1-one;
2-(((S)-1-(7,7-difluoro-2-((S)-2-methylazetidin-1-yl)-6,7-dihydro-5H-cyclopenta[d]pyrimidin-4-yl)pyrrolidin-3-yl)oxy)-1-(piperazin-1-yl)ethan-1one;
2-(((R)-1-(7,7-difluoro-2-((S)-2-methylazetidin-1-yl)-6,7-dihydro-5H-cyclopenta[d]pyrimidin-4-yl)pyrrolidin-3-yl)oxy)-1-(piperazin-1-yl)ethan-1one;
2-((R)-1-(7,7-difluoro-2-((S)-2-methylazetidin-1-yl)-6,7-dihydro-5H-cyclopenta[d]pyrimidin-4-yl)pyrrolidin-3-yl)-1-(piperazin-1-yl)ethan-1one;
(S)-2-((1-(7,7-difluoro-2-(2-methylazetidin-1-yl)-6,7-dihydro-5H-cyclopenta[d]pyrimidin-4-yl)piperazin-4-yl)oxy)-1-(piperazin-1-yl)ethan-1-one;
(S)-2-(4-(2-(2-methylazetidin-1-yl)-6-(trifluoromethyl)pyrimidin-4-yl)piperazin-1-yl)-1-(piperazin-1-yl)ethan-1-one;
(S)-2-(4-(5-chloro-2-(2-methylazetidin-1-yl)-6-(trifluoromethyl)pyrimidin-4-yl)piperazin-1-yl)-1-(piperazin-1-yl)ethan-1-one;
(S)-2-(4-(2-(2-methylazetidin-1-yl)-6-(trifluoromethyl)pyrimidin-4-yl)-1-(2-oxo-2-(piperazin-1-yl)ethyl)piperazin-2-one;
(S)-2-(4-(7,7-difluoro-2-(2-methylazetidin-1-yl)-6,7-dihydro-5H-cyclopenta[d]pyrimidin-4-yl)piperazin-1-yl)-1-(piperazin-1-yl)ethan-1-one;
(S)-2-(4-(7,7-difluoro-2-(2-methylazetidin-1-yl)-6,7-dihydro-5H-cyclopenta[d]pyrimidin-4-yl)piperazin-1-yl)-1-morpholinoethan-1-one;
(S)-1-(4-(7,7-difluoro-2-(2-methylazetidin-1-yl)-6,7-dihydro-5H-cyclopenta[d]pyrimidin-4-yl)piperazin-1-yl)-3-(piperazin-1-yl)propan-1,3-dione;
(S)-(4-(7,7-difluoro-2-(2-methylazetidin-1-yl)-6,7-dihydro-5H-cyclopenta[d]pyrimidin-4-yl)-1-(2-oxo-2-(piperazin-1-yl)ethyl)piperazin-2-one;
(S)-2(4-(7,7-difluoro-2-(2-methylazetidin-1-yl)-6,7-dihydro-5H-cyclopenta[d]-pyrimidin-4-yl)piperidin-1-yl)-1-(piperazin-1-yl)ethan-1-one;
2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)-1-(piperazin-1-yl)ethan-1-one;
2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)-1-(4-methylpiperazin-1-yl)ethan-1-one;
4-(2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)acetyl)piperazin-2-one;
2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)-1-(piperazin-1-yl)ethan-1-one;
1-(4-acetylpiperazin-1-yl)-2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)ethan-1-one;
2-((1R,5S,6S)-3-(8,8-difluoro-2-((S)-2-methylazetidin-1-yl)-5,6,7,8-tetrahydroquinazol-4-yl)-3-azabicyclo[3.1.1]heptan-6-yl)-1-(piperazin-1-yl)ethan-1-one;
2-((1R,5S,6S)-3-(8,8-difluoro-2-((S)-2-methylazetidin-1-yl)-5,8-dihydro-6H-pyrazol[3,4-d]pyrimidin-4-yl)-3-azabicyclo[3.1.1]heptan-6-yl)-1-(piperazin-1-yl)ethan-1-one;
7,7-difluoro-2-((S)-2-methylazetidin-1-yl)-4-((1R,5S,6S)-6-((piperazin-1-ylsulfonyl)methyl)-3-azabicyclo[3.1.0]hexan-3-yl)-6,7-dihydro-5H-cyclopenta[d]pyrimidine;
(S)-2-((1-(5-methyl-2-(2-methylazetidin-1-yl)-6-(trifluoro)pyrimidin-4-yl)azetidin-3-yl)oxy)-1-(piperazin-1-yl)ethan-1-one;
(S)-2-((1-(2-(2-methylazetidin-1-yl)-6-(trifluoromethyl)pyrimidin-4-yl)piperidin-4-yl)oxy)-1-(piperazin-1-yl)ethan-1-one;
(S)-2-((1-(5-methyl-2-(2-methylazetidin-1-yl)-6-(trifluoro)pyrimidin-4-yl)piperidin-4-yl)oxy)-1-(piperazin-1-yl)ethan-1-one;
(S)-2-(4-(7,7-difluoro-2-(2-methylazetidin-1-yl)-6,7-dihydro-5H-cyclopenta[d]-pyrimidin-4-yl)phenyl)acetic acid;
(S)-2-(4-(7,7-difluoro-2-(2-methylazetidin-1-yl)-6,7-dihydro-5H-cyclopenta[d]-pyrimidin-4-yl)phenyl)-1-(piperazin-1-yl)ethan-1-one;
(S)-2-(5-(2-(2-methylazetidin-1-yl)-6-(trifluoromethyl)pyrimidin-4-yl)pyridin-2-yl)-1-(piperazin-1-yl)ethan-1-one;
(S)-2-(4-(7,7-difluoro-2-(2-methylazetidin-1-yl)-6,7-dihydro-5H-cyclopenta[d]pyrimidin-4-yl)phenoxy)-1-(piperazin-1-yl)ethan-1-one;
(S)-2-(4-(7,7-difluoro-2-(2-methylazetidin-1-yl)-6,7-dihydro-5H-cyclopenta[d]pyrimidin-4-yl)-2-fluorophenoxy)-1-(piperazin-1-yl)ethan-1-one;
(S)-2-(4-(7,7-difluoro-2-(2-methylazetidin-1-yl)-6,7-dihydro-5H-cyclopenta[d]pyrimidin-4-yl)-2,6-difluorophenoxy)-1-(piperazin-1-yl)ethan-1-one;
(S)-2-(2-chloro-4-(7,7-difluoro-2-(2-methylazetidin-1-yl)-6,7-dihydro-5H-cyclopenta[d]pyrimidin-4-yl)phenoxy)-1-(piperazin-1-yl)ethan-1-one;
(S)-2-(2-chloro-4-(7,7-difluoro-2-(2-methylazetidin-1-yl)-6,7-dihydro-5H-cyclopenta[d]pyrimidin-4-yl)phenoxy)-1-(1,4-diazepan-1-yl)ethan-1-one;
(S)-2-((5-(7,7-difluoro-2-(2-methylazetidin-1-yl)-6,7-dihydro-5H-cyclopenta[d]pyrimidin-4-yl)pyridin-2-yl)oxy)-1-(piperazin-1-yl)ethan-1-one;
(S)-2-((3-chloro-5-(7,7-difluoro-2-(2-methylazetidin-1-yl)-6,7-dihydro-5H-cyclopenta[d]pyrimidin-4-yl)pyridin-2-yl)oxy)-1-(piperazin-1-yl)ethan-1-one;
(S)-2-((5-(2-(2-methylazetidin-1-yl)-6-(trifluoromethyl)pyrimidin-4-yl)pyridin-2-yl)oxy)-1-(piperazin-1-yl)ethan-1-one;
(S)-2-((5-(5-methyl-2-(2-methylazetidin-1-yl)-6-(trifluoromethyl)pyrimidin-4-yl)pyridin-2-yl)oxy)-1-(piperazin-1-yl)ethan-1-one.

The compound of Formula 1, the pharmaceutically acceptable salt thereof or the isomer thereof according to the present invention has effects as a ketohexokinase (KHK) inhibitor. Accordingly, the compound of Formula 1, the pharmaceutically acceptable salt thereof or the isomer thereof according to the present invention may be used as a ketohexokinase (KHK) inhibitor, and in another embodiment, may be suitably used for preventing or treating metabolic diseases involving ketohexokinase (KHK).

In addition, the present invention provides a pharmaceutical composition for inhibiting ketohexokinase (KHK), comprising the compound of Formula 1, the pharmaceutically acceptable salt thereof or the isomer thereof, as an active ingredient. In addition, various types of predrugs converted into the compound of Formula 1 according to the purpose in vivo are included in the scope of the present invention.

The pharmaceutical composition according to the present invention may be used for preventing or treating metabolic diseases involved in ketohexokinase (KHK). The metabolic diseases involved in the ketohexokinase (KHK) may be induced by the overexpression or overactivation of the ketohexokinase. Particularly, the metabolic diseases prevented or treated by the pharmaceutical composition according to the present invention may include diabetes, complications of diabetes, obesity, nonalcoholic steatohepatitis, and fatty hepatitis, without limitation, and preferably, the pharmaceutical composition may be used for preventing or treating nonalcoholic steatohepatitis.

In addition, the present invention provides a method for preparing a pharmaceutical composition for preventing or treating diabetes, complications of diabetes, obesity, nonalcoholic steatohepatitis, and fatty hepatitis, including a step of mixing the compound of Formula 1, the pharmaceutically acceptable salt thereof or the isomer thereof, as an active ingredient, with a pharmaceutically acceptable carrier.

The "pharmaceutical composition" may include the compound of the present invention and other chemical components such as diluents, carriers, or the like. Accordingly, the pharmaceutical composition may include pharmaceutically acceptable carriers, diluents, excipients, or combinations thereof, as necessary. The pharmaceutical composition facilitates the administration of the compound into an organism. Various methods for administering the compound exist and include, but are not limited to, oral, injection, aerosol, parenteral, and local administration.

The "carrier" means a compound that facilitates the introduction of a compound into a cell or tissue. For example, dimethylsulfoxide (DMSO) is a common carrier facilitating the introduction of many organic compounds into cells or tissues in an organism.

The "diluent" is defined as a compound that not only stabilizes a biologically active form of a compound of interest but is also diluted in water dissolving the compound. Dissolved salts in a buffer solution are used as diluents in this technical field. A commonly used buffer solution is phosphate buffered saline mimicking a salt form of the human body fluid. Since a buffer salt may control the pH of a solution at low concentration, a buffer diluent hardly modify the biological activity of the compound.

The "pharmaceutically acceptable" means properties that do not impair the biological activities and physical properties of the compound.

The compound of the present invention may be formulated as various pharmaceutical dosage forms depending on the purpose. For the preparation of the pharmaceutical composition according to the present invention, an effective ingredient, particularly, the compound of Formula 1, the pharmaceutically acceptable salt thereof or the isomer thereof, is mixed together with various pharmaceutically acceptable carriers which may be selected according to the formulation to be prepared. For example, the pharmaceutical composition according to the present invention may be formulated as preparations for injection, oral preparations, or the like, depending on the purpose.

The compound of the present invention may be formulated by known methods using known pharmaceutical carriers and excipients, and inserted into a unit dose form or a multi-dose container. The preparation may be a solution in oil or aqueous medium, a suspension or an emulsion type, and may include common dispersing agents, suspending agents, or stabilizing agents. In addition, the preparation may be, for example, a dry powder form, which is used by dissolving in sterilized, pyrogen-free water before use. The compound of the present invention may be formulated into suppositories by using a common suppository base such as cocoa butter and other glycerides. As solid dosage forms for oral administration, capsules, tablets, pills, powders, and granules may be prepared, and capsules and tablets are especially useful. Tablets and pills are preferably enteric-coated. Solid dosage forms may be manufactured by mixing the compound of the present invention with carriers including one or more inert diluents, such as sucrose, lactose, and starch, lubricants such as magnesium stearate, disintegrating agents, binders, or the like. The compound or the pharmaceutical composition containing the same, according to the present invention, may be administered in combination with other drugs, for example, other diabetes treatment drugs, as required.

In addition, the present invention provides a method for preventing or treating a metabolic disease involved in ketohexokinase (KHK) in mammals, by administering the compound of Formula 1, the pharmaceutically acceptable salt thereof, or the isomer thereof as an effective ingredient. Typical examples for treating through the ketohexokinase (KHK) inhibitor may include metabolic diseases including the above-described diabetes, complications of diabetes, obesity, nonalcoholic steatohepatitis, and fatty hepatitis, without limitation, but may preferably include nonalcoholic steatohepatitis. In the disclosure, the "treatment" means stopping, delaying or ameliorating the progress of diseases in a subject exhibiting symptoms of diseases. The "prevention" means stopping, delaying or ameliorating the sign of diseases in a subject at risk of exhibiting symptoms of diseases, even if he or she does not exhibit the symptoms.

Meanwhile, the compound of Formula 1 according to an embodiment of the present invention may be prepared through the particular methods as illustrated in Reaction 1 to Reaction 12 below.

Hereinafter, the method for preparing the compound of Formula 1 will be explained based on exemplary reactions to assist the understanding of the present invention. However, a person skilled in the art could prepare the compound of Formula 1 by various methods based on the structure of Formula 1, and such methods should be interpreted as being within the scope of the present invention. That is, the compound of Formula 1 may be prepared by the synthetic methods described herein or by optionally combining various synthetic methods disclosed in the prior art, which should be interpreted as being within the scope of the present invention. The method for preparing the compound of Formula 1 is not limited only to those described below.

When preparing the compound of the present invention, it is possible to appropriately change the reaction sequence. That is, it is possible to run first optional processes or insert optional processes to change substituents, and use any reagents other than the exemplified reagents as needed. Compounds obtained in each process could be separated or purified by conventional methods, such as recrystallization, distillation, or silica gel column chromatography. Furthermore, the compound obtained in each process could be used in the next step without further purification or separation.

In the reactions below, unless indicated otherwise, all substituents are as previously defined. Reagents and starting materials could be obtained readily commercially. Others could be produced by synthetic methods described in the Preparation Examples and Examples below, including known synthetic methods for structurally similar compounds. Unless otherwise noted, compounds used as starting materials are known ones or those which could be prepared by known synthetic methods or similar methods from known compounds.

### MODE FOR CARRYING OUT THE INVENTION

Hereinafter, embodiments of the present invention will be explained in detail so that a person skilled in this technical field in which the present invention belongs to may easily perform. However, the present invention could be accomplished in various different forms and is not limited by the embodiments explained herein.

### Preparation Example 1

Steps A to C were performed in sequence to prepare (4,5-dichloro-2-(methylthio)-6-(trifluoromethyl)pyrimidine of Formula a.

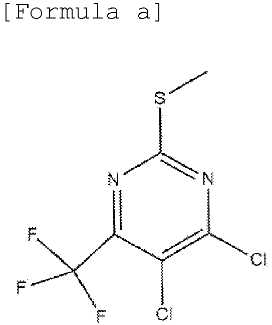

### (1) Step A: Preparation of 2-(methylthio)-6-(trifluoromethyl)pyrimidinyl-4-ol

Ethyl 4,4,4-trifluoro-3-oxobutanoate (4.0 g, 21.7 mmol), S-methylisothiourea hemisulfate (6.0 g, 43.4 mmol) and sodium carbonate (9.2 g, 86.9 mmol) were dissolved in 50 ml of water, followed by stirring at room temperature for 12 hours. After finishing the reaction, a 1 N hydrochloric acid solution was slowly added to the reaction solution at 0°C. The solid thus produced was filtered, washed with water, and dried to obtain 2-(methylthio)-6-(trifluoromethyl)pyrimidinyl-4-ol (2.5 g, 11.9 mmol, yield 55%). The synthesis of the 2-(methylthio)-6-(trifluoromethyl)pyrimidinyl-4-ol thus obtained was confirmed through ¹H NMR analysis.

¹H NMR (400 MHz, DMSO-d₆) δ 2.51 (s, 3H).

### (2) Step B: Preparation of 5-chloro-2-(methylthio)-6-(trifluoromethyl)pyrimidinyl-4-ol

The 2-(methylthio)-6-(trifluoromethyl)pyrimidinyl-4-ol (9.3 g, 44.2 mmol) obtained in Step A, N-chlorosuccinimide (8.9 g, 66.4 mmol) and acetic acid (5.1 ml, 88.0 mmol) were dissolved in 300 ml of acetonitrile, and stirred at 80°C for 12 hours. After finishing the reaction, distillation under a reduced pressure was performed to obtain concentrated 5-chloro-2-(methylthio)-6-(trifluoromethyl)pyrimidinyl-4-ol, and this product was used in the next reaction without a separate purification process. In addition, the synthesis of the 5-chloro-2-(methylthio)-6-(trifluoromethyl)pyrimidinyl-4-ol thus obtained was confirmed through ¹H NMR analysis.

¹H NMR (500MHz, DMSO-d₆) δ 2.51 (s, 3H).

### (3) Step C: Preparation of 4,5-dichloro-2-(methylthio)-6-(trifluoromethyl)pyrimidine

The 5-chloro-2-(methylthio)-6-(trifluoromethyl)pyrimidinyl-4-ol obtained in Step B, phosphoryl trichloride (20.6 ml, 221 mmol) and benzyltriethylammonium chloride (10.1 g, 44.2 mmol) were dissolved in 200 ml of dichloroethane, and stirred at 80°C for 3 hours. After finishing the reaction, the reaction solution was distilled under a reduced pressure to concentrate, a saturated sodium bicarbonate solution was added to the concentrate, and the resultant was extracted with ethyl acetate. An organic layer was washed with an aqueous sodium chloride solution, dried over anhydrous magnesium sulfate, and filtered. A filtrate was concentrated under a reduced pressure and purified by column chromatography (ethyl acetate/hexane = 1/10 weight ratio) to obtain 4,5-dichloro-2-(methylthio)-6-(trifluoromethyl)pyrimidine (8.63 g, 32.8 mmol, yield 740). The synthesis of the 4,5-dichloro-2-(methylthio)-6-(trifluoromethyl)pyrimidine thus obtained was confirmed through ¹H NMR analysis.

¹H NMR (500MHz, DMSO-d₆) δ 2.54 (s, 3H).

### Preparation Example 2

4-Chloro-7,7-difluoro-2-(methylthio)-6,7-dihydro-5H-cyclopenta[d]pyrimidine of Formula b below was prepared by performing Steps A to K in sequence.

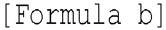

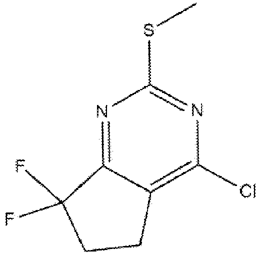

### (1) Step A: Preparation of 5-(tert-butyl) 1-ethyl 2,2-difluoropentanedioate

Ethyl 2-bromo-2,2-difluoroacetate (20 g, 99 mmol), tert-butyl acrylate (11.1 ml, 76 mmol), copper (9.6 g, 152 mmol), N1,N1,N2,N2-tetramethylethan-1,2-diamine (5.7 ml, 37.9 mmol) and acetic acid (4.8 ml, 83 mmol) were dissolved in 100 ml of tetrahydrofuran, and stirred at 50°C for 4 hours. After finishing the reaction, a saturated aqueous ammonium chloride solution was added to the reaction solution, and extraction was performed with ethyl acetate. An organic layer was washed with an aqueous sodium chloride solution, dried over anhydrous magnesium sulfate, and filtered. The filtrate was concentrated under a reduced pressure to obtain 5-(tert-butyl) 1-ethyl 2,2-difluoropentanedioate. The synthesis of the 5-(tert-butyl) 1-ethyl 2,2-difluoropentanedioate thus obtained was confirmed through ¹H NMR analysis.

¹H NMR (400 MHz, CDCl₃) δ 4.31 (q, J=7.0 Hz, 2H), 2.32-2.47 (m, 4H), 1.43 (s, 9H), 1.34 (t, J=7.3 Hz, 3H).

### (2) Step B: Preparation of 5-ethoxy-4,4-difluoro-5-oxopentanoic acid

The 5-(tert-butyl) 1-ethyl 2,2-difluoropentanedioate obtained in Step A was dissolved in trifluoroacetic acid (30 ml) and dichloromethane (70 ml), and stirred at room temperature for 8 hours. After finishing the reaction, the resultant was concentrated under a reduced pressure. An aqueous saturated sodium bicarbonate solution was added to the concentrate, and extraction was performed with ethyl acetate. An organic layer was washed with an aqueous sodium chloride solution, dried over anhydrous magnesium sulfate, and filtered. The filtrate was concentrated under a reduced pressure to obtain 5-ethoxy-4,4-difluoro-5-oxopentanoic acid (15 g, 76 mmol, yield 100%). The synthesis of the 5-ethoxy-4,4-difluoro-5-oxopentanoic acid thus obtained was confirmed through ¹H NMR analysis.

¹H NMR (500MHz, CDCl₃) δ 4.36 (q, J=7.2 Hz, 2H), 2.66 (t, J=7.6 Hz, 2H), 2.42-2.51 (m, 2H), 1.39 (t, J=7.2 Hz, 3H).

### (3) Step C: Preparation of ethyl 2,2-difluoro-5-hydroxypentanoate

The 5-ethoxy-4,4-difluoro-5-oxopentanoic acid (12.0 g, 61 mmol) and a borane dimethylsulfide tetrahydrofuran solution (2 M, 36.6 ml. 73 mmol) were dissolved in 300 ml of tetrahydrofuran, followed by stirring at 50°C for 12 hours. After finishing the reaction, water (10 ml) was added to the reaction solution, and stirring was performed at 60°C for 1 hour. After finishing the reaction, water was added to the reaction solution, and extraction was performed with ethyl acetate. An organic layer was washed with an aqueous sodium chloride solution, dried over anhydrous magnesium sulfate, and filtered. The filtrate was concentrated under a reduced pressure to obtain ethyl 2,2-difluoro-5-hydroxypentanoate (6.5 g, 35.7 mmol, yield 590). The synthesis of the ethyl 2,2-difluoro-5-hydroxypentanoate thus obtained was confirmed through ¹H NMR analysis.

¹H NMR (500MHz, CDCl₃) δ 4.35 (q, J=7.1 Hz, 2H), 3.74 (t, J=6.3 Hz, 2H), 2.16-2.26 (m, 2H), 1.72-1.81 (m, 2H), 1.38 (t, J=7.2 Hz, 3H).

### (4) Step D: Preparation of ethyl 2,2-difluoro-5-((methanesulfonyl)oxy)pentanoate

The ethyl 2,2-difluoro-5-hydroxypentanoate (9.4 g, 51.6 mmol) obtained in Step C, methanesulfonyl chloride (4.42 ml, 56.8 mmol), diisopropylethylamine (18.0 ml, 103 mmol) and 4-dimethylaminopyridine (315 mg, 2.58 mmol) were dissolved in dichloromethane (200 ml), followed by stirring at room temperature for 3 hours. After finishing the reaction, a N hydrochloric acid solution was added to the reaction solution, and the resultant was extracted with ethyl acetate. An organic layer was washed with an aqueous sodium chloride solution, dried over anhydrous magnesium sulfate, and filtered. The filtrate was concentrated under a reduced pressure and purified by column chromatography (ethyl acetate/hexane = 1/3 by a weight ratio) to obtain ethyl 2,2-difluoro-5-((methanesulfonyl)oxy)pentanoate (13.3 g, 51.1 mmol, yield 99%). The synthesis of the ethyl 2,2-difluoro-5-((methanesulfonyl)oxy)pentanoate thus obtained was confirmed through ¹H NMR analysis.

¹H NMR (500MHz, CDCl₃) δ 4.37 (q, J=7.1 Hz, 2H), 4.31 (t, J=6.3 Hz, 2H), 3.06 (s, 3H), 2.20-2.29 (m, 2H), 1.99-2.05 (m, 2H), 1.38-1.40 (m, 3H).

### (5) Step E: Preparation of ethyl 5-cyano-2,2-difluoropentanoate

The ethyl 2,2-difluoro-5-((methanesulfonyl)oxy)pentanoate (13.3 g, 51.1 mmol) prepared in Step D, and potassium cyanate (10,0 g, 153.3 mmol) were dissolved in a mixture solution of 150 ml of tetrahydrofuran and 150 ml of dimethylformamide, followed by stirring at 70°C for 18 hours. After finishing the reaction, the reaction solution was concentrated under a reduced pressure. To the concentrate, an aqueous saturated ammonium chloride solution was added, and the resultant was extracted with ethyl acetate. An organic layer was washed with an aqueous sodium chloride solution, dried over anhydrous magnesium sulfate, and filtered. The filtrate was concentrated under a reduced pressure and purified by column chromatography (ethyl acetate/hexane = 1/1 by a weight ratio) to obtain ethyl 5-cyano-2,2-difluoropentanoate (7.1 g, 37.1 mmol, yield 730). The synthesis of the ethyl 5-cyano-2,2-difluoropentanoate thus obtained was confirmed through ¹H NMR analysis.

¹H NMR (400MHz, CDCl₃) δ 4.27-4.35 (m, 2H), 2.45 (t, J=7.1 Hz, 2H), 2.15-2.29 (m, 2H), 1.91 (t, 7.1 Hz, 2H), 1.31-1.37 (m, 3H).

### (6) Step F: Preparation of diethyl 2,2-difluorohexanedioate

The ethyl 5-cyano-2,2-difluoropentanoate (7.1 g, 37.7 mmol) prepared in Step E and acetyl chloride (30 ml) were dissolved in 60 ml of ethanol, followed by refluxing for 48 hours. After finishing the reaction, the resultant was concentrated under a reduced pressure. To the concentrate, an aqueous saturated sodium bicarbonate solution was added, and the resultant was extracted with ethyl acetate. An organic layer was washed with an aqueous sodium chloride solution, dried over anhydrous magnesium sulfate, and filtered. The filtrate was concentrated under a reduced pressure to obtain diethyl 2,2-difluorohexanedioate (6.5 g, 27.3 mmol, yield 740). The synthesis of the diethyl 2,2-difluorohexanedioate thus obtained was confirmed through ¹H NMR analysis.

¹H NMR (500MHz, CDCl₃) δ 4.35 (q. J=7.1 Hz, 2H), 4.16 (q, J=7.1 Hz, 2H), 2.40 (t, J=7.3 Hz, 2H), 2.07-2.20 (m, 2H), 1.86 (m, 2H), 1.38 (t, J=7.2 Hz, 3H), 1.28 (t, J=7.2 Hz, 3H).

### (7) Step G: Preparation of ethyl 3,3-difluoro-2-oxocyclopentan-1-carboxylate

The diethyl 2,2-difluorohexanedioate (51.57 g, 216 mmol) prepared in Step F was dissolved in tetrahydrofuran (1000 ml), and potassium tert-butoxide (26.7 g, 238 mmol) was slowly added thereto, followed by stirring at room temperature for 4 hours. After finishing the reaction, 240 ml of an aqueous 1 N hydrochloric acid solution was slowly added at 0°C, followed by stirring for about 10 minutes. After diluting the reaction product with ethyl acetate, and an organic layer was washed with an aqueous sodium chloride solution. An aqueous solution was extracted with ethyl acetate, and an organic layer collected was dried over anhydrous magnesium sulfate and filtered. The filtrate was concentrated under a reduced pressure and sufficiently dried under a reduced pressure to obtain ethyl 3,3-difluoro-2-oxocyclopentan-1-carboxylate (41.6 g, 216 mmol, yield 99%), and this compound was used in the next reaction without a separate purification.

### (8) Step H: Preparation of 7,7-difluoro-2-(methylthio)-6,7-dihydro-5H-cyclopenta[d]pyrimidin-4-ol

The ethyl 3,3-difluoro-2-oxocyclopentan-1-carboxylate (540 mg, 2.41 mmol) prepared in Step G, methylisothiourea hemisulfate (672 mg, 4.82 mmol), and sodium carbonate (767 mg, 7.24 mmol) were dissolved in 50 ml of water, followed by stirring at room temperature for 12 hours. After finishing the reaction, an excessive amount of water was added to the reaction solution, and extraction was performed with ethyl acetate. An organic layer was washed with an aqueous sodium chloride solution, dried over anhydrous magnesium sulfate, and filtered. The filtrate was concentrated under a reduced pressure and purified by column chromatography (ethyl acetate/hexane = 1/1 by a weight ratio) to obtain 7,7-difluoro-2-(methylthio)-6,7-dihydro-5H-yclopenta[d]pyrimidin-4-ol (250 mg, 1.15 mmol, yield 480). The synthesis of the 7,7-difluoro-2-(methylthio)-6,7-dihydro-5H-yclopenta[d]pyrimidin-4-ol thus obtained was confirmed through ¹H NMR analysis.

¹H NMR (500MHz, DMSO-D₆) δ 1.92-1.97 (m, 2H), 1.81 (s, 3H), 1.71-1.79 (m, 2H).

### (9) Step I: Preparation of 4-chloro-7,7-difluoro-2-(methylthio)-6,7-dihydro-5H-cyclopenta[d]pyrimidine

The 7,7-difluoro-2-(methylthio)-6,7-dihydro-5H-cyclopenta[d]pyrimidin-4-ol (5.2 g, 23.83 mmol) prepared in Step H, phosphoryl trichloride (22.21 ml, 238 mmol) and benzyltriethylammonium chloride (5.43 g, 23.93 mmol) were dissolved in 150 ml of dichloroethane, followed by stirring at 80°C for 3 hours. After finishing the reaction, the reaction solution was distilled under a reduced pressure. To the concentrate, a saturated sodium bicarbonate solution was added, and the resultant was extracted with ethyl acetate. An organic layer was washed with an aqueous sodium chloride solution, dried over anhydrous magnesium sulfate, and filtered. The filtrate was concentrated under a reduced pressure and purified by column chromatography (ethyl acetate/hexane = 1/10 by a weight ratio) to obtain 4-chloro-7,7-difluoro-2-(methylthio)-6,7-dihydro-5H-cyclopenta[d]pyrimidine (5.00 g, 23.83 mmol, yield 89%). The synthesis of the 4-chloro-7,7-difluoro-2-(methylthio)-6,7-dihydro-5H-cyclopenta[d]pyrimidine thus obtained was confirmed through ¹H NMR analysis.

¹H NMR (500MHz, CDCl₃) δ 2.99-3.03 (m, 2H), 2.62-2.71 (m, 5H).

### Preparation Example 3

4-Chloro-8,8-difluoro-2-(methylthio)-5,6,7,8-tetrahydroquinazoline of Formula c below was prepared by performing the reaction below.

8,8-Difluoro-2-(methylthio)-5,6,7,8-tetrahydroquinazolin-4-ol (1.5 g, 6.45 mmol), and benzyltriethylammonium chloride (1.4 g, 6.45 mmol) were dissolved in 50 ml of dichloroethane, and the temperature was reduced to 0°C. Phosphoryl trichloride (4.95 g, 32.29 mmol) was added thereto, and the temperature was raised to 90°C, followed by stirring for 1 hour. After finishing the reaction, the reaction solution was concentrated under a reduced pressure. An excessive amount of water and ethyl acetate were added, an organic layer was dried over anhydrous magnesium sulfate and filtered. The filtrate was concentrated under a reduced pressure and purified by column chromatography (ethyl acetate/hexane = 1/1 by a weight ratio) to obtain 4-chloro-8,8-difluoro-2-(methylthio)-5,6,7,8-tetrahydroquinazoline (0.95 g, 3.82 mmol, yield 59%) of Formula c. The synthesis of the 4-chloro-8,8-difluoro-2-(methylthio)-5,6,7,8-tetrahydroquinazoline thus obtained was confirmed through ¹H NMR analysis.

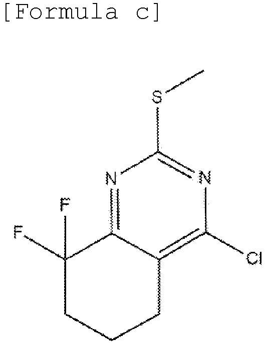

¹H NMR (500MHz, CDCl₃) δ 2.86-2.68 (m, 2H), 2.69-2.51 (m, 3H), 2.50-2.23 (m, 2H), 2.04 (tt, J=9.4, 3.2 Hz, 2H).

### Preparation Example 4

4-Chloro-8,8-difluoro-2-(methylthio)-5,8-dihydro-6H-pyrano[3,4-d]pyrimidine of Formula d was prepared by performing the reaction below.

8,8-Difluoro-2-(methylthio)-5,8-dihydro-6H-pyrano[3,4-d]pyrimidin-4-ol (3.13 g, 13.36 mmol) and benzyltriethylammonium chloride (3.03 g, 13.36 mmol) were dissolved in 50 ml of dichloroethane, and the temperature was reduced to 0°C. Phosphoryl trichloride (10.24 g, 66.78 mmol) was added, and the temperature was raised to 90°C, followed by stirring for 1 hour. After finishing the reaction, the reaction solution was concentrated under a reduced pressure. An excessive amount of water and ethyl acetate were added, and an organic layer was dried over anhydrous magnesium sulfate and filtered. The filtrate was concentrated under a reduced pressure and purified by column chromatography (ethyl acetate/hexane = 1/1 by a weight ratio) to obtain 4-chloro-8,8-difluoro-2-(methylthio)-5,8-dihydro-6H-pyrano[3,4-d]pyrimidine (2.60 g, 10.29 mmol, yield 77%) of Formula d. The synthesis of the 4-chloro-8,8-difluoro-2-(methylthio)-5,8-dihydro-6H-pyrano[3,4-d]pyrimidine thus obtained was confirmed through ¹H NMR analysis.

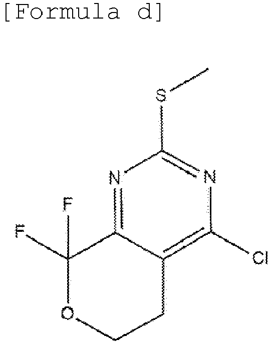

¹H NMR (500MHz, CDCl₃) δ 4.37 (t, J=6.0 Hz, 2H), 3.01-2.87 (m, 2H), 2.60 (s, 3H).

### Preparation Example 5

4-Chloro-2-(methylthio)-6-(trifluoromethyl)pyrimidine of Formula e was prepared by performing the reaction below.

2-(Methylthio)-6-(trifluoromethyl)pyrimidinyl-4-ol (44 g, 209 mmol), phosphoryl trichloride (66 ml, 837 mmol) and benzyltriethylammonium chloride (23.8 g, 104.7 mmol) were stirred at 80°C for 3 hours. After finishing the reaction, the reaction solution was distilled under a reduced pressure to concentrate. To the concentrate, a saturated sodium bicarbonate solution was added, and the resultant was extracted with ethyl acetate. An organic layer was washed with an aqueous sodium chloride solution, dried over anhydrous magnesium sulfate and filtered. The filtrate was concentrated under a reduced pressure and purified by column chromatography (ethyl acetate/hexane = 1/10 by a weight ratio) to obtain 4-chloro-2-(methylthio)-6-(trifluoromethyl)pyrimidine (38.9 g, 170 mmol, yield 81%) of Formula e. The synthesis of the 4-chloro-2-(methylthio)-6-(trifluoromethyl)pyrimidine thus obtained was confirmed through ¹H NMR analysis.

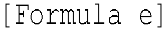

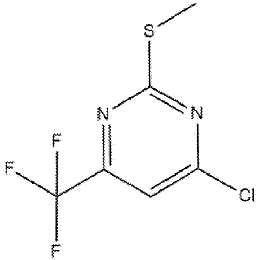

¹H NMR (500MHz, CDCl₃) δ 7.27 (s, 1H), 2.60 (s, 3H).

### Preparation Example 6

4-Chloro-5-methyl-2-(methylthio)-6-(trifluoromethyl)pyrimidine of Formula f was prepared by performing Steps A and B in sequence.

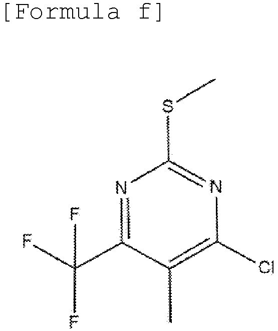

### (1) Step A: Preparation of 5-methyl-2-(methylthio)-6-(trifluoromethyl)pyrimidin-4-ol

Ethyl 4,4,4-trifluoro-2-methyl-3-oxobutanoate (5.0 g, 25.2 mmol), methylisothiourea hemisulfate (7.0 g, 50.3 mmol) and sodium carbonate (5.3 g, 50 mmol) were dissolved in 40 ml of water, followed by stirring at room temperature for 12 hours. After finishing the reaction, an excessive amount of water was added to the reaction solution, and extraction was performed with ethyl acetate. An organic layer was washed with an aqueous sodium chloride solution, dried over anhydrous magnesium sulfate and filtered. The filtrate was concentrated under a reduced pressure and purified by column chromatography (ethyl acetate/hexane = 1/1 by a weight ratio) to obtain 5-methyl-2-(methylthio)-6-(trifluoromethyl)pyrimidin-4-ol (4.0 g, 17.8 mmol, yield 71%). The synthesis of the 5-methyl-2-(methylthio)-6-(trifluoromethyl)pyrimidin-4-ol thus obtained was confirmed through ¹H NMR analysis.

¹H NMR (500MHz, DMSO-d₆) δ 13.29 (s, 1H), 2.49 (s, 3H), 2.03 (s, 3H).

### (2) Step B: Preparation of 4-chloro-5-methyl-2-(methylthio)-6-(trifluoromethyl)pyrimidine

In Preparation Example 5, 5-methyl-2-(methylthio)-6-(trifluoromethyl)pyrimidin-4-ol (18 g, 80 mmol) prepared in Step A was used instead of 2-(methylthio)-6-(trifluoromethyl)pyrimidinyl-4-ol to obtain the title compound of 4-chloro-5-methyl-2-(methylthio)-6-(trifluoromethyl)pyrimidine (16.46 g, 67.8 mmol, yield 840). The synthesis of the 4-chloro-5-methyl-2-(methylthio)-6-(trifluoromethyl)pyrimidine thus obtained was confirmed through ¹H NMR analysis.

¹H NMR (500MHz, CDCl₃) δ 2.57 (s, 3H), 2.42 (q, J=1.5 Hz, 3H).

### Preparation Example 7

Tert-butyl 4-(2-bromoacetyl)piperazin-1-carboxylate of Formula g was prepared by performing the reaction below.

Tert-butyl piperazin-1-carboxylate (6.00 g, 32.21 mmol) and diisopropylethylamine (8.32 g, 64.37 mmol) were dissolved in 200 ml of dichloromethane, and the temperature was reduced to -78°C. Bromoacetyl bromide (7.15 g, 35.43 mmol) was slowly added thereto, the temperature was slowly raised to a room temperature state, and stirring was performed for 1 hour. After finishing the reaction, the reaction solution was concentrated under a reduced pressure. A 1 N aqueous hydrochloric acid solution was added, and the resultant was extracted with ethyl acetate. An organic layer was dried over anhydrous magnesium sulfate and filtered. The filtrate was concentrated under a reduced pressure and purified by column chromatography (ethyl acetate/hexane = 1/1 by a weight ratio) to obtain tert-butyl 4-(2-bromoacetyl)piperazin-1-carboxylate (6.62 g, 21.5 mmol, yield 66%) of Formula g. The synthesis of the tert-butyl 4-(2-bromoacetyl)piperazin-1-carboxylate thus obtained was confirmed through ¹H NMR analysis.

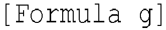

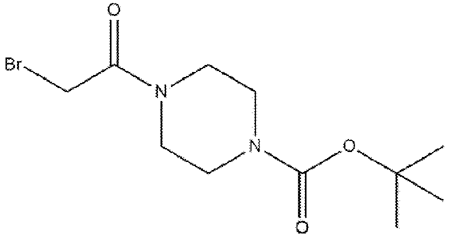

¹H NMR (400MHz, CDCl₃) δ 3.87 (s, 2H), 3.77-3.55 (m, 2H), 3.51 (dd, J=16.2, 5.8 Hz, 4H), 3.47-3.32 (m, 2H), 1.49 (d, J=18.3 Hz, 9H).

### Preparation Example 8

Tert-butyl 4-(2-(azetidin-3-ylol)acetyl)piperazin-1-carboxylate of Formula h was prepared by performing Steps A and B in sequence.

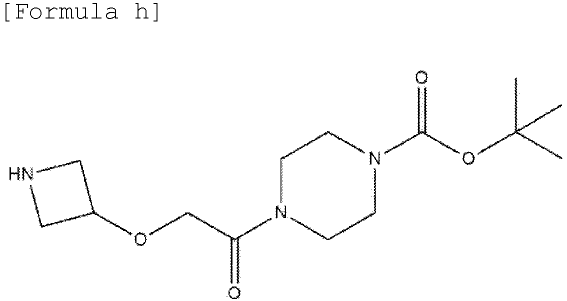

### (1) Step A: Preparation of benzyl 4-(2-((1-tert-butoxycarbonyl)azetidin-3-ylol)acetyl)piperazin-1-carboxylate

Benzyl 3-hydroxyazetidin-1-carboxylate (15 g, 72.34 mmol) was dissolved in 200 ml of dimethylformamide, and the temperature was reduced to 0°C. Sodium hydride (60%, 3.18 g, 79.57 mmol) was added thereto, and the resultant was stirred for 30 minutes. Tert-butyl 4-(2-bromoacetyl)piperazin-1-carboxylate (26.67 g, 86.81 mmol) was slowly added thereto, and the temperature was slowly raised to a room temperature state, while stirring for 18 hours. After finishing the reaction, the reaction solution was concentrated under a reduced pressure. The resultant was extracted using water and ethyl acetate. An organic layer was dried over anhydrous magnesium sulfate and filtered. The filtrate was concentrated under a reduced pressure and purified by column chromatography (ethyl acetate/hexane = 1/1 by a weight ratio) to obtain benzyl 4-(2-((1-tert-butoxycarbonyl)azetidin-3-ylol)acetyl)piperazin-1-carboxylate (27.61 g, 63.68 mmol, yield 88%). The synthesis of the benzyl 4-(2-((1-tert-butoxycarbonyl)azetidin-3-ylol)acetyl)piperazin-1-carboxylate thus obtained was confirmed through ¹H NMR analysis.

¹H NMR (400MHz, MeOD) δ 7.32 (d, J=16.2 Hz, 5H), 5.07 (s, 2H), 4.38 (d, J=4.6 Hz, 1H), 4.20 (d, J=27.2 Jz. 4H), 3.93 (s, 2H), 3.63-3.49 (m, 2H), 3.43 (s, 6H), 1.64-1.35 (m, 9H) .

### (2) Step B: Preparation of tert-butyl 4-(2-(azetidin-3-ylol)acetyl)piperazin-1-carboxylate

Benzyl 4-(2-((1-tert-butoxycarbonyl)azetidin-3-ylol)acetyl)piperazin-1-carboxylate (51.01 g, 117.66 mmol) prepared in Step A was dissolved in 300 ml of methanol, and palladium/charcoal (10 wt%, 3 g) was added thereto, followed by stirring at room temperature for 18 hours. After finishing the reaction, the resultant was filtered using celite and washed with methanol. The filtrate was concentrated under a reduced pressure to obtain tert-butyl 4-(2-(azetidin-3-ylol)acetyl)piperazin-1-carboxylate (38.81 g, 129.63 mmol, yield 110%). The synthesis of the tert-butyl 4-(2-(azetidin-3-ylol)acetyl)piperazin-1-carboxylate thus obtained was confirmed through ¹H NMR analysis.

¹H NMR (400MHz, MeOD) δ 4.48-4.32 (m, 1H), 4.19 (s, 2H), 32.77-3.66 (m, 2H), 3.63 (t, J=7.3 Hz, 2H), 3.55 (s, 2H), 3.50-3.38 (m, 6H), 1.64-1.38 (m, 9H).

### Preparation Example 9

Steps A and B in Preparation Example 8 were performed in sequence except for using benzyl 4-hydroxypiperidin-1-carboxylate (750 mg, 3.19 mmol) instead of benzyl 3-hydroxyazetidin-1-carboxylate in Step A to prepare tert-butyl 4-(2-(piperidin-2-4-ylol)acetyl)piperazin-1-carboxylate (800 mg, 2.44 mmol, yield 76%) of Formula i. The synthesis of the tert-butyl 4-(2-(piperidin-2-4-ylol)acetyl)piperazin-1-carboxylate thus obtained was confirmed through ¹H NMR analysis.

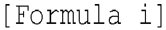

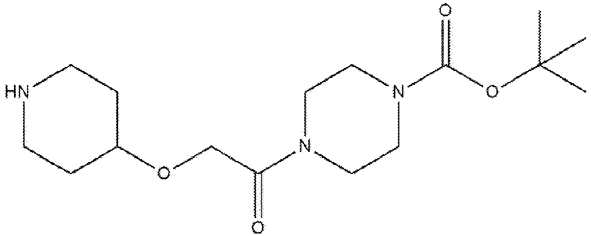

¹H NMR (400MHz, CDCl₃) δ 7.37-7.28 (m, 5H), 5.14 (s, 2H), 4.61 (s, 2H), 3.82 (m, 2H), 3.59-3.44 (m, 9H), 3.25 (m, 2H), 1.88 (m, 2H), 1.59 (m, 2H), 1.49 (s, 9H).

### Preparation Example 10

Tert-butyl 4-((((1R,5S,6R)-3-azabicyclo[3.1.0]hexan-5-yl)methyl)sulfonyl)piperazin-1-carboxylate of Formula j was prepared by performing Steps A to E in sequence.

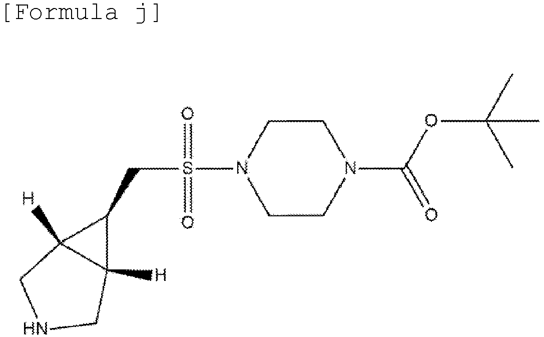

### (1) Step A: Preparation of benzyl (1R,5S,6R)-6-(((methylsulfonyl)oxy)methyl)-3-azabicyclo[3.1.0]hexan-3-carboxylate

Benzyl (1R,5S,6R)-6-(hydroxymethyl)-3-azabicyclo[3.1.0]hexan-3-carboxylate (862 mg, 3.49 mmol) was dissolved in 20 ml of dichloromethane and cooled using ice water, and triethylamine (0.73 ml, 5.23 mmol) was added thereto. Methanesulfonyl chloride (0.35 ml, 4.53 mmol) was slowly added thereto, followed by stirring for 1 hour. After finishing the reaction, water was added to the reaction solution, and extraction was performed with dichloromethane. An organic layer was dried over anhydrous magnesium sulfate and filtered. The filtrate was concentrated under a reduced pressure to obtain benzyl (1R,5S,6R)-6-(((methylsulfonyl)oxy)methyl)-3-azabicyclo[3.1.0]hexan-3-carboxylate (1.174 g, yield 99%). This product was used in the next reaction without a separation purification process. The synthesis of the benzyl (1R,5S,6R)-6-(((methylsulfonyl)oxy)methyl)-3-azabicyclo[3.1.0]hexan-3-carboxylate thus obtained was confirmed through ¹H NMR analysis.

¹H NMR (500MHz, CDCl₃) δ 7.32-7.40 (m, 5H), 5.13 (d, J=2.1 Hz, 2H), 4.10-4.20 (m, 2H), 3.73 (m, 2H), 3.46-3.50 (m, 2H), 3.04 (s, 3H), 1.66 (s, 2H), 1.12-1.16 (m, 1H).

### (2) Step B: Preparation of benzyl (1R,5S,6R)-6-((acetylthio)methyl)-3-azabicyclo[3.1.0]hexan-3-carboxylate

Benzyl (1R,5S,6R)-6-(((methylsulfonyl)oxy)methyl)-3-azabicyclo[3.1.0]hexan-3-carboxylate (135 mg, 0.145 mmol) prepared in Step A, and potassium thioacetate (62 mg, 0.539 mmol) were dissolved in acetonitrile, followed by stirring at 60°C for 2 hours. After finishing the reaction, the resultant was extracted with ethyl acetate and washed using water and a saturated sodium chloride solution, and an organic layer was dried over anhydrous magnesium sulfate and filtered. The filtrate was concentrated under a reduced pressure and purified by column chromatography (ethyl acetate/hexane) to obtain benzyl (1R,5S,6R)-6-((acetylthio)methyl)-3-azabicyclo[3.1.0]hexan-3-carboxylate (93 mg, yield 73%). The synthesis of the benzyl (1R,5S, 6R)-6-((acetylthio)methyl)-3-azabicyclo[3.1.0]hexan-3-carboxylate thus obtained was confirmed through ¹H NMR analysis.

¹H NMR (400MHz, CDCl₃) δ 7.27-7.36 (m, 5H), 5.08 (d, J=2.3 Hz, 2H), 3.63 (dd, J=17.8, 11.0 Hz, 2H), 3.38 (t, J=9.1 Hz, 2H), 2.80-2.87 (m, 2H), 2.32 (s, 3H), 1.46 (s, 2H), 0.81-0.86 (m, 1H).

### (3) Step C: Preparation of benzyl (1R,5S,6R)-6-((chlorosulfonyl)methyl)-3-azabicyclo[3.1.0]hexan-3-carboxylate

Benzyl (1R,5S,6R)-6-((acetylthio)methyl)-3-azabicyclo[3.1.0]hexan-3-carboxylate (93 mg, 0.305 mmol) prepared in Step B was dissolved in 10 ml of acetonitrile, and the resultant was cooled to 0°C. N-chlorosuccinimide (163 mg, 1.218 mmol) and a 1 N hydrochloric acid solution (0.365 ml, 0.365 mmol) were added thereto, followed by stirring at room temperature for 18 hours. After finishing the reaction, the resultant was concentrated under a reduced pressure, dissolved in ethyl acetate and washed with a saturated sodium chloride solution, and an organic layer was dried over anhydrous magnesium sulfate and filtered. The filtrate was concentrated under a reduced pressure to obtain benzyl (1R,5S,6R)-6-((chlorosulfonyl)methyl)-3-azabicyclo[3.1.0]hexan-3-carboxylate (151 mg), and this product was used in the next reaction without purification.

### (4) Step D: Preparation of benzyl (1R,5S,6R)-6-(((4-(tert-butoxycarbonyl)piperazin-1-yl)sulfonyl)methyl)-3-azabicyclo[3.1.0]hexan-3-carboxylate

Benzyl (1R,5S,6R)-6-((chlorosulfonyl)methyl)-3-azabicyclo[3.1.0]hexan-3-carboxylate (100 mg, 0.303 mmol) prepared in Step C was dissolved in 10 ml of dichloromethane and cooled to 0°C. Tert-butyl piperazin-1-carboxylate (68 mg, 0.364 mmol) and diisopropylethylamine (0.11 ml, 0.606 mmol) were added thereto, followed by stirring at room temperature for 3 hours. After finishing the reaction, the resultant was extracted with dichloromethane, and an organic layer was dried over anhydrous magnesium sulfate and filtered. The filtrate was concentrated under a reduced pressure and purified by column chromatography (ethyl acetate/hexane = 1/1 by a weight ratio) to obtain benzyl (1R,5S,6R)-6-(((4-(tert-butoxycarbonyl)piperazin-1-yl)sulfonyl)methyl)-3-azabicyclo[3.1.0]hexan-3-carboxylate (145 mg, yield 86%), and which was confirmed through ¹H NMR analysis.

¹H NMR (400MHz, CDCl₃) δ 7.27-7.37 (m, 5H), 5.09 (m, 2H), 3.70 (dd, J=15.6, 11.0 Hz, 2H), 3.42-3.50 (m, 6H), 3.24 (t, J=5.0 Hz, 4H), 2.88 (m, 2H), 1.58-1.62 (m, 2H), 1.48 (d, J=14.6 Hz, 9H), 0.92-0.97 (m, 1H).

### (5) Step E: Preparation of tert-butyl 4-((((1R,5S,6R)-3-azabicyclo[3.1.0]hexan-6-yl)methyl)sulfonyl)piperazin-1-carboxylate

Benzyl (1R,5S,6R)- 6-(((4-(tert-butoxycarbonyl)piperazin-1-yl)sulfonyl)methyl)-3-azabicyclo[3.1.0]hexan-3-carboxylate (125 mg, 0.261 mmol) prepared in Step D was dissolved in 10 ml of methanol, and 10 wt% of palladium/charcoal (27 mg, 0.026 mmol) was added thereto, followed by stirring together with a hydrogen gas. After finishing the reaction, the resultant was filtered using celite and concentrated under a reduced pressure to obtain tert-butyl 4-((((1R,5S,6R)-3-azabicyclo[3.1.0]hexan-6-yl)methyl)sulfonyl)piperazin-1-carboxylate (89 mg, yield 99%), and which was confirmed through ¹H NMR analysis.

¹H NMR (400MHz, CDCl₃) δ 3.50 (t, J=5.0 Hz, 4H), 3.25 (t, J=4.8 Hz, 4H), 3.00-3.05 (m, 2H), 2.90 (d, J=7.3 Hz, 4H), 1.59 (br, 2H), 1.46 (s, 9H), 0.90-0.95 (m, 1H).

### Example 1: Preparation of (S)-2-((1-(2-(2-methylazetidin-1-yl)-6-(trifluoromethyl)pyrimidin-4-yl)azetidin-3-yl)oxy)-1-(piperazin-1-yl)ethan-1-one

As in Reaction 1, the processes of Steps A to D were performed in sequence to prepare the title compound of Formula 1-1.

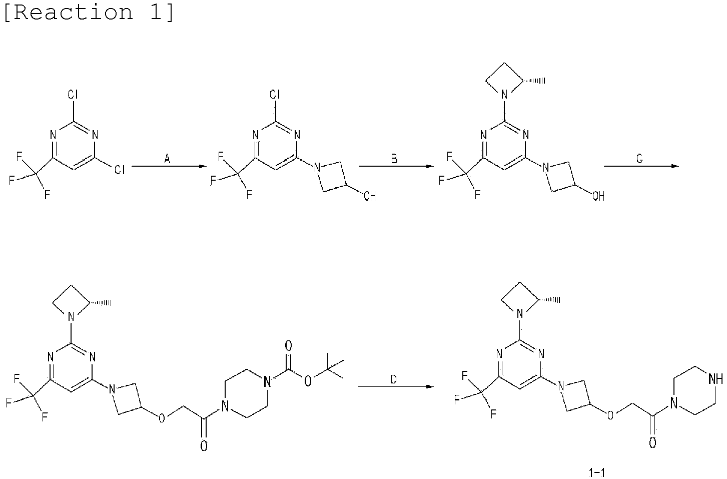

### (1) Step A: Preparation of 1-(2-chloro-6-(trifluoromethyl)pyrimidin-4-yl)azetidin-3-ol

2,4-Dichloro-6-(trifluoromethyl)pyrimidine (500 mg, 2.30 mmol), azetidin-3-ol hydrochloride (252 mg, 2.30 mmol) and diisopropylethylamine (1.2 ml, 6.91 mmol) were dissolved in 50 ml of dichloromethane, followed by stirring at room temperature for 2 hours. After finishing the reaction, a 1 N aqueous hydrochloric acid solution was added to the reaction solution, and the resultant was extracted with dichloromethane. An organic layer was dried over anhydrous magnesium sulfate and filtered. The filtrate was concentrated under a reduced pressure and purified by column chromatography to obtain 1-(2-chloro-6-(trifluoromethyl)pyrimidin-4-yl)azetidin-3-ol (550 mg, 2.17 mmol, yield 94%) (mass [M+H] = 254.6), and which was confirmed through ¹H NMR analysis.

¹H NMR (500MHz, CDCl₃) δ 6.40 (d, J=12.5 Hz, 1H), 4.90 (d, J=4.9 Hz, 1H), 4.63-4.27 (m, 2H), 4.08 (s, 2H), 2.43 (d, J=5.2 Hz, 1H).

### (2) Step B: Preparation of (S)-1-(2-(2-methylazetidin-1-yl)-6-(trifluoromethyl)pyrimidin-4-yl)azetidin-3-ol

1-(2-Chloro-6-(trifluoromethyl)pyrimidin-4-yl)azetidin-3-ol (200 mg, 0.78 mmol) prepared in Step A, (2S)-2-methylazetidine[(1R,4S)-7,7-methyl-2-oxo-norbornan-1-yl]methanesulfonate (285 mg, 0.94 mmol) and triethylamine (236 mg, 2.36 mmol) were dissolved in 10 ml of acetonitrile, followed by stirring at 80°C for 1 hour. After finishing the reaction, the temperature was cooled to room temperature, and the resultant was concentrated under a reduced pressure, dissolved in ethyl acetate, and washed with a saturated chloride solution. An organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain (S)-1-(2-(2-methylazetidin-1-yl)-6-(trifluoromethyl)pyrimidin-4-yl)azetidin-3-ol (219 mg, 0.75 mmol, yield 96%), which was confirmed through ¹H NMR analysis.

¹H NMR (500MHz, CDCl₃) δ 5.84 (d, J=2.4 Hz, 1H), 4.85-4.70 (m, 1H), 4.51-4.37 (m, 1H), 4.37-4.21 (m, 2H), 4.03 (td, J=8.9, 5.0 Hz, 1H), 3.99-3.81 (m, 3H), 2.47-2.30 (m, 1H), 2.23 (d, J=6.1 Hz, 1H), 2.02-1.85 (m, 1H), 1.48 (t, J=5.6 Hz, 3H) .

### (3) Step C: Preparation of tert-butyl (S)-4-(2-((1-(2-(2-methylazetidin-1-yl)-6-(trifluoromethyl)pyrimidin-4-yl)azetidin-3-yl)oxy)acetyl)piperazin-1-carboxylate

(S)-1-(2-(2-methylazetidin-1-yl)-6-(trifluoromethyl)pyrimidin-4-yl)azetidin-3-ol (50 mg, 0.17 mmol) prepared in Step B was dissolved in 10 ml of dimethylformamide, followed by cooling to 0°C. Sodium hydride (60%, 8 mg, 0.20 mmol) was added thereto, followed by stirring for 30 minutes. Tert-butyl 4-(2-bromoacetyl)piperazin-1-carboxylate (64 mg, 0.21 mmol) prepared in Preparation Example 7 was added thereto, followed by stirring at room temperature for 16 hours. After finishing the reaction, the reaction solution was concentrated under a reduced pressure. The resultant was dissolved in ethyl acetate and washed with a saturated sodium chloride solution. An organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl (S)-4-(2-((1-(2-(2-methylazetidin-1-yl)-6-(trifluoromethyl)pyrimidin-4-yl)azetidin-3-yl)oxy)acetyl)piperazin-1-carboxylate (29 mg, 0.05 mmol, yield 21%), which was confirmed through ¹H NMR analysis.

¹H NMR (500MHz, CDCl₃) δ 5.84 (d, J=3.1 Hz, 1H), 4.51 (dd, J=10.4, 4.3 Hz, 1H), 4.48-4.36 (m, 1H), 4.32-4.21 (m, 2H), 4.18 (s, 2H), 4.09-3.95 (m, 2H), 3.66-3.57 (m, 2H), 3.55-3.31 (m, 8H), 2.46-2.30 (m, 1H), 2.01-1.85 (m, 1H), 1.49-1.40 (m, 12H).

### (4) Step D: Preparation of (S)-2-((1-(2-(2-methylazetidin-1-yl)-6-(trifluoromethyl)pyrimidin-4-yl)azetidin-3-yl)oxy)-1-(piperazin-1-yl)ethan-1-one

Tert-butyl (S)-4-(2-((1-(2-(2-methylazetidin-1-yl)-6-(trifluoromethyl)pyrimidin-4-yl)azetidin-3-yl)oxy)acetyl)piperazin-1-carboxylate (29 mg, 0.056 mmol) prepared in Step C and 1 ml of trifluoroacetic acid were dissolved in 5 ml of dichloromethane, followed by stirring for 18 hours. After finishing the reaction, the reaction solution was dried under a reduced pressure, ethyl acetate and an aqueous saturated ammonium solution were added thereto, and the resultant was extracted with ethyl acetate. An organic layer was dried over anhydrous magnesium sulfate and filtered. The filtrate was concentrated under a reduced pressure, and the concentrate under a reduced pressure was purified by column chromatography (dichloromethane/7 N ammonia methanol = 10/1 by a weight ratio) to obtain (S)-2-((1-(2-(2-methylazetidin-1-yl)-6-(trifluoromethyl)pyrimidin-4-yl)azetidin-3-yl)oxy)-1-(piperazin-1-yl)ethan-1-one (23 mg, 0.056 mmol, yield 100%), which was confirmed through ¹H NMR analysis.

¹H NMR (500MHz, MeOH-d₄) δ 5.79 (d, J=2.7 Hz, 1H), 4.59-4.35 (m, 2H), 4.33-4.18 (m, 4H), 4.07-3.83 (m, 4H), 3.64-3.37 (m, 4H), 2.89-2.74 (m, 4H), 2.49-2.32 (m, 1H), 1.96 (d, J=6.4 Hz, 1H), 1.55-1.39 (m, 3H).

### Example 2: Preparation of (S)-2-((1-(5-chloro-2-(2-methylazetidin-1-yl)-6-(trifluoromethyl)pyrimidin-4-yl)azetidin-3-yl)oxy)-1-(piperazin-1-yl)ethan-1-one

As in Reaction 2, the processes of Steps A to D were performed in sequence to prepare the title compound of Formula 1-2.

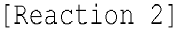

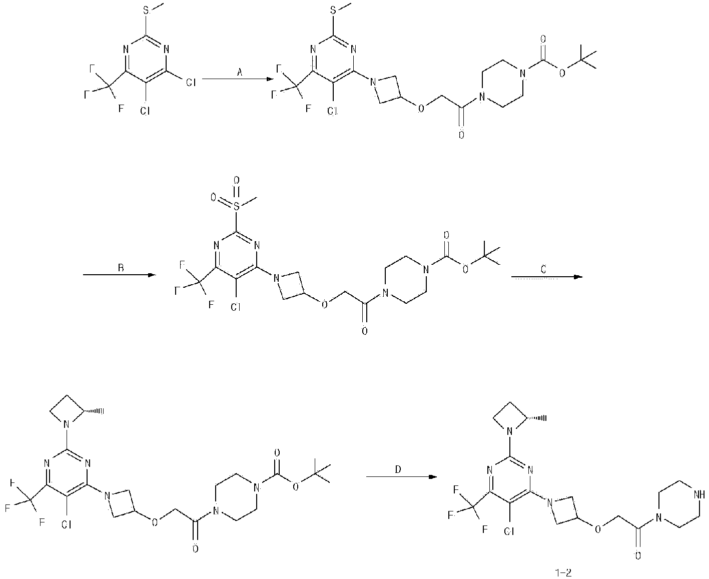

### (1) Step A: Preparation of tert-butyl 4-(2-((1-(5-chloro-2-(methylthio)-6-(trifluoromethyl)pyrimidin-4-yl)azetidin-3-yl)oxy)acetyl)piperazin-1-carboxylate

4,5-Dichloro-2-(methylthio)-6-(trifluoromethyl)pyrimidine (1.0 g, 3.80 mmol), tert-butyl 4-(2-(azetidin-3-yloxy)acetyl)piperazin-1-carboxylate (1.7 g, 5.70 mmol) and diisopropylethylamine (2.0 ml, 11.4 mmol) were dissolved in 100 ml of dichloromethane, followed by stirring at room temperature for 2 hours. After finishing the reaction, an excessive amount of water was added to the reaction solution, and the resultant was extracted with dichloromethane. An organic layer was washed with an aqueous saturated chloride solution, and the organic layer was dried over anhydrous magnesium sulfate and filtered. The filtrate was concentrated under a reduced pressure and purified by column chromatography (ethyl acetate/hexane = 1/1 by a weight ratio) to obtain tert-butyl 4-(2-((1-(5-chloro-2-(methylthio)-6-(trifluoromethyl)pyrimidin-4-yl)azetidin-3-yl)oxy)acetyl)piperazin-1-carboxylate (1.2 g, 2.28 mmol, yield 60%), which was confirmed through ¹H NMR analysis.

¹H NMR (500MHz, CDCl₃) δ 4.62 (br, s, 2H), 4.67 (m, 1H), 4.36 (br, s, 2H), 4.18 (s, 2H), 3.58 (br, s, 2H), 3.44 (br, s, 6H), 2.47 (s, 3H), 1.46 (s, 9H).

### (2) Step B: Preparation of tert-butyl 4-(2-((1-(5-chloro-2-(methylsulfonyl)-6-(trifluoromethyl)pyrimidin-4-yl)azetidin-3-yl)oxy)acetyl)piperazin-1-carboxylate

Tert-butyl 4-(2-((1-(5-chloro-2-(methylthio)-6-(trifluoromethyl)pyrimidin-4-yl)azetidin-3-yl)oxy)acetyl)piperazin-1-carboxylate (1.2 g, 2.28 mmol) prepared in Step A and 3-chloroperbenzoic acid (70%, 1.41 g, 5.70 mmol) were dissolved in 100 ml of dichloromethane, followed by stirring at room temperature for 10 hours. After finishing the reaction, a saturated sodium thiosulfate solution and a saturated sodium bicarbonate solution were added to the reaction solution in sequence, and the resultant was extracted with ethyl acetate. An organic layer was washed with an aqueous saturated sodium chloride solution, dried over anhydrous magnesium sulfate and filtered. The filtrate was concentrated under a reduced pressure, and the concentrate under a reduced pressure was purified by column chromatography (methanol/dichloromethane = 1/9 by a weight ratio) to obtain tert-butyl 4-(2-((1-(5-chloro-2-(methylsulfonyl)-6-(trifluoromethyl)pyrimidin-4-yl)azetidin-3-yl)oxy)acetyl)piperazin-1-carboxylate (1.2 g, 2.28 mmol, yield 94%), which was confirmed through ¹H NMR analysis.

¹H NMR (500MHz, CDCl₃) δ 5.11-4.90 (1H), 4.48-4.53 (m, 2H), 4.21 (s, 2H), 3.58 (s, 2H), 3.39-3.44 (m, 6H), 3.29 (d, J=6.9 Hz, 3H), 1.46 (s, 9H).

### (3) Step C: Preparation of tert-butyl (S)-4-(2-((1-(5-chloro-2-(2-methylazetidin-1-yl)-6-(trifluoromethyl)pyrimidin-4-yl)azetidin-3-yl)oxy)acetyl)piperazin-1-carboxylate

Tert-butyl 4-(2-((1-(5-chloro-2-(methylsulfonyl)-6-(trifluoromethyl)pyrimidin-4-yl)azetidin-3-yl)oxy)acetyl)piperazin-1-carboxylate (200 mg, 0.36 mmol) prepared in Step B, (2S)-2-methylazetidine[(1R,4S)-7,7-dimethyl-2-oxo-norbornan-1-yl]methanesulfonate (253 mg, 0.54 mmol) and potassium carbonate (149 mg, 1.08 mmol) were dissolved in 20 ml of acetonitrile, followed by stirring at 80°C for 2 hours. After finishing the reaction, an excessive amount of water was added to the reaction solution, and the resultant was extracted with ethyl acetate. An organic layer was washed with an aqueous saturated sodium chloride solution, dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl (S)-4-(2-((1-(5-chloro-2-(2-methylazetidin-1-yl)-6-(trifluoromethyl)pyrimidin-4-yl)azetidin-3-yl)oxy)acetyl)piperazin-1-carboxylate (140 mg, 0.26 mmol, yield 72%), which was confirmed through ¹H NMR analysis.

¹H NMR (500MHz, CDCl₃) δ 4.59 (m, 2H), 4.45 (m, 2H), 4.26 (m, 2H), 4.19 (s, 2H), 4.01 (m, 1H), 3.03 (m, 1H), 3.61 (s, 2H), 3.48 (br s, 6H), 2.40 (m, 1H), 1.95 (m, 1H), 1.60 (s, 9H), 1.59 (d, 3H).

### (4) Step D: Preparation of (S)-4-(2-((1-(5-chloro-2-(2-methylazetidin-1-yl)-6-(trifluoromethyl)pyrimidin-4-yl)azetidin-3-yl)oxy)-1-(piperazin-1-yl)ethan-1-one

Tert-butyl (S)-4-(2-((1-(5-chloro-2-(2-methylazetidin-1-yl)-6-(trifluoromethyl)pyrimidin-4-yl)azetidin-3-yl)oxy)acetyl)piperazin-1-carboxylate (140 mg, 0.26 mmol) prepared in Step C and 1 ml of trifluoroacetic acid were dissolved in 3 ml of dichloromethane, followed by stirring at room temperature for 10 hours. After finishing the reaction, a saturated sodium bicarbonate solution was added to the reaction solution, and the resultant was extracted with ethyl acetate. An organic layer was washed with an aqueous saturated sodium chloride solution, dried over anhydrous magnesium sulfate and filtered. The filtrate was concentrated under a reduced pressure, and the concentrate under a reduced pressure was purified by column chromatography (7 N ammonia methanol solution/dichloromethane = 1/9 by a weight ratio) to obtain (S)-4-(2-((1-(5-chloro-2-(2-methylazetidin-1-yl)-6-(trifluoromethyl)pyrimidin-4-yl)azetidin-3-yl)oxy)-1-(piperazin-1-yl)ethan-1-one (100 mg, 0.22 mmol, yield 87%), which was confirmed through ¹H NMR analysis.

¹H NMR (500MHz, CDCl₃) δ 4.50 (d, J=6.4 Hz, 2H), 4.36-4.44 (m, 2H), 4.23 (d, J=10.1 Hz, 2H), 4.16 (s, 2H), 3.86-4.00 (m, 2H), 3.64 (s, 2H), 3.51 (s, 2H), 2.91 (s, 4H), 2.32-2.41 (m, 1H), 1.88-1.96 (m, 1H), 1.46 (t, J=6.4 Hz, 3H).

### Example 3: Preparation of (S)-2-((1-(7,7-difluoro-2-(2-methylazetidin-1-yl)-6,7-dihydro-5H-cyclopenta[d]pyrimidin-4-yl)azetidin-3-yl)oxy)-1-(piperazin-1-yl)ethan-1-one

As in Reaction 3, the processes of Steps A to E were performed in sequence to prepare the title compound of Formula 1-3.

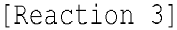

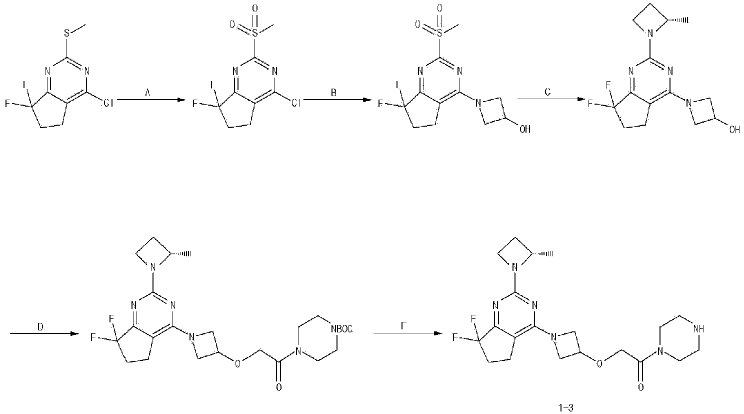

### (1) Step A: Preparation of 4-chloro-7,7-difluoro-2-(methylsulfonyl)-6,7-dihydro-5H-cyclopenta[d]pyrimidine

4-Chloro-7,7-difluoro-2-(methylthio)-6,7-dihydro-5H-cyclopenta[d]pyrimidine (3.00 g, 12.73 mmol), and 3-chloroperbenzoic acid (5.51 g, 31.82 mmol) was dissolved in 200 ml of dichloromethane, followed by stirring at room temperature for 16 hours. After finishing the reaction, a saturated sodium thiosulfate solution and a saturated sodium bicarbonate solution were added in sequence to the reaction solution, and the resultant was extracted with ethyl acetate. An organic layer was washed with an aqueous saturated chloride solution, dried over anhydrous magnesium sulfate and filtered. The filtrate was concentrated under a reduced pressure and purified by column chromatography (ethyl acetate/hexane = 1/1 by a weight ratio) to obtain 4-chloro-7,7-difluoro-2-(methylsulfonyl)-6,7-dihydro-5H-cyclopenta[d]pyrimidine (3.21 g, 11.94 mmol, yield 93%), which was confirmed through ¹H NMR analysis.

¹H NMR (500MHz, CDCl₃) δ 3.44 (d, J=12.5 Hz, 3H), 3.34-3.11 (m, 2H), 2.88-2.68 (m, 2H).

### (2) Step B: Preparation of 1-(7,7-difluoro-2-(methylsulfonyl)-6,7-dihydro-5H-cyclopenta[d]pyrimidin-4-yl)azetidin-3-ol

4-Chloro-7,7-difluoro-2-(methylsulfonyl)-6,7-dihydro-5H-cyclopenta[d]pyrimidine (348 mg, 1.29 mmol) prepared in Step A, azetidin-3-ol hydrochloride (156 mg, 1.42 mmol) and triethylamine (393 mg, 3.88 mmol) were dissolved in 20 ml of dichloromethane, followed by stirring at room temperature for 2 hours. After finishing the reaction, a 1 N aqueous hydrochloric acid solution was added to the reaction solution, and the resultant was extracted with dichloromethane. An organic layer was dried over anhydrous magnesium sulfate and filtered. The filtrate was concentrated under a reduced pressure, and the concentrate under a reduced pressure was purified by column chromatography (methanol/dichloromethane = 1/10 by a weight ratio) to obtain 1-(7,7-difluoro-2-(methylsulfonyl)-6,7-dihydro-5H-cyclopenta[d]pyrimidin-4-yl)azetidin-3-ol (257 mg, 0.84 mmol, yield 65%), which was confirmed through ¹H NMR analysis.

¹H NMR (500MHz, CDCl₃) δ 4.89 (s, 1H), 4.66 (s, 1H), 4.31 (s, 1H), 3.35 (s, 3H), 3.03 (d, J=7.6 Hz, 2H), 2.72-2.55 (m, 2H), 1.37-1.26 (m, 2H).

### (3) Step C: Preparation of (S)-1-(7,7-difluoro-2-(2-methylazetidin-1-yl)-6,7-dihydro-5H-cyclopenta[d]pyrimidin-4-yl)azetidin-3-ol

1-(7,7-Difluoro-2-(methylsulfonyl)-6,7-dihydro-5H-cyclopenta[d]pyrimidin-4-yl)azetidin-3-ol (257 mg, 0.84 mmol) prepared in Step B, (2S)-2-methylazetidine[(1R,4S)-7,7-dimethyl-2-oxo-norbornan-1-yl]methanesulfonate (306 mg, 1.01 mmol) and potassium carbonate (348 mg, 2.52 mmol) were dissolved in 30 ml of acetonitrile, followed by stirring at 80°C for 1 hour. After finishing the reaction, the reaction solution was concentrated under a reduced pressure, a saturated sodium bicarbonate solution was added thereto, and the resultant was extracted with ethyl acetate. An organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain (S)-1-(7,7-difluoro-2-(2-methylazetidin-1-yl)-6,7-dihydro-5H-cyclopenta[d]pyrimidin-4-yl)azetidin-3-ol (182 mg, 0.61 mmol, yield 72%), which was confirmed through ¹H NMR analysis.

¹H NMR (500MHz, MeOH-d₄) δ 4.73-4.59 (m, 1H), 4.51-4.33 (m, 3H), 4.00 (td, J=8.7, 4.9 Hz, 3H), 3.88 (dd, J=16.0, 8.7 Hz, 1H), 2.85 (d, J=4.3 Hz, 2H), 2.55-2.30 (m, 3H), 2.01-1.84 (m, 1H), 1.52 (dd, J=48.1, 6.3 Hz, 3H).

### (4) Step D: Preparation of tert-butyl (S)-4-(2-((1-(7,7-difluoro-2-(2-methylazetidin-1-yl)-6,7-dihydro-5H-cyclopenta[d]pyrimidin-4-yl)azetidin-3-yl)oxy)acetyl)piperazin-1-carboxylate

(S)-1-(7,7-difluoro-2-(2-methylazetidin-1-yl)-6,7-dihydro-5H-cyclopenta[d]pyrimidin-4-yl)azetidin-3-ol (182 mg, 0.61 mmol) prepared in Step C was dissolved in 10 ml of dimethylformamide and cooled to 0°C. Sodium hydride (60%, 30 mg, 0.73 mmol) was added thereto, followed by stirring for 30 minutes. Tert-butyl 4-(2-bromoacetyl)piperazin-1-carboxylate (283 mg, 0.92 mmol) prepared in Preparation Example 7 was added thereto, followed by stirring at room temperature for 16 hours. After finishing the reaction, the reaction solution was concentrated under a reduced pressure, an aqueous saturated ammonium chloride solution was added, and the resultant was extracted with ethyl acetate. An organic layer was dried over anhydrous magnesium sulfate and filtered. The filtrate was concentrated under a reduced pressure, and the concentrate under a reduced pressure was purified by column chromatography (ethyl acetate/hexane = 2/1 by a weight ratio) to obtain tert-butyl (S)-4-(2-((1-(7,7-difluoro-2-(2-methylazetidin-1-yl)-6,7-dihydro-5H-cyclopenta[d]pyrimidin-4-yl)azetidin-3-yl)oxy)acetyl)piperazin-1-carboxylate (171 mg, 0.32 mmol, yield 53%), which was confirmed through ¹H NMR analysis.

¹H NMR (500MHz, CDCl₃) δ 4.58-4.47 (m, 1H), 4.39 (s, 1H), 4.20 (s, 2H), 4.14 (s, 3H), 4.04-3.90 (m, 1H), 3.76-3.56 (m, 2H), 3.48 (s, 8H), 2.80 (d, J=4.3 Hz, 2H), 2.57-2.42 (m, 2H), 2.42-2.29 (m, 1H), 2.01-1.86 (m, 1H), 1.50 (d, J=6.4 Hz, 12H) .

### (5) Step E: Preparation of (S)-2-((1-(7,7-difluoro-2-(2-methylazetidin-1-yl)-6,7-dihydro-5H-cyclopenta[d]pyrimidin-4-yl)azetidin-3-yl)oxy)-1-(piperazin-1-yl)ethan-1-one

Tert-butyl (S)-4-(2-((1-(7,7-difluoro-2-(2-methylazetidin-1-yl)-6,7-dihydro-5H-cyclopenta[d]pyrimidin-4-yl)azetidin-3-yl)oxy)acetyl)piperazin-1-carboxylate (133 mg, 0.25 mmol) prepared in Step D and 1 ml of trifluoroacetic acid were dissolved in 5 ml of dichloromethane, followed by stirring for 18 hours. After finishing the reaction, the reaction solution was concentrated under a reduced pressure, an aqueous saturated ammonium chloride solution was added thereto, and the resultant was extracted with ethyl acetate. An organic layer was dried over anhydrous magnesium sulfate and filtered. The filtrate was concentrated under a reduced pressure, and the concentrate under a reduced pressure was purified by column chromatography (dichloromethane/7 N ammonia methanol = 10/1 by a weight ratio) to obtain (S)-2-((1-(7,7-difluoro-2-(2-methylazetidin-1-yl)-6,7-dihydro-5H-cyclopenta[d]pyrimidin-4-yl)azetidin-3-yl)oxy)-1-(piperazin-1-yl)ethan-1-one (68 mg, 0.16 mmol, yield 63%), which was confirmed through ¹H NMR analysis.

¹H NMR (500MHz, MeOH-d₄) δ 4.52-4.25 (m, 4H), 4.21 (s, 2H), 4.07 (s, 2H), 4.00-3.75 (m, 2H), 3.44 (dt, J=58.6, 4.9 Hz, 4H), 2.85-2.67 (m, 6H), 2.49-2.24 (m, 3H), 1.96-1.79 (m, 1H), 1.42 (d, J=6.4 Hz, 3H).

### Example 4: Preparation of (S)-2-((1-(8,8-difluoro-2-(2-(methylazetidin-1-yl)-5,6,7,8-tetrahydroquinazolin-4-yl)azetidin-3-yl)oxy)-1-(piperazin-1-yl)ethan-1-one

As in Reaction 4, the processes of Steps A to E were performed in sequence to prepare the title compound of Formula 1-4.

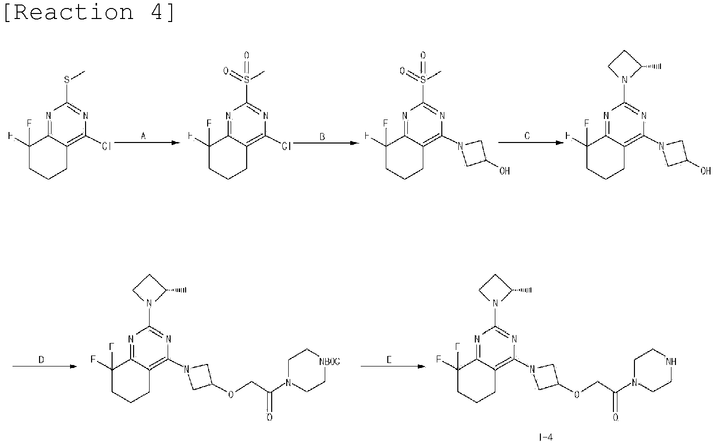

### (1) Step A: Preparation of 4-chloro-8,8-difluoro-2-(methylsulfonyl)-5,6,7,8-tetrahydroquinazoline

The same procedure in Step A of Example 3 was performed except for using 4-chloro-8,8-difluoro-2-(methylthio)-5,6,7,8-tetrahydroquinazoline (89 mg, 0.35 mmol) instead of 4-chloro-7,7-difluoro-2-(methylthio)-6,7-dihydro-5H-cyclopenta[d]pyrimidine in Step A of Example 3, to prepare 4-chloro-8,8-difluoro-2-(methylsulfonyl)-5,6,7,8-tetrahydroquinazoline.

### (2) Step B: Preparation of 1-(8,8-difluoro-2-(methylsulfonyl)-5,6,7,8-tetrahydroquinazolin-4-yl)azetidin-3-ol

The same procedure in Step B of Example 3 was performed except for reacting 4-chloro-8,8-difluoro-2-(methylsulfonyl)-5,6,7,8-tetrahydroquinazoline prepared in Step A instead of 4-chloro-7,7-difluoro-2-(methylsulfonyl)-6,7-dihydro-5H-cyclopenta[d]pyrimidine with azetidin-3-ol hydrochloride (41 mg, 0.38 mmol) in Step B of Example 3, to prepare 1-(8,8-difluoro-2-(methylsulfonyl)-5,6,7,8-tetrahydroquinazolin-4-yl)azetidin-3-ol.

### (3) Step C: Preparation of (S)-1-(8,8-difluoro-2-(2-methylazetidin-1-yl)-5,6,7,8-tetrahydroquinazolin-4-yl)azetidin-3-ol

The same procedure in Step C of Example 3 was performed except for reacting 1-(8,8-difluoro-2-(methylsulfonyl)-5,6,7,8-tetrahydroquinazolin-4-yl)azetidin-3-ol prepared in Step B instead of 1-(7,7-difluoro-2-(methylsulfonyl)-6,7-dihydro-5H-cyclopenta[d]pyrimidin-4-yl)azetidin-3-ol with (2S)-2-methylazetidine[(1R,4S)-7,7-dimethyl-2-oxo-norbornan-1-yl]methanesulfonate (105 mg, 0.41 mmol) in Step C of Example 3, to prepare (S)-1-(8,8-difluoro-2-(2-methylazetidin-1-yl)-5,6,7,8-tetrahydroquinazolin-4-yl)azetidin-3-ol.

### (4) Step D: Preparation of tert-butyl (S)-4-(2-((1-(8,8-difluoro-2-(2-methylazetidin-1-yl)-5,6,7,8-tetrahydroquinazolin-4-yl)azetidin-3-yl)oxy)acetyl)piperazin-1-carboxylate

The same procedure in Step D of Example 3 was performed except for reacting (S)-1-(8,8-difluoro-2-(2-methylazetidin-1-yl)-5,6,7,8-tetrahydroquinazolin-4-yl)azetidin-3-ol prepared in Step C instead of (S)-1-(7,7-difluoro-2-(2-methylazetidin-1-yl)-6,7-dihydro-5H-cyclopenta[d]pyrimidin-4-yl)azetidin-3-ol with tert-butyl 4-(2-bromoacetyl)piperazin-1-carboxylate (105 mg, 0.34 mmol) prepared in Preparation Example 7 in Step D of Example 3, to prepare tert-butyl (S)-4-(2-((1-(8,8-difluoro-2-(2-methylazetidin-1-yl)-5,6,7,8-tetrahydroquinazolin-4-yl)azetidin-3-yl)oxy)acetyl)piperazin-1-carboxylate.

### (5) Step E: Preparation of (S)-2-((1-(8,8-difluoro-2-(2-methylazetidin-1-yl)-5,6,7,8-tetrahydroquinazolin-4-yl)azetidin-3-yl)oxy)-1-(piperazin-1-yl)ethan-1-one

The same procedure in Step E of Example 3 was performed except for using tert-butyl (S)-4-(2-((1-(8,8-difluoro-2-(2-methylazetidin-1-yl)-5,6,7,8-tetrahydroquinazolin-4-yl)azetidin-3-yl)oxy)acetyl)piperazin-1-carboxylate prepared in Step D instead of tert-butyl (S)-4-(2-((1-(7,7-difluoro-2-(2-methylazetidin-1-yl)-6,7-dihydro-5H-cyclopenta[d]pyrimidin-4-yl)azetidin-3-yl)oxy)acetyl)piperazin-1-carboxylate in Step E of Example 3, to prepare (S)-2-((1-(8,8-difluoro-2-(2-methylazetidin-1-yl)-5,6,7,8-tetrahydroquinazolin-4-yl)azetidin-3-yl)oxy)-1-(piperazin-1-yl)ethan-1-one (73 mg, 0.13 mmol, yield 39%), which was confirmed through ¹H NMR analysis.

¹H NMR (500MHz, MeOH-d₄) δ 4.49-4.30 (m, 4H), 4.24 (s, 2H), 4.19-4.01 (m, 2H), 4.02-3.92 (m, 1H), 3.85 (q, J=8.3 Hz, 1H), 3.63-3.50 (m, 2H), 3.43 (t, J=4.9 Hz, 2H), 2.89-2.73 (m, 4H), 2.55 (s, 2H), 2.43-2.31 (m, 1H), 2.31-2.10 (m, 2H), 2.00-1.89 (m, 1H), 1.85 (q, J=6.1 Hz, 2H), 1.55-1.37 (m, 3H).

### Example 5: Preparation of 2-(((2S,3R)-1-(7,7-difluoro-2-((S)-2-methylazetidin-1-yl)-6,7-dihydro-5H-cyclopenta[d]pyrimidin-4-yl)-2-methylazetidin-3-yl)oxy)-1-(piperazin-1-yl)ethan-1-one

As in Reaction 5, the processes of Steps A to E were performed in sequence to prepare the title compound of Formula 1-5.

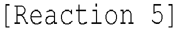

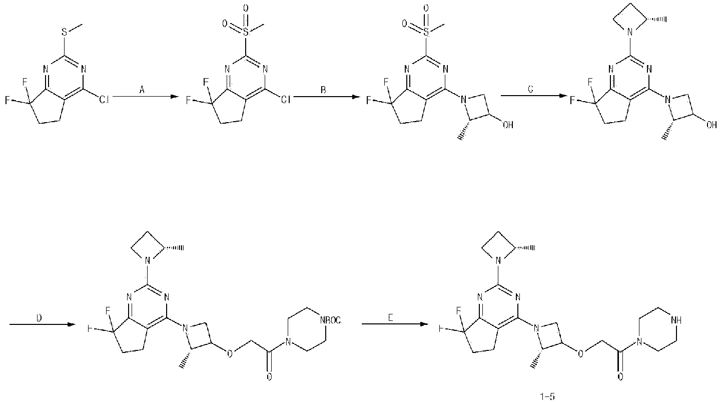

### (1) Step A: Preparation of 4-chloro-7,7-difluoro-2-(methylsulfonyl)-6,7-dihydro-5H-cyclopenta[d]pyrimidine

The same procedure in Step A of Example 3 was performed except for using 4-chloro-7,7-difluoro-2-(methylthio)-6,7-dihydro-5H-cyclopenta[d]pyrimidine in 94 mg (0.40 mmol) in Step A of Example 3, to prepare 4-chloro-7,7-difluoro-2-(methylsulfonyl)-6,7-dihydro-5H-cyclopenta[d]pyrimidine.

### (2) Step B: Preparation of (2S)-1-(7,7-difluoro-2-(methylsulfonyl)-6,7-dihydro-5H-cyclopenta[d]pyrimidin-4-yl)-2-methylazetidin-3-ol

The same procedure in Step B of Example 3 was performed except for using (2S,3R)-2-methylazetidin-3-ol(7,7-dimethyl-2-oxobicyclo[2.2.1]heptan-1-yl)methanesulfonate (142 mg, 0.44 mmol) instead of azetidin-3-ol hydrochloride in Step B of Example 3, to prepare (2S)-1-(7,7-difluoro-2-(methylsulfonyl)-6,7-dihydro-5H-cyclopenta[d]pyrimidin-4-yl)-2-methylazetidin-3-ol.

### (3) Step C: Preparation of (2S)-1-(7,7-difluoro-2-((S)-2-methylazetidin-1-yl)-6,7-dihydro-5H-cyclopenta[d]pyrimidin-4-yl)-2-methylazetidin-3-ol

The same procedure in Strep C of Example 3 was performed except for reacting (2S)-1-(7,7-difluoro-2-(methylsulfonyl)-6,7-dihydro-5H-cyclopenta[d]pyrimidin-4-yl)-2-methylazetidin-3-ol prepared in Step B instead of 1-(7,7-difluoro-2-(methylsulfonyl)-6,7-dihydro-5H-cyclopenta[d]pyrimidin-4-yl)azetidin-3-ol with (2S)-2-methylazetidine[(1R,4S)-7,7-dimethyl-2-oxo-norbornan-1-yl]methanesulfonate (134 mg, 0.44 mmol) in Step C of Example 3, to prepare (2S)-1-(7,7-difluoro-2-((S)-2-methylazetidin-1-yl)-6,7-dihydro-5H-cyclopenta[d]pyrimidin-4-yl)-2-methylazetidin-3-ol.

### (4) Step D: Preparation of tert-butyl 4-(2-(((2S)-1-(7,7-difluoro-2-((S)-2-methylazetidin-1-yl)-6,7-dihydro-5H-cyclopenta[d]pyrimidin-4-yl)-2-methylazetidin-3-yl)oxy)acetyl)piperazin-1-carboxylate

The same procedure in Step D of Example 3 was performed except for reacting (2S)-1-(7,7-difluoro-2-((S)-2-methylazetidin-1-yl)-6,7-dihydro-5H-cyclopenta[d]pyrimidin-4-yl)-2-methylazetidin-3-ol prepared in Step C instead of (S)-1-(7,7-difluoro-2-(2-methylazetidin-1-yl)-6,7-dihydro-5H-cyclopenta[d]pyrimidin-4-yl)azetidin-3-ol with tert-butyl 4-(2-bromoacetyl)piperazin-1-carboxylate (149 mg, 0.48 mmol) prepared in Preparation Example 7 in Step D of Example 3, to prepare tert-butyl 4-(2-(((2S)-1-(7,7-difluoro-2-((S)-2-methylazetidin-1-yl)-6,7-dihydro-5H-cyclopenta[d]pyrimidin-4-yl)-2-methylazetidin-3-yl)oxy)acetyl)piperazin-1-carboxylate.

### (5) Step E: Preparation of 2-(((2S,3R)-1-(7,7-difluoro-2-((S)-2-methylazetidin-1-yl)-6,7-dihydro-5H-cyclopenta[d]pyrimidin-4-yl)-2-methylazetidin-3-yl)oxy)-1-(piperazin-1-yl)ethan-1-one

The same procedure in Step E of Example 3 was performed except for using tert-butyl 4-(2-(((2S)-1-(7,7-difluoro-2-((S)-2-methylazetidin-1-yl)-6,7-dihydro-5H-cyclopenta[d]pyrimidin-4-yl)-2-methylazetidin-3-yl)oxy)acetyl)piperazin-1-carboxylate prepared in Step D instead of tert-butyl (S)-4-(2-((1-(7,7-difluoro-2-(2-methylazetidin-1-yl)-6,7-dihydro-5H-cyclopenta[d]pyrimidin-4-yl)azetidin-3-yl)oxy)acetyl)piperazin-1-carboxylate in Step E of Example 3, to obtain 2-(((2S,3R)-1-(7,7-difluoro-2-((S)-2-methylazetidin-1-yl)-6,7-dihydro-5H-cyclopenta[d]pyrimidin-4-yl)-2-methylazetidin-3-yl)oxy)-1-(piperazin-1-yl)ethan-1-one (59 mg, 0.13 mmol, yield 32%), which was confirmed through ¹H NMR analysis.

¹H NMR (500MHz, MeOH-d₄) δ 4.52-4.35 (m, 3H), 4.35-4.19 (m, 2H), 4.13-3.96 (m, 3H), 3.89 (q, J=8.2 Hz, 1H), 3.54 (d, J=4.3 Hz, 2H), 3.44 (s, 2H), 2.95-2.86 (m, 1H), 2.86-2.70 (m, 5H), 2.54-2.30 (m, 3H), 2.01-1.86 (m, 1H), 1.59-1.50 (m, 3H), 1.47 (dd, J=14.8, 6.3 Hz, 3H).

### Example 6: Preparation of 2-(((S)-1-(2-((S)-2-methylazetidin-1-yl)-6-(trifluoromethyl)pyrimidin-4-yl)pyrrolidin-3-yl)oxy)-1-(piperazin-1-yl)ethan-1-one

As in Reaction 6, the processes of Steps A to D were performed in sequence to prepare the title compound of Formula 1-6.

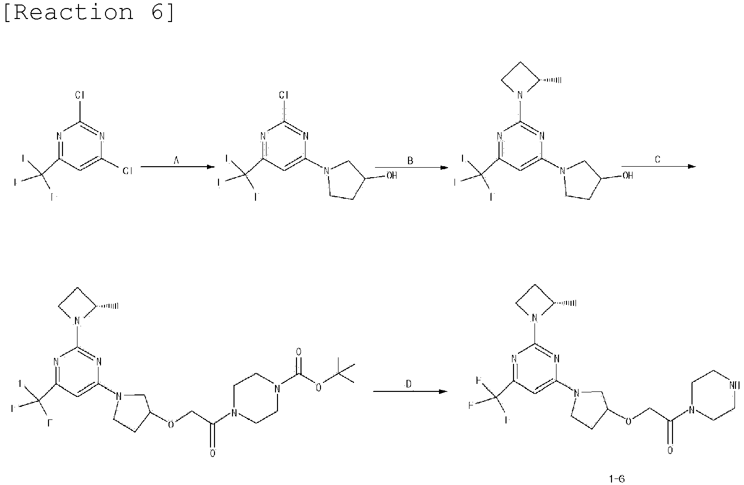

### (1) Step A: Preparation of 1-(2-chloro-6-(trifluoromethyl)pyrimidin-4-yl)pyrrolidin-3-ol

The same procedure in Step A of Example 1 was performed except for reacting 2,4-dichloro-6-(trifluoromethyl)pyrimidine (45 mg, 0.17 mmol) with (S)-pyrrolidin-3-ol (14 mg, 0.17 mmol) instead of azetidin-3-ol hydrochloride in Step A of Example 1, to prepare 1-(2-chloro-6-(trifluoromethyl)pyrimidin-4-yl)pyrrolidin-3-ol.

### (2) Step B: Preparation of 1-(2-((S)-2-methylazetidin-1-yl)-6-(trifluoromethyl)pyrimidin-4-yl)pyrrolidin-3-ol

The same procedure in Step B of Example 1 was performed except for reacting 1-(2-chloro-6-(trifluoromethyl)pyrimidin-4-yl)pyrrolidin-3-ol prepared in Step A instead of 1-(2-chloro-6-(trifluoromethyl)pyrimidin-4-yl)azetidin-3-ol with (S)-2-methylazetidine(7,7-dimethyl-2-oxobicyclo[2.2.1]heptan-1-yl)methanesulfonate (62 mg, 0.20 mmol) in Step B of Example 1, to prepare 1-(2-((S)-2-methylazetidin-1-yl)-6-(trifluoromethyl)pyrimidin-4-yl)pyrrolidin-3-ol.

### (3) Step C: Preparation of tert-butyl 4-(2-((1-(2-((S)-2-methylazetidin-1-yl)-6-(trifluoromethyl)pyrimidin-4-yl)pyrrolidin-3-yl)oxy)acetyl)piperazin-1-carboxylate

The same procedure in Step C of Example 1 was performed except for reacting 1-(2-((S)-2-methylazetidin-1-yl)-6-(trifluoromethyl)pyrimidin-4-yl)pyrrolidin-3-ol prepared in Step B instead of (S)-1-(2-(2-methylazetidin-1-yl)-6-(trifluoromethyl)pyrimidin-4-yl)azetidin-3-ol with tert-butyl 4-(2-bromoacetyl)piperazin-1-carboxylate (61 mg, 0.19 mmol) prepared in Preparation Example 7 in Step C of Example 1, to prepare tert-butyl 4-(2-((1-(2-((S)-2-methylazetidin-1-yl)-6-(trifluoromethyl)pyrimidin-4-yl)pyrrolidin-3-yl)oxy)acetyl)piperazin-1-carboxylate.

### (4) Step D: Preparation of 2-(((S)-1-(2-((s)-2-methylazetidin-1-yl)-6-(trifluoromethyl)pyrimidin-4-yl)pyrrolidin-3-yl)oxy)-1-(piperazin-1-yl)ethan-1-one

The same procedure in Step D of Example 1 was performed except for using tert-butyl 4-(2-((1-(2-((S)-2-methylazetidin-1-yl)-6-(trifluoromethyl)pyrimidin-4-yl)pyrrolidin-3-yl)oxy)acetyl)piperazin-1-carboxylate prepared in Step C instead of tert-butyl (S)-4-(2-((1-(2-(2-methylazetidin-1-yl)-6-(trifluoromethyl)pyrimidin-4-yl)azetidin-3-yl)oxy)acetyl)piperazin-1-carboxylate in Step D of Example 1, to prepare 2-(((S)-1-(2-((s)-2-methylazetidin-1-yl)-6-(trifluoromethyl)pyrimidin-4-yl)pyrrolidin-3-yl)oxy)-1-(piperazin-1-yl)ethan-1-one (28 mg, 0.07 mmol, yield 41%), which was confirmed through ¹H NMR analysis.

¹H NMR (500MHz, CDCl₃) δ 5.96 (s, 1H), 4.52-4.36 (m, 1H), 4.28 (s, 1H), 4.18 (s, 2H), 4.10-3.86 (m, 2H), 3.67-3.35 (m, 7H), 2.84 (d, J=4.1 Hz, 4H), 2.46-2.29 (m, 1H), 2.30-1.85 (m, 3H), 1.72 (s, 1H), 1.56-1.41 (m, 3H).

### Example 7: Preparation of 2-(((2S,3R)-1-(7,7-difluoro-2-((S)-2-methylazetidin-1-yl)-6,7-dihydro-5H-cyclopenta[d]pyrimidin-4-yl)pyrrolidin-3-yl)oxy)-1-(piperazin-1-yl)ethan-1-one

As in Reaction 7, the processes of Steps A to E were performed in sequence to prepare the title compound of Formula 1-7.

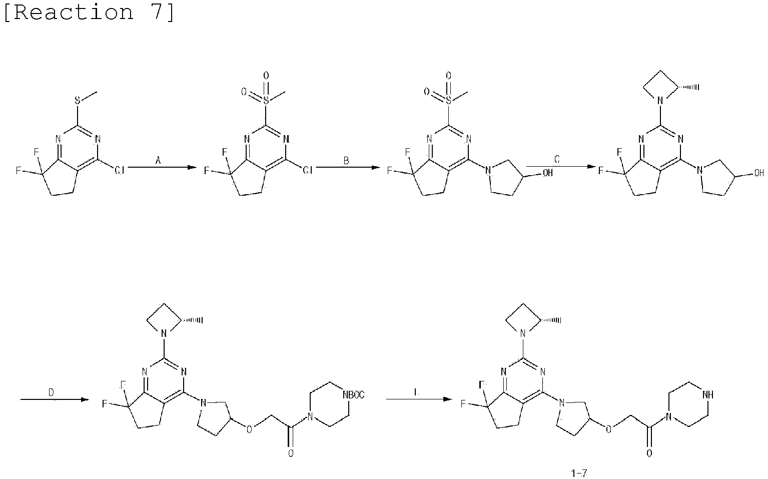

### (1) Step A: Preparation of 4-chloro-7,7-difluoro-2-(methylsulfonyl)-6,7-dihydro-5H-cyclopenta[d]pyrimidine

The same procedure in Step A of Example 3 was performed except for using 4-chloro-7,7-difluoro-2-(methylthio)-6,7-dihydro-5H-cyclopenta[d]pyrimidine in 141 mg (0.60 mmol) in Step A of Example 3, to prepare 4-chloro-7,7-difluoro-2-(methylsulfonyl)-6,7-dihydro-5H-cyclopenta[d]pyrimidine.

### (2) Step B: Preparation of 1-(7,7-difluoro-2-(methylsulfonyl)-6,7-dihydro-5H-cyclopenta[d]pyrimidin-4-yl)pyrrolidin-3-ol

The same procedure in Step B of Example 3 was performed except for using (S)-pyrrolidin-3-ol (53 mg, 0.61 mmol) instead of azetidin-3-ol hydrochloride in Step B of Example 3, to prepare 1-(7,7-difluoro-2-(methylsulfonyl)-6,7-dihydro-5H-cyclopenta[d]pyrimidin-4-yl)pyrrolidin-3-ol.

### (3) Step C: Preparation of 1-(7,7-difluoro-2-((S)-2-methylsulfonyl-1-yl)-6,7-dihydro-5H-cyclopenta[d]pyrimidin-4-yl)pyrrolidin-3-ol

The same procedure in Step C of Example 3 was performed except for reacting 1-(7,7-difluoro-2-(methylsulfonyl)-6,7-dihydro-5H-cyclopenta[d]pyrimidin-4-yl)pyrrolidin-3-ol prepared in Step B instead of 1-(7,7-difluoro-2-(methylsulfonyl)-6,7-dihydro-5H-cyclopenta[d]pyrimidin-4-yl)azetidin-3-ol with (2S)-2-methylazetidine[(1R,4S)-7,7-dimethyl-2-oxo-norbornan-1-yl]methanesulfonate (203 mg, 0.67 mmol) in Step C of Example 3, to prepare 1-(7,7-difluoro-2-((S)-2-methylsulfonyl-1-yl)-6,7-dihydro-5H-cyclopenta[d]pyrimidin-4-yl)pyrrolidin-3-ol.

### (4) Step D: Preparation of tert-butyl 4-(2-((1-(7,7-difluoro-2-((S)-2-methylazetidin-1-yl)-6,7-dihydro-5H-cyclopenta[d]pyrimidin-4-yl)pyrrolidin-3-ol)oxy)acetyl)piperazin-1-carboxylate

The same procedure in Step D of Example 3 was performed except for reacting 1-(7,7-difluoro-2-((S)-2-methylsulfonyl-1-yl)-6,7-dihydro-5H-cyclopenta[d]pyrimidin-4-yl)pyrrolidin-3-ol prepared in Step C instead of (S)-1-(7,7-difluoro-2-(2-methylazetidin-1-yl)-6,7-dihydro-5H-cyclopenta[d]pyrimidin-4-yl)azetidin-3-ol with tert-butyl 4-(2-bromoacetyl)piperazin-1-carboxylate (204 mg, 0.66 mmol) prepared in Preparation Example 7 in Step D of Example 3, to prepare tert-butyl 4-(2-((1-(7,7-difluoro-2-((S)-2-methylazetidin-1-yl)-6,7-dihydro-5H-cyclopenta[d]pyrimidin-4-yl)pyrrolidin-3-ol)oxy)acetyl)piperazin-1-carboxylate.

### (5) Step E: Preparation of 2-(((S)-1-(7,7-difluoro-2-((S)-2-methylazetidin-1-yl)-6,7-dihydro-5H-cyclopenta[d]pyrimidin-4-yl)pyrrolidin-3-yl)oxy)-1-(piperazin-1-yl)ethan-1-one

The same procedure in Step E of Example 3 was performed except for using tert-butyl 4-(2-((1-(7,7-difluoro-2-((S)-2-methylazetidin-1-yl)-6,7-dihydro-5H-cyclopenta[d]pyrimidin-4-yl)pyrrolidin-3-ol)oxy)acetyl)piperazin-1-carboxylate prepared in Step D instead of tert-butyl (S)-4-(2-((1-(7,7-difluoro-2-(2-methylazetidin-1-yl)-6,7-dihydro-5H-cyclopenta[d]pyrimidin-4-yl)azetidin-3-yl)oxy)acetyl)piperazin-1-carboxylate in Step E of Example 3, to obtain 2-(((S)-1-(7,7-difluoro-2-((S)-2-methylazetidin-1-yl)-6,7-dihydro-5H-cyclopenta[d]pyrimidin-4-yl)pyrrolidin-3-yl)oxy)-1-(piperazin-1-yl)ethan-1-one (93 mg, 0.17 mmol, yield 28%), which was confirmed through ¹H NMR analysis.

¹H NMR (500MHz, MeOH-d₄) δ 4.41 (q, J=6.5 Hz, 1H), 4.25 (q, J=13.6 Hz, 3H), 4.07-3.56 (m, 6H), 3.48 (dt, J=36.5, 4.7 Hz, 4H), 3.09 (d, J=23.2 Hz, 2H), 2.86-2.68 (m, 4H), 2.53-2.29 (m, 3H), 2.17 (s, 1H), 2.04 (s, 1H), 1.97-1.89 (m, 1H), 1.48 (d, J=6.4 Hz, 3H).

### Example 8: Preparation of 2-(((R)-1-(7,7-difluoro-2-((S)-2-methylazetidin-1-yl)-6,7-dihydro-5H-cyclopenta[d]pyrimidin-4-yl)pyrrolidin-3-yl)oxy)-1-(piperazin-1-yl)ethan-1-one

The same procedure in Example 7 was performed except for using (R)-pyrrolidin-3-ol hydrochloride (75 mg, 0.61 mmol) instead of (S)-pyrrolidin-3-ol in Step B, using (2S)-2-methylazetidine[(1R,4S)-7,7-dimethyl-2-oxo-norbornan-1-yl]methanesulfonate in 188 mg (0.62 mmol) in Step C, and using tert-butyl 4-(2-bromoacetyl)piperazin-1-carboxylate in 194 mg (0.63 mmol) in Step D in Example 7, to prepare the title compound of Formula 1-8 (108 mg, 0.25 mmol, yield 41%), which is the isomer of the compound of Formula 1-7.

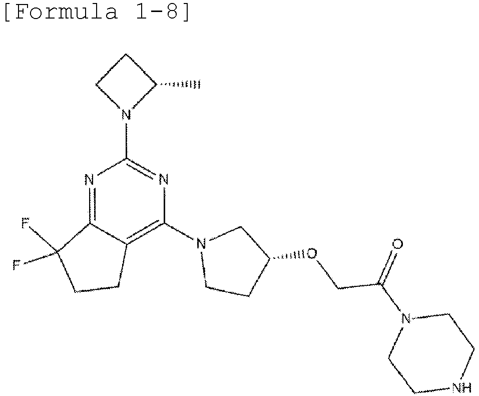

The synthesis was confirmed through ¹H NMR analysis.

¹H NMR (500MHz, MeOH-d₄) δ 4.40 (q, J=6.5 Hz, 1H), 4.24 (q, J=14.0 Hz, 3H), 4.07-3.59 (m, 6H), 3.58-3.38 (m, 4H), 3.08 (dd, J=33.9, 15.0 Hz, 2H), 2.86-2.68 (m, 4H), 2.53-2.29 (m, 3H), 2.17 (s, 1H), 2.03 (d, J=8.8 Hz, 1H), 1.97-1.85 (m, 1H), 1.48 (d, J=6.1 Hz, 3H).

### Example 9: Preparation of 2-((R)-1-(7,7-difluoro-2-((S)-2-methylazetidin-1-yl)-6,7-dihydro-5H-cyclopenta[d]pyrimidin-4-yl)pyrrolidin-3-yl)-1-(piperazin-1-yl)ethan-1-one)

As in Reaction 8, the processes of Steps A to E were performed in sequence to prepare the title compound of Formula 1-9.

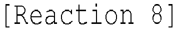

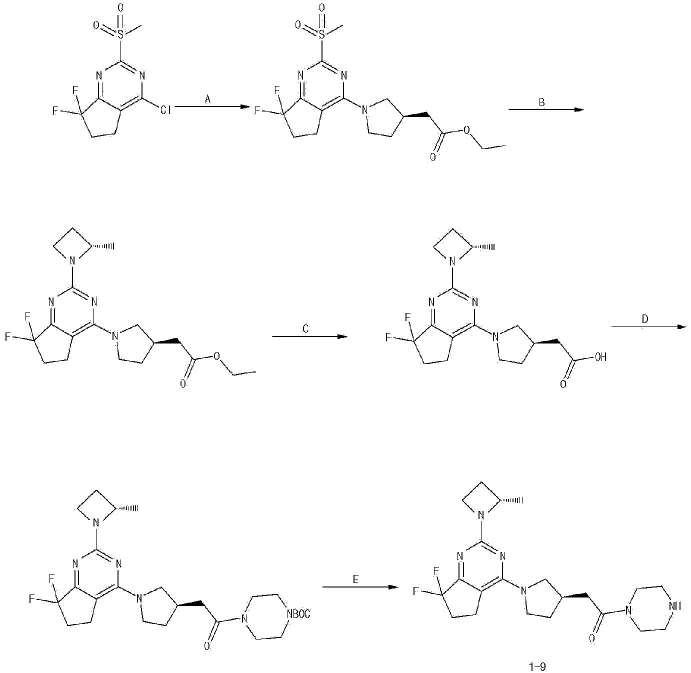

### (1) Step A: Preparation of ethyl (R)-2-(1-(7,7-difluoro-2-(methylsulfonyl)-6,7-dihydro-5H-cyclopenta[d]pyrimidin-4-yl)pyrrolidin-3-yl)acetate

The 4-chloro-7,7-difluoro-2-(methylsulfonyl)-6,7-dihydro-5H-cyclopenta[d]pyrimidine (100 mg, 0.37 mmol) prepared in Step A of Example 3, ethyl (R)-2-(pyrrolidin-3-yl)acetate hydrochloride (86 mg, 0.44 mmol), and triethylamine (113 mg, 1.11 mmol) were dissolved in 10 ml of dichloromethane, followed by stirring at room temperature for 16 hours. After finishing the reaction, the reaction solution was concentrated under a reduced pressure, a 1 N aqueous hydrochloric acid solution was added thereto, and the resultant was extracted with ethyl acetate. An organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain (R)-2-(1-(7,7-difluoro-2-(methylsulfonyl)-6,7-dihydro-5H-cyclopenta[d]pyrimidin-4-yl)pyrrolidin-3-yl)acetate (129 mg, 0.33 mmol, yield 89%), which was confirmed through ¹H NMR analysis.

¹H NMR (400MHz, CDCl₃) δ 4.28-4.06 (m, 2H), 4.08-3.62 (m, 3H), 3.59-3.34 (m, 1H), 3.31 (d, J=11.3 Hz, 3H), 3.30-3.17 (m, 2H), 2.73 (s, 1H), 2.64-2.49 (s, 3H), 2.49-2.14 (m, 2H), 1.82-1.58 (m, 1H), 1.37-1.23 (m, 3H).

### (2) Step B: Preparation of ethyl 2-((R)-1-(7,7-difluoro-2-((S)-2-methylazetidin-1-yl)-6,7-dihydro-5H-cyclopenta[d]pyrimidin-4-yl)pyrrolidin-3-yl)acetate

The (R)-2-(1-(7,7-difluoro-2-(methylsulfonyl)-6,7-dihydro-5H-cyclopenta[d]pyrimidin-4-yl)pyrrolidin-3-yl)acetate (129 mg, 0.33 mmol) prepared in Step A, (2S)-2-methylazetidine[(1R,4S)-7,7-dimethyl-2-oxo-norbornan-1-yl]methanesulfonate (120 mg, 0.39 mmol) and potassium carbonate (137 mg, 0.99 mmol) were dissolved in 10 ml of acetonitrile, followed by stirring at 80°C for 1 hour. After finishing the reaction the reaction solution was concentrated under a reduced pressure. To the concentrate under a reduced pressure, an aqueous saturated sodium chloride solution was added, and the resultant was extracted with ethyl acetate. An organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain ethyl 2-((R)-1-(7,7-difluoro-2-((S)-2-methylazetidin-1-yl)-6,7-dihydro-5H-cyclopenta[d]pyrimidin-4-yl)pyrrolidin-3-yl)acetate (103 mg, 0.27 mmol, yield 81%), which was confirmed through ¹H NMR analysis.

¹H NMR (400MHz, CDCl₃) δ 4.28-4.32 (m, 1H), 4.24-4.08 (m, 2H), 4.06-3.99 (m, 1H), 3.95 (q, J=8.2 Hz, 2H), 3.87-3.69 (m, 1H), 3.62 (q, J=8.7 Hz, 1H), 3.37-3.22 (m, 1H), 3.13-2.93 (m, 2H), 2.71-2.54 (m, 1H), 2.53-2.37 (m, 4H), 2.16 (dd, J=10.8, 4.7 Hz, 1H), 2.01-1.84 (m, 1H), 1.63 (dt, J=20.5, 8.7 Hz, 1H), 1.49 (d, J=6.1 Hz, 3H), 1.24 (dt, J=30.2, 7.0 Hz, 3H) .

### (3) Step C: Preparation of S-((R)-1-(7,7-difluoro-2-((S)-2-methylazetidin-1-yl)-6,7-dihydro-5H-cyclopenta[d]pyrimidin-4-yl)pyrrolidin-3-yl)acetic acid

The ethyl 2-((R)-1-(7,7-difluoro-2-((S)-2-methylazetidin-1-yl)-6,7-dihydro-5H-cyclopenta[d]pyrimidin-4-yl)pyrrolidin-3-yl)acetate (103 mg, 0.27 mmol) prepared in Step B and lithium hydride monohydrate (45 mg, 1.08 mmol) were dissolved in a mixture of tetrahydrofuran/water/methanol (4/1/1 by a weight ratio, 24 ml), followed by stirring at room temperature for 16 hours. After finishing the reaction, a 1 N hydrochloric acid solution was added to the reaction solution, and the resultant was extracted with ethyl acetate. An organic layer was washed with an aqueous sodium chloride solution, dried over anhydrous magnesium sulfate and filtered. The filtrate was concentrated under a reduced pressure, and the concentrate under a reduced pressure was purified by column chromatography (dichloromethane/methanol = 20/1 by a weight ratio) to obtain S-((R)-1-(7,7-difluoro-2-((S)-2-methylazetidin-1-yl)-6,7-dihydro-5H-cyclopenta[d]pyrimidin-4-yl)pyrrolidin-3-yl)acetic acid (75 mg, 0.21 mmol, yield 78%), which was confirmed through ¹H NMR analysis.

¹H NMR (500MHz, MeOH-d₄) δ 4.42 (td, J=13.3, 6.7 Hz, 1H), 4.12-3.92 (m, 2H), 3.92-3.55 (m, 3H), 3.34 (s, 1H), 3.19-2.99 (m, 2H), 2.59 (q, J=7.4 Hz, 1H), 2.53-2.41 (m, 4H), 2.41-2.29 (m, 1H), 2.17 (d, J=5.8 Hz, 1H), 2.03-1.84 (m, 1H), 1.66 (dd, J=19.1, 9.0 Hz, 1H), 1.55-1.40 (m, 3H).

### (4) Step D: Preparation of tert-butyl 4-(2-((R)-1-(7,7-difluoro-2-((S)-2-methylazetidin-1-yl)-6,7-dihydro-5H-cyclopenta[d]pyrimidin-4-yl)pyrrolidin-3-yl)acetyl)piperazin-1-carboxylate

The S-((R)-1-(7,7-difluoro-2-((S)-2-methylazetidin-1-yl)-6,7-dihydro-5H-cyclopenta[d]pyrimidin-4-yl)pyrrolidin-3-yl)acetic acid (75 mg, 0.21 mmol) prepared in Step C, N-[(dimethylamino)-1H-1,2,3-triazol-[4-5-b]pyridin-1-ylmethylene]-N-methylmethanaminium hexafluorophosphate N-oxide (121 mg, 0.31 mmol), tert-butyl piperazin-1-carboxylate (59 mg, 0.31 mmol) and N,N-diisopropylethylamine (82 mg, 0.63 mmol) were dissolved in 10 ml of dimethylformamide, followed by stirring at room temperature for 18 hours. After finishing the reaction, the reaction solution was concentrated under a reduced pressure, water was added thereto, and the resultant was extracted with ethyl acetate. An organic layer was dried over anhydrous magnesium sulfate and filtered. The filtrate was concentrated under a reduced pressure, and the concentrate under a reduced pressure was purified by column chromatography (dichloromethane/methanol = 20/1 by a weight ratio) to obtain tert-butyl 4-(2-((R)-1-(7,7-difluoro-2-((S)-2-methylazetidin-1-yl)-6,7-dihydro-5H-cyclopenta[d]pyrimidin-4-yl)pyrrolidin-3-yl)acetyl)piperazin-1-carboxylate (109 mg, 0.20 mmol, yield 98%), which was confirmed through ¹H NMR analysis.

¹H NMR (500MHz, CDCl₃) δ 4.41 (dd, J=13.9, 6.3 Hz, 1H), 4.05 (td, J=8.7, 4.6 Hz, 1H), 3.96 (q, J=8.2 Hz, 2H), 3.78 (s, 1H), 3.62 (d, J=21.4 Hz, 3H), 3.53-3.34 (m, 6H), 3.34-3.23 (m, 1H), 3.12-2.98 (m, 2H), 2.76-2.63 (m, 1H), 2.54-2.26 (m, 6H), 2.16 (d, J=47.0 Hz, 1H), 2.01-1.84 (m, 1H), 1.71-1.55 (m, 1H), 1.55-1.30 (m, 12H).

### (5) Step E: Preparation of 2-((R)-1-(7,7- difluoro-2-((S)-2-methylazetidin-1-yl)-6,7-dihydro-5H-cyclopenta[d]pyrimidin-4-yl)pyrrolidin-3-yl)-1-(piperazin-1-yl)ethan-1-one

The tert-butyl 4-(2-((R)-1-(7,7- difluoro-2-((S)-2-methylazetidin-1-yl)-6,7-dihydro-5H-cyclopenta[d]pyrimidin-4-yl)pyrrolidin-3-yl)acetyl)piperazin-1-carboxylate (109 mg, 0.20 mmol) prepared in Step D and 1 ml of trifluoroacetic acid were dissolved in 10 ml of dichloromethane, followed by stirring for 18 hours. After finishing the reaction, the reaction solution was concentrated under a reduced pressure, an aqueous saturated ammonium solution was added thereto, and the resultant was extracted with ethyl acetate. An organic layer was dried over anhydrous magnesium sulfate and filtered. The filtrate was concentrated under a reduced pressure, and the concentrate under a reduced pressure was purified by column chromatography (dichloromethane/7 N ammonia methanol = 10/1 by a weight ratio) to obtain 2-((R)-1-(7,7-difluoro-2-((S)-2-methylazetidin-1-yl)-6,7-dihydro-5H-cyclopenta[d]pyrimidin-4-yl)pyrrolidin-3-yl)-1-(piperazin-1-yl)ethan-1-one (80 mg, 0.19 mmol, yield 90%), which was confirmed through ¹H NMR analysis.

¹H NMR (500MHz, MeOH-d₄) δ 4.39 (q, J=6.7 Hz, 1H), 3.99 (td, J=8.7, 4.7 Hz, 2H), 3.87 (t, J=8.1 Hz, 1H), 3.84-3.60 (m, 1H), 3.60-3.45 (m, 4H), 3.15-2.98 (m, 2H), 2.79 (dt, J=18.4, 5.0 Hz, 4H), 2.67-2.49 (m, 3H), 2.48-2.27 (m, 3H), 2.15 (s, 1H), 1.91 (dd, J=17.2, 7.5 Hz, 1 H), 1.65 (s, 1H), 1.53-1.39 (m, 3H).

### Example 10: Preparation of (S)-2-((1-(7,7-difluoro-2-(2-methylazetidin-1-yl)-6,7-dihydro-5H-cyclopenta[d]pyrimidin-4-yl)piperidin-4-yl)oxy)-1-(piperazin-1-yl)ethan-1-one

As in Reaction 9, the processes of Steps A to E were performed in sequence prepare the title compound of Formula 1-10.

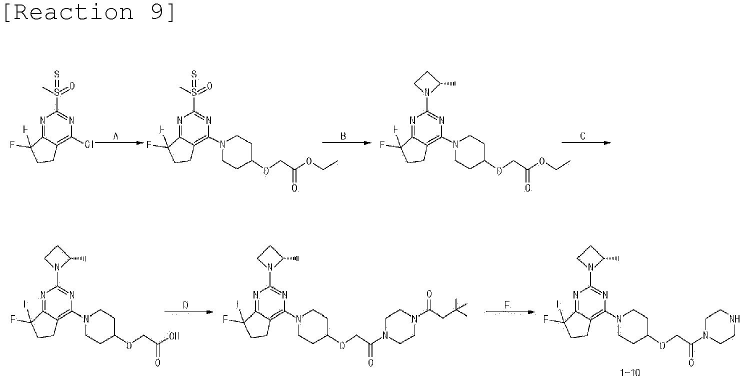

The same procedures in Step A to Step E in Example 9 were performed in sequence except for using 4-chloro-7,7-difluoro-2-(methylsulfonyl)-6,7-dihydro-5H-cyclopenta[d]pyrimidine prepared in 200 mg (0.74 mmol) and using ethyl 2-(piperidin-4-yloxy)acetate hydrochloride (251 mg, 1.12 mmol) instead of ethyl (R)-2-(pyrrolidin-3-yl)acetate hydrochloride in Step A, and using (2S)-2-methylazetidine[(1R,4S)-7,7-dimethyl-2-oxo-norbornan-1-yl]methanesulfonate in 302 mg (0.64 mmol) in Step B in Example 9, to prepare the title compound of Formula 1-10 (110 mg, 0.24 mmol, yield 33%), which was confirmed through ¹H NMR analysis.

¹H NMR (400MHz, CDCl₃) δ 4.40 (q, J=6.9 Hz, 1H), 4.18 (s, 2H), 3.91-4.07 (m, 4H), 3.67 (td, J=7.9, 4.0, 1H), 3.57 (t, J=4.8 Hz, 2H), 3.50 (t, J=5.0 Hz, 2H), 3.31-3.38 (m, 2H), 2.83-2.91 (m, 6H), 2.31-2.49 (m, 3H), 1.88-1.96 (m, 3H), 1.61 (m, 2H), 1.47 (d, J=5.9 Hz, 3H).

### Example 11: Preparation of (S)-2-(4-(2-(2-methylazetidin-1-yl)-6-(trifluoromethyl)pyrimidin-4-yl)piperazin-1-yl)-1-(piperazin-1-yl)ethan-1-one

As in Reaction 10, the processes of Steps A to E were performed in sequence to prepare the title compound of Formula 1-11.

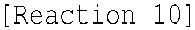

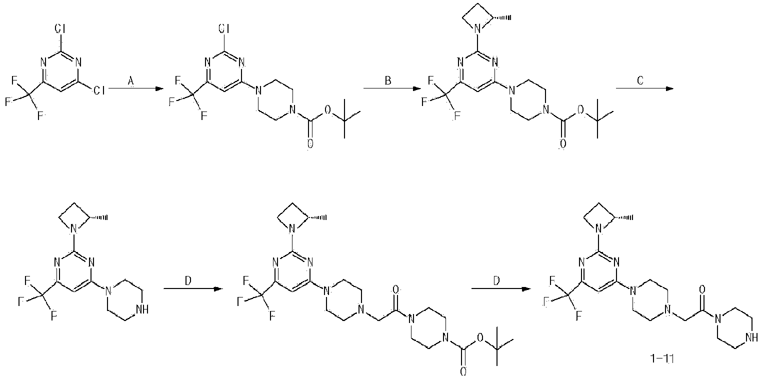

### (1) Step A: Preparation of tert-butyl 4-(2-chloro-6-(trifluoro)pyrimidin-4-yl)piperazin-1-carboxylate

2,4-Dichloro-6-(trifluoro)pyrimidine (5 g, 23.04 mmol), tert-butyl piperazin-1-carboxylate (4.3 g, 23.04 mmol) and diisopropylethylamine (12 ml, 69.12 mmol) were dissolved in 200 ml of dichloromethane, followed by stirring at room temperature for 2 hours. After finishing the reaction, a 1 N aqueous hydrochloric acid solution was added to the reaction solution, and the resultant was extracted with dichloromethane. An organic layer was dried over anhydrous magnesium sulfate and filtered. The filtrate was concentrated under a reduced pressure, and the concentrate under a reduced pressure was purified by column chromatography to obtain tert-butyl 4-(2-chloro-6-(trifluoro)pyrimidin-4-yl)piperazin-1-carboxylate (7.28 g, 19.84 mmol, yield 86%), which was confirmed through ¹H NMR analysis.

¹H NMR (400MHz, CDCl₃) δ 6.71 (s, 1H), 3.78 (s, 4H), 3.56 (s, 4H), 1.49 (s, 9H).

### (2) Step B: Preparation of tert-butyl (S)-4-(2-(2-methylazetidin-1-yl)-6-(trifluoromethyl)pyrimidin-4-yl)piperazin-1-carboxylate

The tert-butyl 4-(2-chloro-6-(trifluoro)pyrimidin-4-yl)piperazin-1-carboxylate (7.28 g, 19.84 mmol) prepared in Step A, (2S)-2-methylazetidine[(1R,4S)-7,7-dimethyl-2-oxo-norbornan-1-yl]methanesulfonate (7.23 g, 23.81 mmol) and potassium carbonate (8.23 g, 59.52 mmol) were dissolved in 300 ml of acetonitrile, followed by stirring at 80°C for 1 hour. After finishing the reaction, the reaction solution was cooled to room temperature, and the resultant was concentrated under a reduced pressure, dissolved in ethyl acetate, and then, washed with a saturated chloride solution. An organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl (S)-4-(2-(2-methylazetidin-1-yl)-6-(trifluoromethyl)pyrimidin-4-yl)piperazin-1-carboxylate (7.48 g, 18.63 mol, yield 93%), which was confirmed through ¹H NMR analysis.

¹H NMR (400MHz, CDCl₃) δ 6.15 (s, 1H), 4.45 (td, J=13.0, 6.5 Hz, 1H), 4.12-3.87 (m, 2H), 3.61 (s, 4H), 3.50 (d, J=4.6 Hz, 4H), 2.49-2.31 (m, 1H), 2.03-1.86 (m, 1H), 1.53-1.30 (m, 12H).

### (3) Step C: Preparation of (S)-2-(2-methylazetidin-1-yl)-4-(piperazin-1-yl)-6-(trifluoromethyl)pyrimidine

The tert-butyl (S)-4-(2-(2-methylazetidin-1-yl)-6-(trifluoromethyl)pyrimidin-4-yl)piperazin-1-carboxylate (7.48 g, 18.63 mmol) prepared in Step B and 14 ml of trifluoroacetic acid were dissolved in 10 ml of dichloromethane, followed by stirring at room temperature for 18 hours. After finishing the reaction, the reaction solution was concentrated under a reduced pressure, an aqueous saturated ammonium solution was added thereto, and the resultant was extracted with ethyl acetate. An organic layer was dried over anhydrous magnesium sulfate and filtered. The filtrate was concentrated under a reduced pressure to obtain (S)-2-(2-methylazetidin-1-yl)-4-(piperazin-1-yl)-6-(trifluoromethyl)pyrimidine (5.6 g, 18.63 mmol, yield 100%), which was confirmed through ¹H NMR analysis.

¹H NMR (400MHz, CDCl₃) δ 6.18 (s, 1H), 4.50 (s, 1H), 4.21-3.94 (m, 2H), 3.89 (d, J=4.0 Hz, 4H), 3.29-3.16 (m, 4H), 2.55-2.36 (m, 1H), 2.02-1.88 (m, 1H), 1.51 (d, J=6.4 Hz, 3H).

### (4) Step D: Preparation of tert-butyl (S)-4-(2-(4-(2-(2-methylazetidin-1-yl)-6-(trifluoromethyl)pyrimidin-4-yl)piperazin-1-yl)acetyl)piperazin-1-carboxylate

The (S)-2-(2-methylazetidin-1-yl)-4-(piperazin-1-yl)-6-(trifluoromethyl)pyrimidine (5.6 g, 18.63 mmol) prepared in Step C was dissolved in 100 ml of dimethylformamide, potassium carbonate (6.86 g, 22.36 mmol) was added thereto, and the tert-butyl 4-(2-bromoacetyl)piperazin-1-carboxylate (7.72 g, 55.89 mmol) prepared in Preparation Example 7 was added thereto, followed by stirring at room temperature for 16 hours. After finishing the reaction, the reaction solution was concentrated under a reduced pressure, the resultant was dissolved in ethyl acetate, and the resultant was washed with a saturated sodium chloride solution. An organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl (S)-4-(2-(4-(2-(2-methylazetidin-1-yl)-6-(trifluoromethyl)pyrimidin-4-yl)piperazin-1-yl)acetyl)piperazin-1-carboxylate (10.22 g, 19.37 mmol, yield 104%), which was confirmed through ¹H NMR analysis.

¹H NMR (500MHz, CDCl₃) δ 6.15 (s, 1H), 4.44 (q, J=6.5 Hz, 1H), 4.03 (dd, J=13.7, 8.8 Hz, 1H), 3.95 (q, J=8.2 Hz, 1H), 3.61 (d, J=28.4 Hz, 8H), 3.44 (d, J=22.3 Hz, 4H), 3.25 (d, J=12.8 Hz, 2H), 2.56 (s, 4H), 2.39 (dd, J=23.7, 9.3 Hz, 1H), 1.93 (dd, J=17.5, 8.4 Hz, 1H), 1.53-1.42 (m, 12H).

### (5) Step E: Preparation of ((S)-2-(4-(2-(2-methylazetidin-1-yl)-6-(trifluoromethyl)pyrimidin-4-yl)piperazin-1-yl)-1-(piperazin-1-yl)ethan-1-one

The tert-butyl (S)-4-(2-(4-(2-(2-methylazetidin-1-yl)-6-(trifluoromethyl)pyrimidin-4-yl)piperazin-1-yl)acetyl)piperazin-1-carboxylate (10.22 g, 19.37 mmol) prepared in Step D and 4 ml of trifluoroacetic acid were dissolved in 20 ml of dichloromethane, followed by stirring for 18 hours. After finishing the reaction, the reaction solution was concentrated under a reduced pressure, ethyl acetate and an aqueous saturated ammonium solution were added thereto, and the resultant was extracted with ethyl acetate. An organic layer was dried over anhydrous magnesium sulfate and filtered. The filtrate was concentrated under a reduced pressure, and the concentrate under a reduced pressure was purified by column chromatography (dichloromethane/7 N ammonia methanol = 10/1 by a weight ratio) to obtain ((S)-2-(4-(2-(2-methylazetidin-1-yl)-6-(trifluoromethyl)pyrimidin-4-yl)piperazin-1-yl)-1-(piperazin-1-yl)ethan-1-one (8.28 g, 19.37 mmol, yield 100%), which was confirmed through ¹H NMR analysis.

¹H NMR (400MHz, MeOH-d₄) δ 6.35 (s, 1H), 4.42 (d, J=7.3 Hz, 1H), 4.05-3.85 (m, 2H), 3.69 (s, 4H), 3.64-3.49 (m, 4H), 3.27 (s, 2H), 2.81 (dt, J=24.9, 5.0 Hz, 4H), 2.55 (t, J=5.0 Hz, 4H), 2.40 (s, 1H), 1.95 (s, 1H), 1.54-1.41 (m, 3H).

### Example 12: Preparation of (S)-4-(2-(2-methylazetidin-1-yl)-6-(trifluoromethyl)pyrimidin-4-yl)-1-(2-oxo-2-(piperazin-1-yl)ethyl)piperazin-2-one

As in Reaction 11, the processes of Step A to Step D were performed in sequence to prepare the title compound of Formula 1-12.

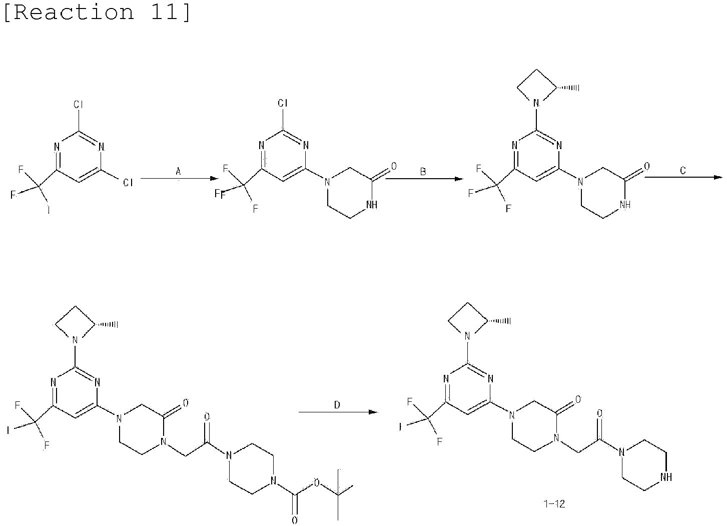

The same procedures in Step A to Step D in Example 1 were performed in sequence except for using 2,4-dichloro-6-(trifluoromethyl)pyrimidine in 104 mg (0.48 mmol) and using piperazin-2-one (48 mg, 0.48 mmol) instead of azetidin-3-ol hydrochloride in Step A, using (2S)-2-methylazetidine[(1R,4S)-7,7-methyl-2-oxo-norbornan-1-yl]methanesulfonate in 181 mg (0.59 mmol) in Step B, and using tert-butyl 4-(2-bromoacetyl)piperazin-1-carboxylate in 166 mg (0.54 mmol) in Step C in Example 1, to prepare the title compound of Formula 1-13 (71 mg, 0.16 mmol, yield 34%), which was confirmed through ¹H NMR analysis.

¹H NMR (400MHz, MeOH-d₄) δ 6.38 (s, 1H), 4.60-4.41 (m, 1H), 4.34 (d, J=11.6 Hz, 4H), 3.95 (s, 4H), 3.61-3.43 (m, 6H), 2.92-2.75 (m, 4H), 2.47-2.37 (m, 1H), 2.02-1.84 (m, 1H), 1.50 (d, J=6.1 Hz, 3H).

### Example 13: Preparation of (S)-2-4-(7,7-difluoro-2-(2-methylazetidin-1-yl)-6,7-dihydro-5H-cyclopenta[d]pyrimidin-1-yl)-1-(piperazin-1-yl)ethan-1-one

As in Reaction 12, the processes of Step A to Step F were performed in sequence to prepare the title compound of Formula 1-13.

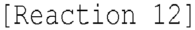

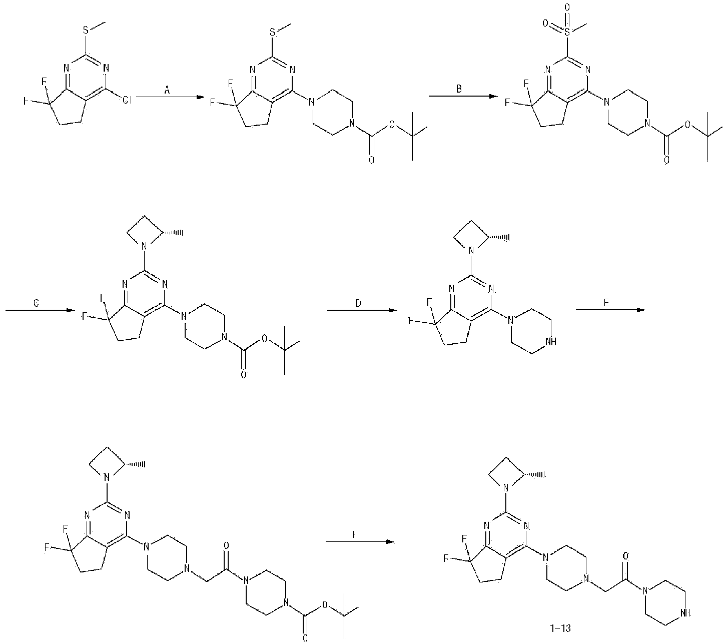

Step A and B in Reaction 12 followed Step A and B of Example 2, and Step C to Step F in Reaction 12 followed Step B to E of Example 11.

Particularly, the same procedures in Step A and Step B of Example 2 were performed except for using 4-chloro-2-(methylthio)-6,7-dihydro-5H-cyclopenta[d]pyrimidine in 20 mg (0.07 mmol) instead of 4,5-dichloro-2-(methylthio)-6-(trifluoromethyl)pyrimidine, and tert-butyl piperazin-1-carboxylate in 15 mg (0.11 mmol) instead of tert-butyl 4-(2-(azetidin-3-yloxy)acetyl)piperazin-1-carboxylate to perform Step A and Step B of Reaction 12, and then, the same procedures in Step B to Step E of Example 11 were continuously performed except for using (2S)-2-methylazetidine[(1R,4S)-7,7-methyl-2-oxo-norbornan-1-yl]methanesulfonate in 25 mg (0.08 mmol) in Step B, using tert-butyl 4-(2-bromoacetyl)piperazin-1-carboxylate in 18 mg (0.12 mmol) in Step D to perform Step C to Step F in Reaction 12, to prepare the title compound of Formula 1-13 (29 mg, 0.06 mmol, yield 95%), which was confirmed through ¹H NMR analysis.

¹H NMR (400MHz, MeOH-d₄) δ 4.48-4.33 (m, 1H), 4.01 (td, J=8.7, 4.8 Hz, 1H), 3.89 (q, J=8.2 Hz, 1H), 3.78 (t, J=4.7 Hz, 4H), 3.58 (dt, J=23.8, 4.9 Hz, 4H), 3.26 (s, 2H), 2.96 (s, 2H), 2.82 (dt, J=26.5, 4.9 Hz, 4H), 2.57 (t, J=4.7 Hz, 4H), 2.53-2.32 (m, 3H), 1.94 (dd, J=19.1, 7.2 Hz, 1H), 1.55-1.40 (m, 3H).

### Example 14: Preparation of (S)-2-4-(7,7-difluoro-2-(2-methylazetidin-1-yl)-6,7-dihydro-5H-cyclopenta[d]pyrimidin-1-yl)-1-(piperazin-1-yl)morpholinoethan-1-one

As in Reaction 13, the processes of Step A to Step E were performed in sequence to prepare the title compound of Formula 1-14.

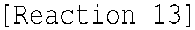

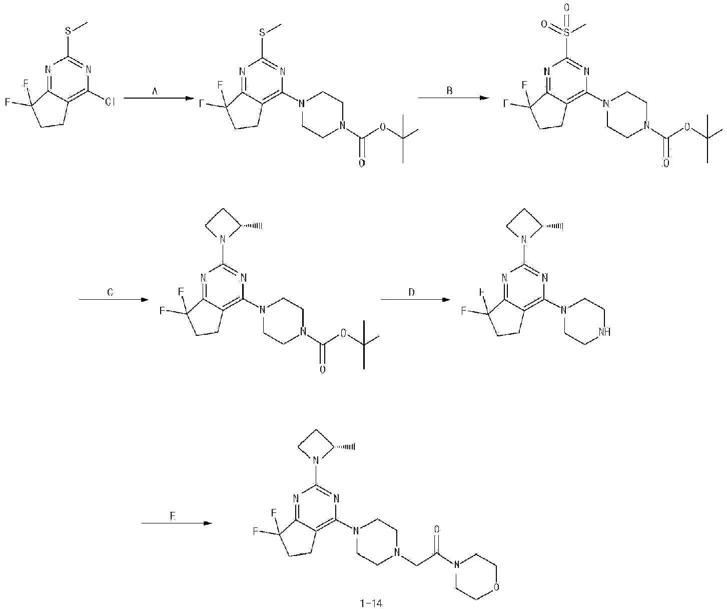

Step A to Step E of Reaction 13 followed Step A to Step E of Example 13.

Particularly, the same procedures in Step A to Step E in Example 13 were performed in sequence except for using 4-chloro-2-(methylthio)-6,7-dihydro-5H-cyclopenta[d]pyrimidine in 10 mg (0.04 mmol) and tert-butyl piperazin-1-carboxylate in 8 mg (0.06 mmol) in Step A, using (2S)-2-methylazetidine[(1R,4S)-7,7-methyl-2-oxo-norbornan-1-yl]methanesulfonate in 12 mg (0.04 mmol) in Step C, and using 2-bromo-1-morpholinoethan-1-one (10 mg, 0.05 mmol) instead of tert-butyl 4-(2-bromoacetyl)piperazin-1-carboxylate in Step D in Example 13, to prepare the title compound of Formula 1-14 (10 mg, 0.02 mmol, yield 50%), which was confirmed through ¹H NMR analysis.

¹H NMR (400MHz, MeOH-d₄) δ 4.41 (q, J=6.8 Hz, 1H), 4.01 (td, J=8.8, 4.9 Hz, 1H), 3.89 (q, J=8.2 Hz, 1H), 3.78 (t, J=4.9 Hz, 4H), 3.73-3.61 (m, 6H), 3.57 (t, J=4.6 Hz, 2H), 3.27 (s, 2H), 2.96 (d, J=3.1 Hz, 2H), 2.64-2.53 (m, 4H), 2.53-2.31 (m, 3H), 2.02-1.85 (m, 1H), 1.47 (d, J=6.1 Hz, 3H).

### Example 15: Preparation of (S)-1-(4-(7,7-difluoro-2-(2-methylazetidin-1-yl)-6,7-dihydro-5H-cyclopenta[d]pyrimidin-4-yl)-3-(piperazin-1-yl)propan-1,3-dione

As in Reaction 14, the processes of Step A to Step D were performed in sequence to prepare the title compound of Formula 1-15.

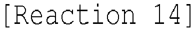

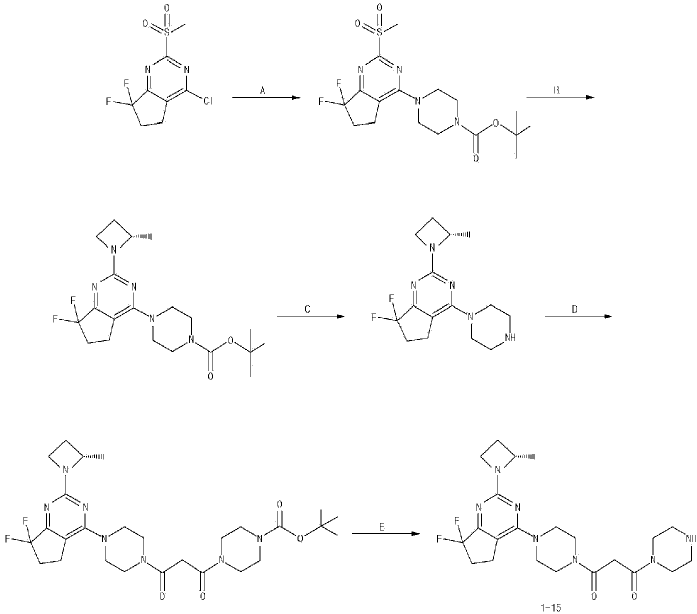

### (1) Step A: Preparation of tert-butyl 4-(7,7-difluoro-2-(methylsulfonyl)-6,7-dihydro-5H-cyclopenta[d]pyrimidin-4-yl)piperazin-1-carboxylate

4-Chloro-7,7-difluoro-2-(methylsulfonyl)-6,7-dihydro-5H-cyclopenta[d]pyrimidine (385 mg, 1.433 mmol), tert-butyl piperazin-1-carboxylate (294 mg, 1.576 mmol) and triethylamine (0.3 ml, 2.150 mmol) were dissolved in 20 ml of dichloromethane, followed by stirring at room temperature for 18 hours. After finishing the reaction, dichloromethane and water were added to the reaction solution, and extraction was performed. An organic layer was dried over anhydrous magnesium sulfate and filtered. The filtrate was concentrated under a reduced pressure and purified by column chromatography (ethyl acetate/hexane = 1/1 by a weight ratio) to obtain tert-butyl 4-(7,7-difluoro-2-(methylsulfonyl)-6,7-dihydro-5H-cyclopenta[d]pyrimidin-4-yl)piperazin-1-carboxylate (555.8 mg, yield 93%), which was confirmed through ¹H NMR analysis.

¹H NMR (400MHz, CDCl₃) δ 3.88 (t, J=5.3 Hz, 4H), 3.55 (t, J=5.3 Hz, 4H), 3.33 (s, 3H), 3.11-3.14 (m, 2H), 2.59 (dt, J=21.0, 7.5 Hz, 2H), 1.47 (s, 9H).

### (2) Step B: Preparation of tert-butyl (S)-4-(7,7-difluoro-2-(2-methylazetidin-1-yl)-6,7-dihydro-5H-cyclopenta[d]pyrimidin-4-yl)piperazin-1-carboxylate

The tert-butyl 4-(7,7-difluoro-2-(methylsulfonyl)-6,7-dihydro-5H-cyclopenta[d]pyrimidin-4-yl)piperazin-1-carboxylate (555 mg, 1.326 mmol) prepared in Step A, (2S)-2-methylazetidine[(1R,4S)-7,7-dimethyl-2-oxo-norbornan-1-yl]methanesulfonate (443 mg, 1.459 mmol) and potassium carbonate (458 mg, 3.32 mmol) were dissolved in 50 ml of acetonitrile, followed by stirring at 80°C for 6 hours. After finishing the reaction, ethyl acetate and water were added to the reaction solution, followed by extracting. An organic layer was dried over anhydrous magnesium sulfate and filtered. The filtrate was concentrated under a reduced pressure and purified by column chromatography (0-50% ethyl acetate/hexane = 1/1 by a weight ratio) to obtain tert-butyl (S)-4-(7,7-difluoro-2-(2-methylazetidin-1-yl)-6,7-dihydro-5H-cyclopenta[d]pyrimidin-4-yl)piperazin-1-carboxylate (395 mg, yield 72%), which was confirmed through ¹H NMR analysis.

¹H NMR (400MHz, CDCl₃) δ 4.42 (dd, J=14.2, 5.9 Hz, 1H), 4.05 (td, J=8.7, 4.6 Hz, 1H), 3.96 (q, J=8.2 Hz, 1H), 3.66 (t, J=5.3 Hz, 4H), 3.47 (t, J=5.3 Hz, 4H), 2.88-2.91 (m, 2H), 2.33-2.51 (m, 3H), 1.89-1.97 (m, 1H), 1.47-1.49 (m, 12H).

### (3) Step C: Preparation of (S)-4-(7,7-difluoro-2-(2-methylazetidin-1-yl)-4-(piperazin-1-yl)-6,7-dihydro-5H-cyclopenta[d]pyrimidine

The tert-butyl (S)-4-(7,7-difluoro-2-(2-methylazetidin-1-yl)-6,7-dihydro-5H-cyclopenta[d]pyrimidin-4-yl)piperazin-1-carboxylate (390 mg, 0.952 mmol) prepared in Step B was dissolved in 12 ml of dichloromethane, and 3 ml of trifluoroacetic acid was added thereto, followed by stirring at room temperature for 18 hours. After finishing the reaction, the reaction solution was concentrated under a reduced pressure. The concentrate was dissolved in dichloromethane, neutralized with saturated sodium bicarbonate, and then, extracted with ethyl acetate. An organic layer was dried over anhydrous magnesium sulfate and filtered. The filtrate was concentrated under a reduced pressure to obtain (S)-4-(7,7-difluoro-2-(2-methylazetidin-1-yl)-4-(piperazin-1-yl)-6,7-dihydro-5H-cyclopenta[d]pyrimidine (308 mg), which was used in the next reaction without separation and purification.

### (4) Step D: Preparation of (S)-3-(4-(7,7-difluoro-2-(2-methylazetidin-1-yl)-6,7-dihydro-5H-cyclopenta[d]pyrimidin-4-yl)piperazin-1-yl)-3-(piperazin-1-yl)propan-1,3-dione

The (S)-4-(7,7-difluoro-2-(2-methylazetidin-1-yl)-4-(piperazin-1-yl)-6,7-dihydro-5H-cyclopenta[d]pyrimidine (106 mg, 0.343 mmol) prepared in Step C, 3-(4-(tert-butoxycarbonyl)piperazin-1-yl)-3-oxopropanoic acid (103 mg, 0.377 mmol) and triethylamine (0.096 ml, 0.685 mmol) were dissolved in 10 ml of dichloromethane, and propylphosphonic anhydride (50 wt%, 0.255 ml, 0.428 mmol) was added thereto, followed by stirring at room temperature for 4 hours. After finishing the reaction, dichloromethane and water were added to the reaction solution, and extraction was performed. An organic layer was dried over anhydrous magnesium sulfate and filtered. The filtrate was concentrated under a reduced pressure and purified by column chromatography to obtain tert-butyl (S)-3-(4-(7,7-difluoro-2-(2-methylazetidin-1-yl)-6,7-dihydro-5H-cyclopenta[d]pyrimidin-4-yl)piperazin-1-yl)-3-oxopropanoyl)piperazin-1-carboxylate (122 mg, yield 63%).

The tert-butyl (S)-3-(4-(7,7-difluoro-2-(2-methylazetidin-1-yl)-6,7-dihydro-5H-cyclopenta[d]pyrimidin-4-yl)piperazin-1-yl)-3-oxopropanoyl)piperazin-1-carboxylate thus prepared and 1 ml of trifluoroacetic acid were dissolved in 4 ml of dichloromethane, followed by stirring at room temperature for 3 hours. After finishing the reaction, the resultant was concentrated under a reduced pressure. The concentrate was neutralized with an aqueous saturated sodium bicarbonate solution, and the resultant was extracted with dichloromethane. An organic layer was dried over anhydrous magnesium sulfate and filtered. The filtrate was concentrated under a reduced pressure and purified by column chromatography to obtain (S)-(3-(4-(7,7-difluoro-2-(2-methylazetidin-1-yl)-6,7-dihydro-5H-cyclopenta[d]pyrimidin-4-yl)piperazin-1-yl)-3-(piperazin-1-yl)propan-1,3-dione (88 mg, yield 88%), which was confirmed through ¹H NMR analysis.

¹H NMR (500MHz, CDCl₃) δ 4.38-4.43 (m, 1H), 4.02-4.06 (m, 1H), 3.95 (q, J=8.2 Hz, 1H), 3.74 (m, 2H), 3.68 (m, 6H), 3.60 (m, 4H), 3.55 (s, 2H), 2.85-2.90 (m, 6H), 2.55 (s, 1H), 2.41-2.49 (m, 2H), 2.34-2.39 (m, 1H), 1.90-1.97 (m, 1H), 1.48 (d, J=6.1 Hz, 3H).

### Example 16: Preparation of (S)-4-(7,7-difluoro-2-(2-methylazetidin-1-yl)-6,7-dihydro-5H-cyclopenta[d]pyrimidin-4-yl)-1-(2-oxo-2-(piperazin-1-yl)ethyl)piperazin-2-one

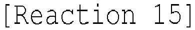

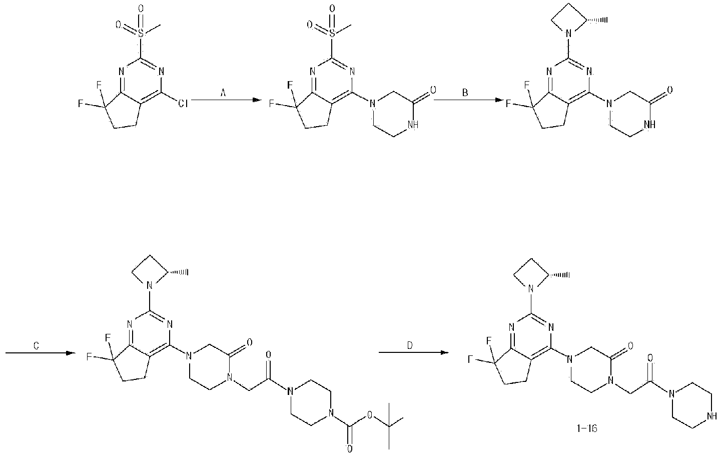

By applying Step B to Step E of Example 3, the title compound of Formula 1-16 was prepared according to Reaction 15.

### (1) Step A

The same procedure in Step B of Example 3 was performed except for using 4-chloro-7,7-difluoro-2-(methylsulfonyl)-6,7-dihydro-5H-cyclopenta[d]pyrimidine (100 mg, 0.37 mmol) and using piperazin-2-one (224 mg, 2.23 mmol) instead of azetidin-3-ol hydrochloride in Step B of Example 3.

### (2) Step B

The same procedure in Step C of Example 3 was performed except for using (S)-2-methylazetidine(7,7-dimethyl-2-oxobicyclo[2.2.1]heptan-1-yl)methanesulfonate (209 mg, 0.45 mmol) instead of (2S)-2-methylazetidine[(1R,4S)-7,7-methyl-2-oxo-norbornan-1-yl]methanesulfonate in Step C of Example 3.

### (3) Step C

The same procedure in Step D of Example 3 was performed except for using tert-butyl 4-(2-bromoacetyl)piperazin-1-carboxylate in 125 mg (0.41 mmol) in Step D of Example 3.

### (4) Step D

The same procedure in Step E of Example 3 was performed except for using the compound prepared in Step C instead of tert-butyl (S)-4-(2-((1-(7,7-difluoro-2-(2-methylazetidin-1-yl)-6,7-dihydro-5H-cyclopenta[d]pyrimidin-4-yl)azetidin-3-yl)oxy)acetyl)piperazin-1-carboxylate in Step E of Example 3, to prepare the title compound of Formula 1-16 (25 mg, 0.06 mmol, yield 15%), which was confirmed through ¹H NMR analysis.

¹H NMR (400MHz, CDCl₃) δ 4.40-4.45 (m, 1H), 4.32-4.38 (m, 2H), 4.22 (s, 2H), 4.06 (m, 1H), 3.93-4.01 (m, 3H), 3.60 (t, J=5.0 Hz, 2H), 3.53 (t, J=5.3 Hz, 2H), 3.45 (t, J=4.8 Hz, 2H), 3.93-2.98 (m, 2H), 2.89 (td, J=9.4, 4.3 Hz, 4H), 2.34-2.51 (m, 3H), 1.89-1.97 (m, 1H), 1.48 (d, J=6.4 Hz, 3H).

### Example 17: Preparation of (S)-2-(4-(7,7-difluoro-2-(2-methylazetidin-1-yl)-6,7-dihydro-5H-cyclopenta[d]pyrimidin-4-yl)piperidin-1-yl)-1-(piperazin-1-yl)ethan-1-one

As in Reaction 16, the processes of Step A to Step G were performed in sequence to prepare the title compound of Formula 1-17.

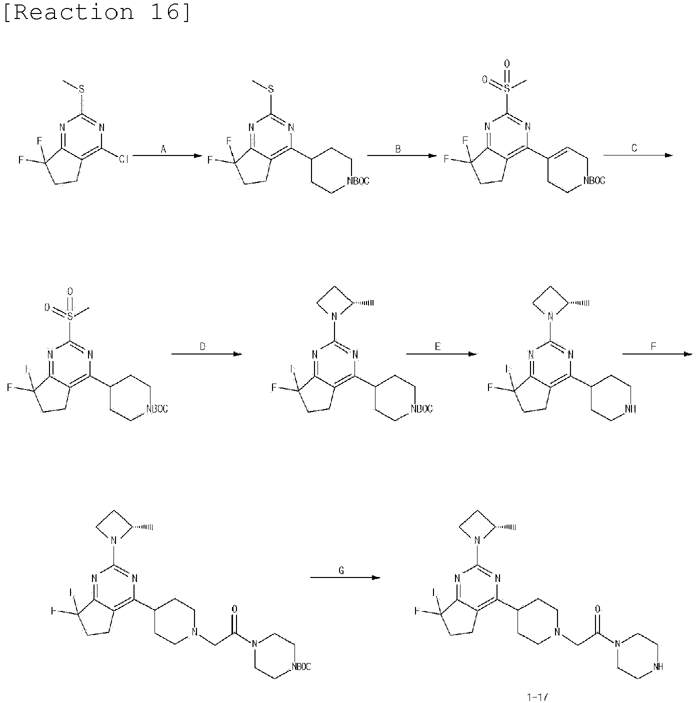

### (1) Step A: Preparation of tert-butyl 4-(7,7-difluoro-2-(methylthio)-6,7-dihydro-5H-cyclopenta[d]pyrimidin-4-yl)-3,6-dihydropyridin-1(2H)-carboxylate

4-Chloro-7,7-difluoro-2-(methylthio)-6,7-dihydro-5H-cyclopenta[d]pyrimidine (577 mg, 2.438 mmol), tert-butyl 4-(4,4,5,5-tetramethyl-1,3,2-dioxaborolan-2-yl)-3,6-dihydropyridin-1(2H)-carboxylate (1131 mg, 3.66 mmol), tetrakis(triphenylphosphine)palladium(O) (141 mg, 0.122 mmol) and a 2 M aqueous sodium carbonate solution (3.66 ml, 7.31 mmol) were dissolved in 30 ml of 1,4-dioxane, followed by stirring at 80°C for 18 hours. After finishing the reaction, the reaction solution was cooled to room temperature and extracted with ethyl acetate. An organic layer was dried over anhydrous magnesium sulfate and filtered. The filtrate was concentrated under a reduced pressure and purified by column chromatography (ethyl acetate/hexane=4/1 by a weight ratio) to obtain tert-butyl 4-(7,7-difluoro-2-(methylthio)-6,7-dihydro-5H-cyclopenta[d]pyrimidin-4-yl)-3,6-dihydropyridin-1(2H)-carboxylate (1.104 g, yield 99%), which was confirmed through ¹H NMR analysis.

¹H NMR (400MHz, CDCl₃) δ 6.52 (m, 1H), 4.17 (d, J=2.3 Hz, 2H), 3.61 (t, J=5.7 Hz, 2H), 3.05-3.10 (m, 2H), 2.68 (d, J=1.4 Hz, 2H), 2.53-2.64 (m, 5H), 1.48 (s, 9H).

### (2) Step B: Preparation of tert-butyl 4-(7,7-difluoro-2-(methylsulfonyl)-6,7-dihydro-5H-cyclopenta[d]pyrimidin-4-yl)-3,6-dihydropyridin-1(2H)-carboxylate

The tert-butyl 4-(7,7-difluoro-2-(methylthio)-6,7-dihydro-5H-cyclopenta[d]pyrimidin-4-yl)-3,6-dihydropyridin-1(2H)-carboxylate (1.1 g, 2.87 mmol) prepared in Step A, and m-chlorobenzoicperoxy acid (70 wt%, 1.556 mg, 6.31 mmol) were dissolved in 100 ml of dichloromethane, followed by stirring at room temperature for 18 hours. After finishing the reaction, water and sodium thiosulfate were added to the reaction solution, and the resultant was stirred. Dichloromethane and an aqueous saturated sodium bicarbonate solution were added thereto, and extraction was performed. An organic layer was dried over anhydrous magnesium sulfate and filtered. The filtrate was concentrated under a reduced pressure to obtain tert-butyl 4-(7,7-difluoro-2-(methylsulfonyl)-6,7-dihydro-5H-cyclopenta[d]pyrimidin-4-yl)-3,6-dihydropyridin-1(2H)-carboxylate (552 mg, yield 46%), which was confirmed through ¹H NMR analysis.

¹H NMR (400MHz, CDCl₃) δ 6.74 (s, 1H), 4.23 (br, s, 2H), 3.64 (t, J=5.5 Hz, 2H), 3.41 (s, 3H), 3.25 (m, 2H), 2.65-2.76 (m, 4H), 1.48 (s, 9H).

### (3) Step C: Preparation of tert-butyl 4-(7,7-difluoro-2-(methylsulfonyl)-6,7-dihydro-5H-cyclopenta[d]pyrimidin-4-yl)piperidin-1-carboxylate

The tert-butyl 4-(7,7-difluoro-2-(methylsulfonyl)-6,7-dihydro-5H-cyclopenta[d]pyrimidin-4-yl)-3,6-dihydropyridin-1(2H)-carboxylate (456 mg, 1.098 mmol) prepared in Step C was dissolved in 20 ml of methanol, and palladium/charcoal (10 wt%, 50 mg) was added thereto, followed by stirring at room temperature for 18 hours. After finishing the reaction, the reaction solution was filtered using celite and then, washed with methanol. The filtrate was concentrated under a reduced pressure to obtain tert-butyl 4-(7,7-difluoro-2-(methylsulfonyl)-6,7-dihydro-5H-cyclopenta[d]pyrimidin-4-yl)piperidin-1-carboxylate (473 mg, yield 99%), which was confirmed through ¹H NMR analysis.

¹H NMR (400MHz, CDCl₃) δ 4.30 (m, 2H), 3.40 (s, 3H), 3.12-3.15 (m, 2H), 2.98-3.04 (m, 1H), 2.67-2.86 (m, 4H), 1.95 (m, 2H), 1.80 (m, 2H), 1.47 (s, 9H).

### (4) Step D: Preparation of tert-butyl (S)-4-(7,7-difluoro-2-(2-methylazetidin-1-yl)-6,7-dihydro-5H-cyclopenta[d]pyrimidin-4-yl)piperidin-1-carboxylate

The tert-butyl 4-(7,7-difluoro-2-(methylsulfonyl)-6,7-dihydro-5H-cyclopenta[d]pyrimidin-4-yl)piperidin-1-carboxylate (150 mg, 0.359 mmol) prepared in Step C, (2S)-2-methylazetidine[(1R,4S)-7,7-dimethyl-2-oxo-norbornan-1-yl]methanesulfonate (120 mg, 0.395 mmol), and potassium carbonate (124 mg, 0.898 mmol) were dissolved in 20 ml of acetonitrile, followed by stirring at 80°C for 4 hours. After finishing the reaction, the reaction solution was cooled to room temperature and extracted with water and ethyl acetate. An organic layer was dried over anhydrous magnesium sulfate and filtered. The filtrate was concentrated under a reduced pressure and purified by column chromatography to obtain tert-butyl (S)-4-(7,7-difluoro-2-(2-methylazetidin-1-yl)-6,7-dihydro-5H-cyclopenta[d]pyrimidin-4-yl)piperidin-1-carboxylate (140 mg, yield 95%), which was confirmed through ¹H NMR analysis.

¹H NMR (400MHz, CDCl₃) δ 4.48 (m, 1H), 3.98-4.20 (m, 5H), 2.81 (m, 4H), 2.69 (m, 1H), 2.44-2.57 (m, 3H), 2.01 (m, 1H), 1.71-1.82 (m, 4H), 1.50 (d, J=5.9 Hz, 3H), 1.47 (s, 9H).

### (5) Step E: Preparation of (S)-7,7-difluoro-2-(2-methylazetidin-1-yl)-4-(piperidin-4-yl)-6,7-dihydro-5H-cyclopenta[d]pyrimidine

The tert-butyl (S)-4-(7,7-difluoro-2-(2-methylazetidin-1-yl)-6,7-dihydro-5H-cyclopenta[d]pyrimidin-4-yl)piperidin-1-carboxylate (140 mg, 0.343 mmol) prepared in Step D was dissolved in 4 ml of dichloromethane, and 0.5 ml of trifluoroacetic acid was added thereto, followed by stirring. After finishing the reaction, the reaction solution was concentrated under a reduced pressure, and the concentrate was neutralized with an aqueous saturated sodium bicarbonate solution and extracted with dichloromethane. An organic layer was dried over anhydrous magnesium sulfate and filtered. The filtrate was concentrated under a reduced pressure to obtain (S)-7,7-difluoro-2-(2-methylazetidin-1-yl)-4-(piperidin-4-yl)-6,7-dihydro-5H-cyclopenta[d]pyrimidine (86 mg, yield 82%), which was confirmed through ¹H NMR analysis.

¹H NMR (400MHz, CDCl₃) δ 4.48 (q, J=6.9 Hz, 1H), 3.96-4.12 (m, 2H), 3.20 (d, J=13.3 Hz, 2H), 2.80 (m, 2H), 2.66-2.76 (m, 3H), 2.37-2.57 (m, 3H), 1.93-2.01 (m, 1H), 1.76-1.86 (m, 2H), 1.70 (m, 2H), 1.52 (d, J=6.4 Hz, 3H).

### (6) Step F: Preparation of tert-butyl (S)-4-(2-(7,7-difluoro-2-(2-methylazetidin-1-yl)-6,7-dihydro-5H-cyclopenta[d]pyrimidin-4-yl)piperidin-1-yl)acetyl)piperazin-1-carboxylate

The (S)-7,7-difluoro-2-(2-methylazetidin-1-yl)-4-(piperidin-4-yl)-6,7-dihydro-5H-cyclopenta[d]pyrimidine (34 mg, 0.110 mmol) prepared in Step E and tert-butyl 4-(2-bromoacetyl)piperazin-1-carboxylate (45 mg, 0.331 mmol) were dissolved in N,N-dimethylformamide (5 ml), followed by stirring at room temperature for 8 hours. After finishing the reaction, the reaction solution was concentrated under a reduced pressure and extracted with ethyl acetate and water. An organic layer was dried over anhydrous magnesium sulfate and filtered. The filtrate was concentrated under a reduced pressure and purified by column chromatography to obtain tert-butyl (S)-4-(2-(7,7-difluoro-2-(2-methylazetidin-1-yl)-6,7-dihydro-5H-cyclopenta[d]pyrimidin-4-yl)piperidin-1-yl)acetyl)piperazin-1-carboxylate (58 mg, yield 99%), which was confirmed through ¹H NMR analysis.

¹H NMR (400MHz, CDCl₃) δ 4.47 (q, J=6.9 Hz, 1H), 4.06-4.11 (m, 1H), 3.98 (m, 1H), 3.57-3.65 (m, 4H), 3.39-3.46 (m, 4H), 3.20 (s, 2H), 2.93-2.97 (m, 2H), 2.78-2.81 (m, 2H), 2.36-2.57 (m, 4H), 2.15-2.21 (m, 2H), 1.85-2.01 (m, 3H), 1.71 (m, 2H), 1.50 (d, J=6.4 Hz, 3H), 1.45 (s, 9H).

### (7) Step G: Preparation of (S)-2-(4-(7,7-difluoro-2-(2-methylazetidin-1-yl)-6,7-dihydro-5H-cyclopenta[d]pyrimidin-4-yl)piperidin-1-yl)-1-(piperazin-1-yl)ethan-1-one

The tert-butyl (S)-4-(2-(7,7-difluoro-2-(2-methylazetidin-1-yl)-6,7-dihydro-5H-cyclopenta[d]pyrimidin-4-yl)piperidin-1-yl)acetyl)piperazin-1-carboxylate (58 mg, 0.108 mmol) prepared in Step F was dissolved in 4 ml of dichloromethane, and 1 ml of trifluoroacetic acid was added thereto, followed by stirring. After finishing the reaction, the reaction solution was concentrated under a reduced pressure, and the resultant was neutralized with an aqueous saturated sodium bicarbonate solution and extracted with dichloromethane. An organic layer was dried over anhydrous magnesium sulfate and filtered, and the filtrate was concentrated under a reduced pressure and purified by column chromatography to obtain (S)-2-(4-(7,7-difluoro-2-(2-methylazetidin-1-yl)-6,7-dihydro-5H-cyclopenta[d]pyrimidin-4-yl)piperidin-1-yl)-1-(piperazin-1-yl)ethan-1-one (32 mg, yield 68%), which was confirmed through ¹H NMR analysis.

¹H NMR (400MHz, CDCl₃) δ 4.70 (br, 1H), 4.46 (q, J=6.7 Hz, 1H), 4.07 (td, J=8.9, 5.0 Hz, 1H), 3.97 (q, J=8.2 Hz, 1H), 3.59-3.72 (m, 4H), 3.20 (s, 2H), 2.95-2.98 (m, 4H), 2.78 (t, J=3.4 Hz, 2H), 2.35-2.57 (m, 6H), 2.19 (t, J=11.7 Hz, 2H), 1.87-2.00 (m, 3H), 1.71 (d, J=12.8 Hz, 2H), 1.49 (q, J=2.9 Hz, 3H).

### Example 18: Preparation of 2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)-1-(piperazin-1-yl)ethan-1-one

As in Reaction 17, the processes of Steps A and B were performed in sequence to prepare the title compound of Formula 1-18.

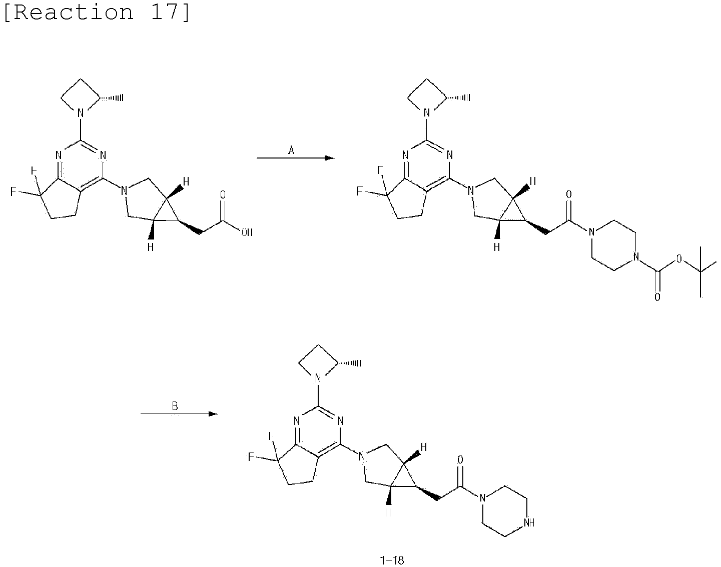

### (1) Step A: Preparation of tert-butyl 4-(2-((1R,5S,6R)-2-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)acetyl)piperazin-1-carboxylate

2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)acetate (200 mg, 0.518 mmol, prepared according to Example 9 of KR10-2021-0129607A and used), tert-butyl piperazin-1-carboxylate (116 mg, 0.621 mmol), propylphosphonic anhydride (50 wt%, 0.244 ml, 0.647 mmol) and triethylamine (0.144 ml, 1.035 mmol) were dissolved in 10 ml of dichloromethane, followed by stirring at room temperature for 1 hour. After finishing the reaction, water and dichloromethane were added to the reaction solution, and extraction was performed. An organic layer was dried over anhydrous magnesium sulfate and filtered. The filtrate was concentrated under a reduced pressure, and the concentrate under a reduced pressure was purified by column chromatography to obtain tert-butyl 4-(2-((1R,5S,6R)-2-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)acetyl)piperazin-1-carboxylate (93 mg, yield 34%), which was confirmed through ¹H NMR analysis.

¹H NMR (400MHz, CDCl₃) δ 4.38 (q, J=6.9 Hz, 1H), 3.90-4.05 (m, 4H), 3.58-3.63 (m, 4H), 3.42 (s, 6H), 2.94-3.01 (m, 2H), 2.28-2.46 (m, 5H), 1.87-1.95 (m, 1H), 1.50 (s, 2H), 1.42-1.48 (m, 12H), 0.94 (td, J=6.9, 3.2 Hz, 1H).

### (2) Step B: Preparation of 2-((1R,5S,6R)-2-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)-1-(piperazin-1-yl)ethan-1-one

The tert-butyl 4-(2-((1R,5S,6R)-2-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)acetyl)piperazin-1-carboxylate (93 mmol, 0.175 mmol) prepared in Step A was dissolved in 4 ml of dichloromethane, and 0.5 ml of trifluoroacetic acid was added thereto, followed by stirring at room temperature. After finishing the reaction, the reaction solution was concentrated under a reduced pressure, and the resultant was neutralized using an aqueous saturated sodium bicarbonate solution and extracted with dichloromethane. An organic layer was dried over anhydrous magnesium sulfate and filtered. The filtrate was concentrated under a reduced pressure, and the concentrate under a reduced pressure was purified by column chromatography to obtain 2-((1R,5S,6R)-2-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)-1-(piperazin-1-yl)ethan-1-one (67 mg, yield 88%), which was confirmed through ¹H NMR analysis.

¹H NMR (500MHz, CDCl₃) δ 4.41 (q, J=6.7 Hz, 1H), 3.93-4.07 (m, 4H), 3.61-3.74 (m, 4H), 3.45-3.50 (m, 2H), 3.01 (t, J=3.8 Hz, 2H), 2.93 (d, J=4.3 Hz, 4H), 2.32-2.51 (m, 5H), 1.90-1.97 (m, 1H), 1.54 (s, 2H), 1.50 (d, J=6.1 Hz, 3H), 0.95 (td, J=6.7, 3.5 Hz, 1H).

### Example 19: Preparation of 2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)-1-(4-methylpiperazin-1-yl)ethan-1-one

The same procedure in Step A of Example 18 was performed except for using 2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)acetate in 50 mg (0.13 mmol), and using 1-methylpiperazine (19 mg, 0.19 mmol) instead of piperazin-1-carboxylate in Step A of Example 18, to obtain 2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)-1-(4-methylpiperazin-1-yl)ethan-1-one of Formula 1-19 (5.0 mg, 0.01 mmol, yield 8%), which was confirmed through ¹H NMR analysis.

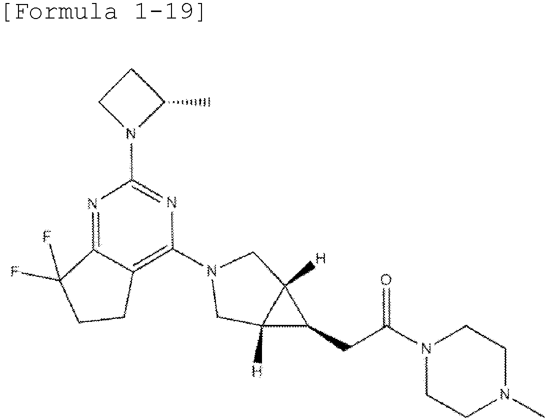

¹H NMR (400MHz, MeOH-d₄) δ 4.40 (q, J=6.7 Hz, 1H), 4.12-3.96 (m, 3H), 3.87 (q, J=8.2 Hz, 1H), 3.61 (s, 4H), 3.58-3.47 (m, 2H), 3.06 (d, J=3.7 Hz, 2H), 2.52-2.34 (m, 9H), 2.31 (s, 3H), 1.93 (t, J=9.5 Hz, 1H), 1.62-1.49 (m, 2H), 1.49-1.40 (m, 3H), 0.94-0.79 (m, 1H).

### Example 20: Preparation of 4-(2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)acetyl)piperazin-2-one

The same procedure in Step A of Example 18 was performed except for using 2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)acetate in 50 mg (0.13 mmol), and using 2-oxopiperazine (19 mg, 0.19 mmol) instead of piperazin-1-carboxylate in Step A of Example 18, to obtain 4-(2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)acetyl)piperazin-2-one of Formula 1-20 (47 mg, 0.10 mmol, yield 81%), which was confirmed through ¹H NMR analysis.

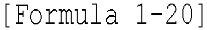

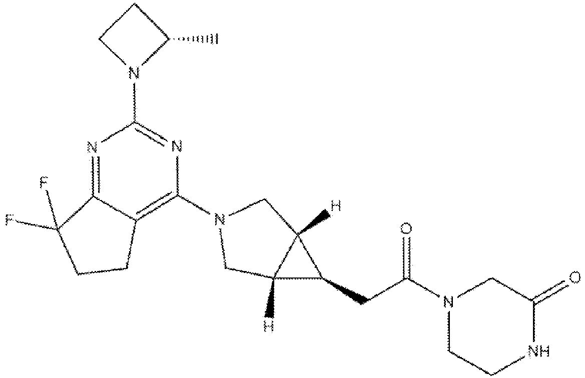

¹H NMR (400MHz, MeOH-d₄) δ 4.39 (t, J=7.0 Hz, 1H), 4.14 (s, 2H), 4.10-3.93 (m, 3H), 3.87 (d, J=7.6 Hz, 1H), 3.74 (dt, J=27.5, 5.3 Hz, 2H), 3.64 (s, 2H), 3.43-3.32 (m, 1H), 3.07 (d, J=12.2 Hz, 2H), 2.55-2.21 (m, 5H), 1.93 (s, 1H), 1.57 (s, 2H), 1.48 (d, J=6.1 Hz, 3H), 1.37 (d, J=7.0 Hz, 1H), 0.90 (s, 1H).

### Example 21: Preparation of 2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.0]heptan-6-yl)-1-(piperazin-1-yl)ethan-1-one

As in Reaction 18, the processes of Steps A and B were performed in sequence to prepare the title compound of Formula 1-21.

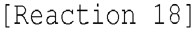

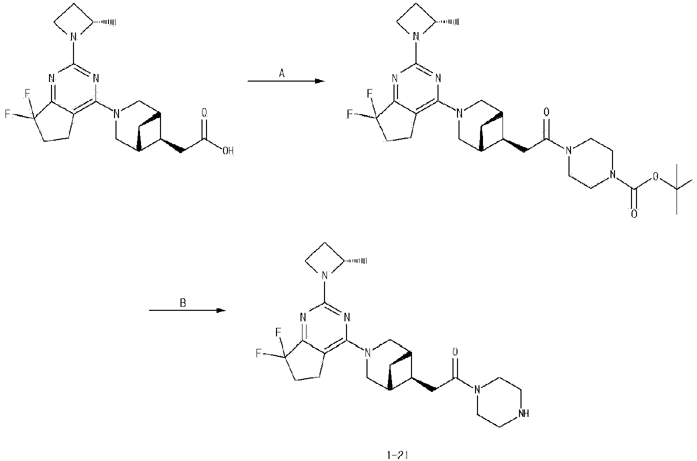

### (1) Step A: Preparation of tert-butyl 4- (2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.0]heptan-6-yl)acetyl)piperazin-1-carboxylate

2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetic acid (177 mg, 0.468 mmol, prepared according to Example 8 of KR10-2021-0129607A and used), tert-butyl piperazin-1-carboxylate (87 mg, 0.468 mmol), and N-[(dimethylamino)-1H-1,2,3-triazol-[4-5-b]pyridin-1-ylmethylene]-N-methylmethaneaminium hexafluorophosphate N-oxide (213 mg, 0.561 mmol) were dissolved in 10 ml of dimethylformamide, followed by stirring at room temperature for 2 hours. After finishing the reaction, ethyl acetate and water were added to the reaction solution, and extraction was performed. An organic layer was dried over anhydrous magnesium sulfate and filtered. The filtrate was concentrated under a reduced pressure, and the concentrate under a reduced pressure was purified by column chromatography to obtain tert-butyl 4-(2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetyl)piperazin-1-carboxylate (272 mg, yield 99%), which was confirmed through ¹H NMR analysis.

¹H NMR (400MHz, CDCl₃) δ 4.40 (q, J=6.9 Hz, 1H), 3.92-4.10 (m, 5H), 3.58 (m, 2H), 3.39-3.46 (m, 6H), 3.14 (m, 2H), 2.68 (d, J=7.8 Hz, 2 H), 2.29-2.46 (m, 6H), 2.11 (m, 1H), 1.87-1.96 (m, 1H), 1.46-1.51 (m, 12H), 1.41 (m, 1H).

### (2) Step B: Preparation of 2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)-1-(piperazin-1-yl)ethan-1-one

The tert-butyl 4-(2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetyl)piperazin-1-carboxylate (270 mg, 0.464 mmol) prepared in Step A was dissolved in 1 ml of dichloromethane, and 1 ml of trifluoroacetic acid was added thereto, followed by stirring at room temperature. After finishing the reaction, the reaction solution was concentrated under a reduced pressure, and the resultant was neutralized and extracted using dichloromethane and an aqueous saturated sodium bicarbonate solution. An organic layer was dried over anhydrous magnesium sulfate and filtered. The filtrate was concentrated under a reduced pressure, and the concentrate under a reduced pressure was purified by column chromatography to obtain 2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)-1-(piperazin-1-yl)ethan-1-one (210 mg, yield 95%), which was confirmed through ¹H NMR analysis.

¹H NMR (500MHz, CDCl₃) δ 4.40 (m, 1H), 3.92-4.07 (m, 6H), 3.73 (m, 2H), 3.63 (s, 2H), 3.15 (m, 2H), 2.94-3.02 (m, 4H), 2.67 (d, J=7.8 Hz, 2H), 2.29-2.46 (m, 6H), 2.10 (m, 1H), 1.88-1.96 (m, 1H), 1.48 (d, J=5.9 Hz, 3H), 1.42 (dd, J=9.1, 5.5 Hz, 1H).

### Example 22: Preparation of 1-(4-acetylpiperazin-1-yl)-2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)ethan-1-one

The 2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)-1-(piperazin-1-yl)ethan-1-one (30 mg, 0.067 mmol) prepared according to Example 21 and acetic anhydride (9.51 ul, l 0.101 mmol) were dissolved in 4 ml of dichloromethane and cooled to 0°C. Triethylamine (18.73 ul, 0.134 mmol) was added thereto, followed by stirring at room temperature for 1 hour. After finishing the reaction, dichloromethane and an aqueous saturated sodium bicarbonate solution were added to the reaction solution, and extraction was performed. An organic layer was dried over anhydrous magnesium sulfate and filtered. The filtrate was concentrated under a reduced pressure, and the concentrate under a reduced pressure was purified by column chromatography to obtain the title compound of Formula 1-22 (17 mg, yield 52%), which was confirmed through ¹H NMR analysis.

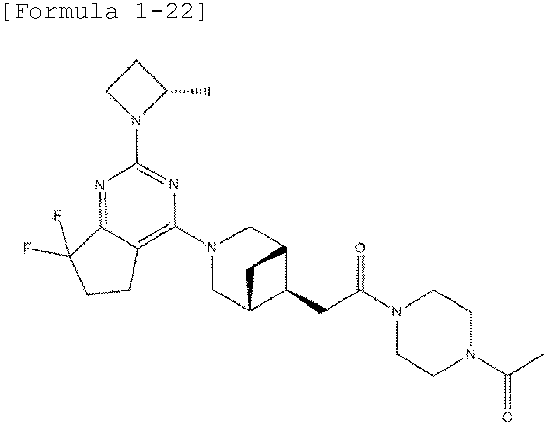

¹H NMR (500MHz, CDCl₃) δ 4.42-4.47 (m, 1H), 3.97-4.10 (m, 6H), 3.66 (m, 4H), 3.49-3.55 (m, 4H), 3.18 (m, 2H), 2.73 (d, J=7.6 Hz, 2H), 2.34-2.48 (m, 6H), 2.16 (s, 3H), 1.92-1.99 (m, 1H), 1.51 (d, J=6.1 Hz, 3H), 1.46 (q, J=4.8 Hz, 1H).

### Example 23: Preparation of 2-((1R,5S,6S)-3-(8,8-difluoro-2-((S)-2-methylazetidin-1-yl)-5,6,7,8-tetrahydroquinazolin-4-yl)-3-azabicyclo[3.1.1]heptan-6-yl)-1-(piperazin-1-yl)ethan-1-one

The same procedure in Example 21 was performed except for using 2-((1R,5S,6S)-3-(8,8-difluoro-2-((S)-2-methylazetidin-1-yl)-5,6,7,8-tetrahydroquinazolin-4-yl)-3-azabicyclo[3.1.1]heptan-6-yl)acetic acid (68 mg, 0.164 mmol) instead of 2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetic acid, and using tert-butyl piperazin-1-carboxylate in 33.6 mg (0.180 mmol) in Step A of Example 21, to prepare the title compound of Formula 1-23 (69 mg. 0.150 mmol, yield 91%), which was confirmed through ¹H NMR analysis.

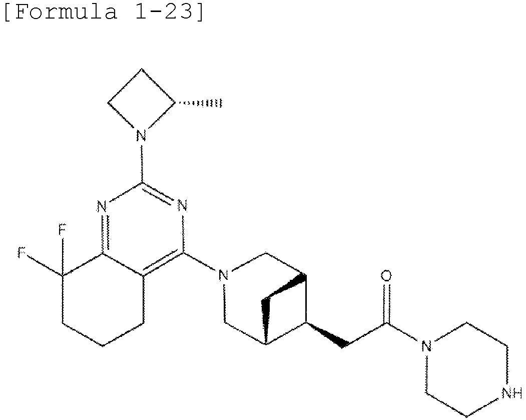

¹H NMR (500MHz, CDCl₃) δ 4.39-4.44 (m, 1H), 3.88-4.09 (m, 6H), 3.65 (t, J=4.9 Hz, 2H), 3.54 (t, J=4.9 Hz, 2H), 3.19 (s, br, 1H), 2.92 (m, 4H), 2.77 (m, 2H), 2.68 (d, J=7.6 Hz, 2H), 2.30-2.38 (m, 4H), 2.19-2.30 (m, 2H), 2.15 (dd, J=13.1, 7.6 Hz, 1H), 1.91-1.97 (m, 1H), 1.83 (d, J=5.8 Hz, 2H), 1.50 (d, J=6.1 Hz, 3H), 1.44 (dd, J=9.0, 5.3 Hz, 1H).

### Example 24: Preparation of 2-((1R,5S,6S)-3-(8,8-difluoro-2-((S)-2-methylazetidin-1-yl)-5,8-dihydro-6H-pyrazol[3,4-d]pyrimidin-4-yl)-3-azabicyclo[3.1.1]heptan-6-yl)-1-(piperazin-1-yl)ethan-1-one

The same procedure in Example 21 was performed except for using 2-((1R,5S,6S)-3-(8,8-difluoro-2-((S)-2-methylazetidin-1-yl)-5,8-dihydro-6H-pyrano[3,4-d]pyrimidin-4-yl)-3-azabicyclo[3.1.1]heptan-6-yl)acetic acid (160 mg, 0.384 mmol) instead of 2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetic acid, and using tert-butyl piperazin-1-carboxylate in 75 mg (0.403 mmol) in Step A of Example 21, to prepare the title compound of Formula 1-24 (69 mg. 0.150 mmol, yield 91%), which was confirmed through ¹H NMR analysis.

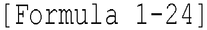

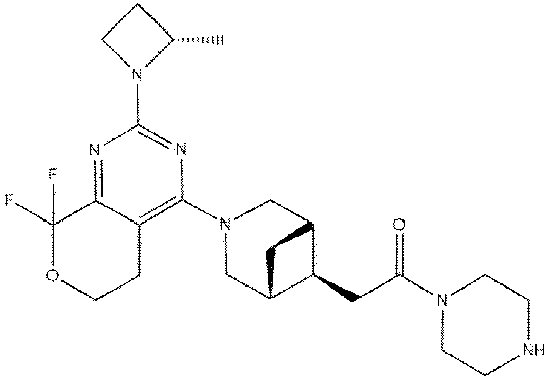

¹H NMR (500MHz, CDCl₃) δ 4.40-4.46 (m, 1H), 4.14 (t, J=5.3 Hz, 2H), 3.94-4.09 (m, 6H), 3.52-3.82 (m, 4H), 2.99-3.11 (m, 6H), 2.70 (d, J=7.3 Hz, 2H), 2.34-2.40 (m, 4H), 2.15 (dd, J=12.4, 7.2 Hz, 1H), 1.92-1.99 (m, 1H), 1.50 (d, J=6.1 Hz, 3H),. 1.42-1.46 (m, 1H).

### Example 25: Preparation of 7,7-difluoro-2-((S)-2-methylazetidin-1-yl)-4-((1R,5S,6S)-6-((piperazin-1-ylsulfonyl)methyl)-3-azabicyclo[3.1.0]hexan-3-yl)-6,7-dihydro-5H-cyclopenta[d]pyrimidine

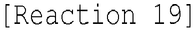

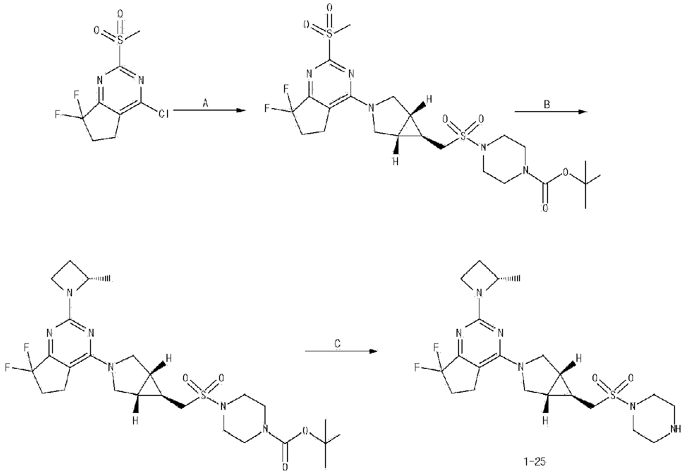

By applying the methods of Step A, Step B and Step D of Example 16, but following Reaction 19 in sequence, the title compound of Formula 1-25 was prepared.

### (1) Step A: Preparation of tert-butyl 4-((((1R,5S,6R)-3-(7,7-difluoro-2-(methylsulfonyl)-6,7-dihydro-5H-cyclopenta[d]pyrimidin-4-yl)-3-azabicyclo[3.1.0]hexan-6-yl)methyl)sulfonyl)piperazin-1-carboxylate

The same procedure in Step A of Example 16 was performed except for using the tert-butyl 4-((((1R,5S,6R)-3-azabicyclo[3.1.0]hexan-6-yl)methyl)sulfonyl)piperazin-1-carboxylate (89 mg, 0.258 mmol) prepared in Preparation Example 10 instead of piperazin-2-one in Step A of Example 16, to prepare the title compound (135 mg, 0.234 mmol), which was confirmed through mass analysis (mass [M+H]=522.1).

### (2) Step B: Preparation of tert-butyl 4-((((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)methyl)sulfonyl)piperazin-1-carboxylate

The same procedure in Step B of Example 16 was applied except for reacting the tert-butyl 4-((((1R,5S,6R)-3-(7,7-difluoro-2-(methylsulfonyl)-6,7-dihydro-5H-cyclopenta[d]pyrimidin-4-yl)-3-azabicyclo[3.1.0]hexan-6-yl)methyl)sulfonyl)piperazin-1-carboxylate (135 mg, 0.234 mmol) prepared in Step A with (2S)-2-methylazetidine[(1R,4S)-7,7-dimethyl-2-oxo-norbornan-1-yl] methanesulfonate in Step B of Example 16, to prepare the title compound (95 mg, 0.167 mmol), which was confirmed through ¹H NMR analysis.

¹H NMR (500MHz, CDCl₃) δ 4.43 (q, J=6.7 Hz, 1H), 3.97-4.09 (m, 4H), 3.64-3.69 (m, 2H), 3.54 (s, 4H), 3.29 (s, 4H), 3.01 (d, J=3.7 Hz, 2H), 2.92-2.95 (m, 2H), 2.43-2.50 (m, 2H), 2.38 (s, 1H), 1.96 (d, J=9.5 Hz, 1H), 1.75 (s, 2H), 1.51 (d, J=6.4 Hz, 3H), 1.49 (s, 8H). 1.07 (dd, J=6.6, 3.6 Hz, 1H).

### (3) Step C: Preparation of 7,7-difluoro-2-((S)-2-methylazetidin-1-yl)-4-((1R,5S,6S)-6-((piperazin-1-ylsulfonyl)methyl)-3-azabicyclo[3.1.0]hexan-3-yl)-6,7-dihydro-5H-cyclopenta[d]pyrimidine

The same procedure in Step D of Example 16 was performed except for using the tert-butyl 4-((((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)methyl)sulfonyl)piperazin-1-carboxylate (95 mg, 0.167 mmol) prepared in Step B, in Step D of Example 16 to prepare the title compound of Formula 1-25 (61 mg, 0.130 mmol), which was confirmed through ¹H NMR analysis.

¹H NMR (500MHz, CDCl₃) δ 4.42 (td, J=13.2, 6.5 Hz, 1H), 4.01-4.08 (m, 3H), 3.97 (q, J=8.2 Hz, 1H), 3.66 (t, J=12.2 Hz, 2H), 3.30 (t, J=4.7 Hz, 4H), 2.98-3.01 (m, 2H), 2.95-2.98 (m, 4H), 2.88-2.93 (m, 2H), 2.40-2.49 (m, 2H), 2.34-2.39 (m, 1H), 1.91-1.98 (m, 1H), 1.79 (s, 1H), 1.75 (s, 2H), 1.46-1.51 (m, 3H), 1.06-1.10 (m, 1H).

### Example 26: Preparation of (S)-2-((1-(5-methyl-2-(2-methylazetidin-1-yl)-6-(trifluoro)pyrimidin-4-yl)azetidin-3-yl)oxy)-1-(piperazin-1-yl)ethan-1-one

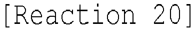

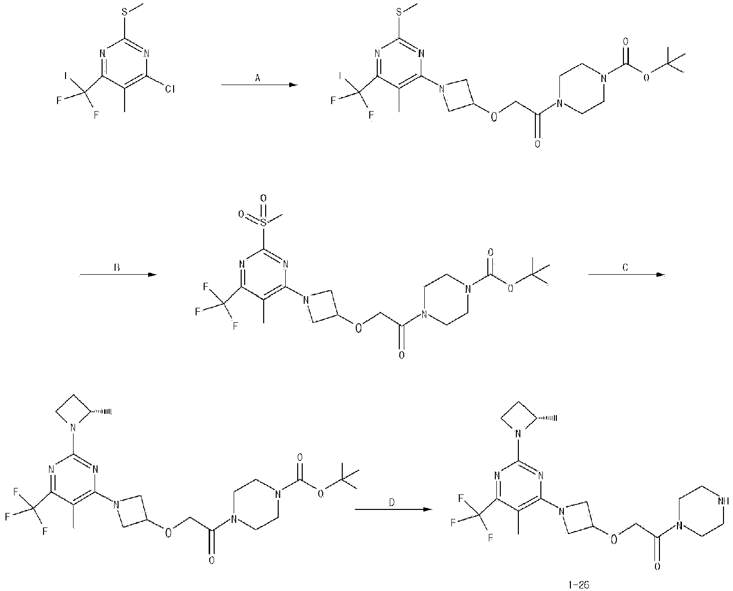

By applying Step A to Step D of Example 2, but following Reaction 20 in sequence, the title compound of Formula 1-26 was prepared.

Particularly, the same procedure in Example 2 was performed except for reacting 4-chloro-5-methyl-2-(methylthio)-6-(trifluoro)pyrimidine (12.0 g, 49.45 mmol) instead of 4,5-dichloro-2-(methylthio)-6-(trifluoromethyl)pyrimidine with tert-butyl 4-(2-(azetidin-3-yloxy)acetyl)piperazin-1-carboxylate (14.8 g, 49.45 mmol) in Step A, and using (2S)-2-methylazetidine[(1R,4S)-7,7-dimethyl-2-oxo-norbornan-1-yl]methanesulfonate in 18.0 g (59.28 mmol) in Step C in Example 2, to prepare the title compound of Formula 1-26 (12.1 g, 28.31 mmol, yield 57%), which was confirmed through ¹H NMR analysis.

¹H NMR (500MHz, MeOH-d₄) δ 4.84 (br, s, 3H), 4.42 (m, 1H). 4.35 (s, 2H), 4.14 (m, 2H), 3.95 (m, 1H), 3.87 (m, 1H), 3.55 (br, s, 2H), 3.44 (br, s, 2H), 2.81 (br, s, 4H), 2.37 (m, 1H). 2.09 (m, 1H), 1.47 (d, 3H).

### Example 27: Preparation of ((S)-2-((1-(2--(2-methylazetidin-1-yl)-6-(trifluoro)pyrimidin-4-yl)piperidin-4-yl)oxy)-1-(piperazin-1-yl)ethan-1-one

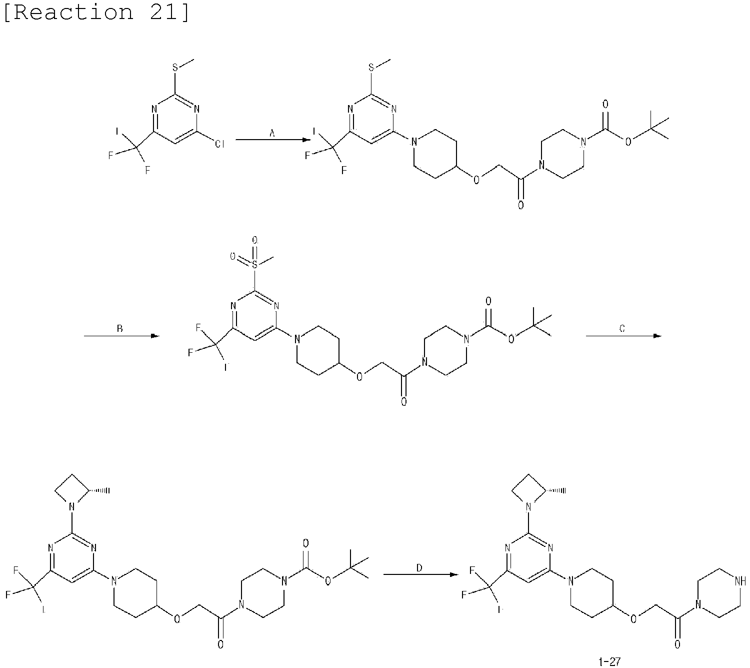

By applying Step A to Step D of Example 2, but performing Reaction 21 in sequence, the title compound of Formula 1-27 was prepared.

Particularly, the same procedure of Example 2 was performed except for reacting 4-chloro-2-(methylthio)-6-(trifluoro)pyrimidine (229 mg, 1.00 mmol) instead of 4,5-dichloro-2-(methylthio)-6-(trifluoromethyl)pyrimidine with tert-butyl 4-(2-(piperidin-4-yloxy)acetyl)piperazin-1-carboxylate (354 mg, 1.08 mmol) prepared in Preparation Example 9 instead of tert-butyl 4-(2-(azetidin-3-yloxy)acetyl)piperazin-1-carboxylate in Step A, and using (2S)-2-methylazetidine[(1R,4S)-7,7-dimethyl-2-oxo-norbornan-1-yl]methanesulfonate in 225 mg (0.744 mmol) in Step C in Example 2, to prepare the title compound of Formula 1-27 (219 mg, 0.50 mmol, yield 50%), which was confirmed through ¹H NMR analysis.

¹H NMR (500MHz, MeOH-d₄) δ 6.37 (s, 1H), 4.42 (q, J=6.5 Hz, 1H), 4.27 (s, 2H), 4.11-3.83 (m, 4H), 3.70 (s, 1H), 3.53 (d, J=23.8 Hz, 4 H), 3.40 (t, J=10.2 Hz, 2H), 2.81 (d, J=14.0 Hz, 4H), 2.49-2.32 (m, 1H), 2.03-1.84 (m, 3H), 1.60 (d, J=8.8 Hz, 2H), 1.48 (d, J=6.1 Hz, 3H).

### Example 28: Preparation of ((S)-2-((1-(5-methyl-2-(2-methylazetidin-1-yl)-6-(trifluoro)pyrimidin-4-yl)piperidin-4-yl)oxy)-1-(piperazin-1-yl)ethan-1-one

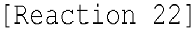

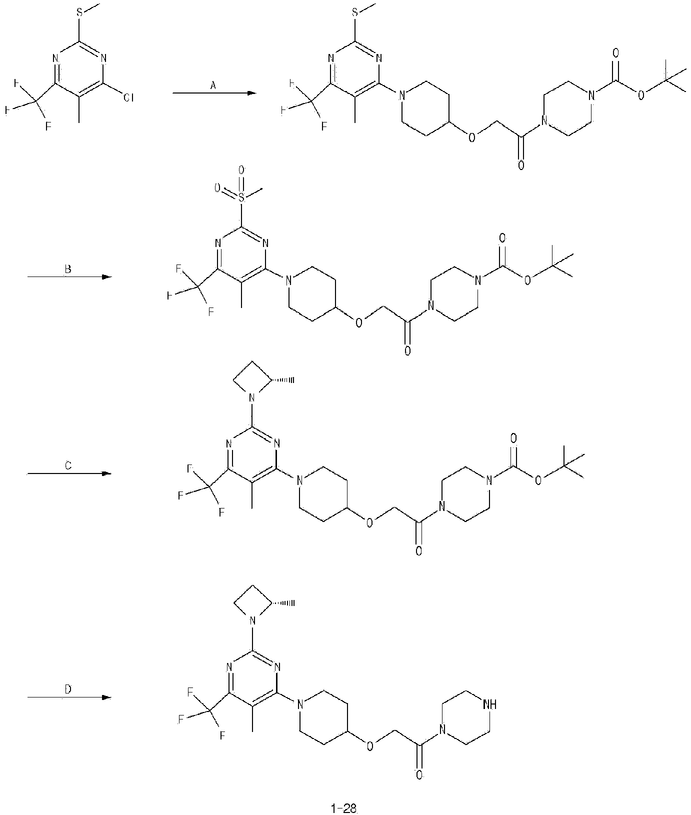

By applying the methods of Step A to Step D of Example 2, but performing Reaction 22 in sequence, the title compound of Formula 1-28 was prepared.

Particularly, the same procedure in Example 2 was performed except for reacting 4-chloro-5-methyl-2-(methylthio)-6-(trifluoro)pyrimidine (243 mg, 1.00 mmol) instead of 4,5-dichloro-2-(methylthio)-6-(trifluoromethyl)pyrimidine with tert-butyl 4-(2-(piperidin-4-yloxy)acetyl)piperazin-1-carboxylate (354 mg, 1.08 mmol) prepared in Preparation Example 9 instead of tert-butyl 4-(2-(azetidin-3-yloxy)acetyl)piperazin-1-carboxylate in Step A, and using (2S)-2-methylazetidine[(1R,4S)-7,7-dimethyl-2-oxo-norbornan-1-yl]methanesulfonate in 264 mg (0.87 mmol) in Step C in Example 2, to prepare the title compound of Formula 1-28 (213 mg, 0.47 mmol, yield 47%), which was confirmed through ¹H NMR analysis.

¹H NMR (500MHz, MeOH-d₄) δ 4.48-4.32 (m, 1H), 4.26 (s, 2H), 4.04-3.81 (m, 2H), 3.65 (s, 3H), 3.53 (d, J=18.9 Hz, 4H), 3.11 (t, J=9.6 Hz, 2H), 2.82 (d, J=16.2 Hz, 4H), 2.41 (d, J=13.7 Hz, 1H), 2.14 (s, 3H), 2.09-1.84 (m, 3H), 1.71 (q, J=8.8 Hz, 2H), 1.48 (d, J=6.1 Hz, 3H).

### Preparation Example 11

Step A to Step K were performed in sequence to prepare 4-chloro-7,7-difluoro-2-(methylthio)-6,7-dihydro-5H-cyclopenta[d]pyrimidine of Formula k.

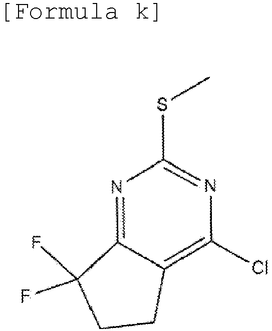

### (1) Step A: Preparation of 5-(tert-butyl) 1-ethyl 2,2-difluoropentanedioate

Ethyl 2-bromo-2,2-difluoroacetate (20 g, 99 mmol), tert-butyl acrylate (11.1 ml, 76 mmol), copper (9.6 g, 152 mmol), N1,N1,N2,N2-tetramethylethan-1,2-diamine (5.7 ml, 37.9 mmol) and acetic acid (4.8 ml, 83 mmol) were dissolved in 100 ml of tetrahydrofuran, followed by stirring at 50°C for 4 hours. After finishing the reaction, an aqueous saturated ammonium chloride solution was added to the reaction solution, and the resultant was extracted with ethyl acetate. An organic layer was washed with an aqueous sodium chloride solution, dried over anhydrous magnesium sulfate and filtered. The filtrate was concentrated under a reduced pressure to obtain 5-(tert-butyl) 1-ethyl 2,2-difluoropentanedioate. The synthesis of the 5-(tert-butyl) 1-ethyl 2,2-difluoropentanedioate thus obtained was confirmed through ¹H NMR analysis.

¹H NMR (400 MHz, CDCl₃) δ 4.31 (q, J=7.0 Hz, 2H), 2.32-2.47 (m, 4H), 1.43 (s, 9H), 1.34 (t, J=7.3 Hz, 3H).

### (2) Step B: Preparation of 5-ethoxy-4,4-difluoro-5-oxopentanoic acid

The 5-(tert-butyl) 1-ethyl 2,2-difluoropentanedioate obtained in Step A was dissolved in trifluoroacetic acid (30 ml) and dichloromethane (70 ml), followed by stirring at room temperature for 8 hours. After finishing the reaction, the resultant was concentrated under a reduced pressure. To the concentrate, an aqueous saturated sodium bicarbonate solution was added, and the resultant was extracted with ethyl acetate. An organic layer was washed with an aqueous sodium chloride solution, dried over anhydrous magnesium sulfate and filtered. The filtrate was concentrated under a reduced pressure to obtain 5-ethoxy-4,4-difluoro-5-oxopentanoic acid (15 g, 76 mmol, yield 100%). The synthesis of the 5-ethoxy-4,4-difluoro-5-oxopentanoic acid thus obtained was confirmed through ¹H NMR analysis.

¹H NMR (500MHz, CDCl₃) δ 4.36 (q, J=7.2 Hz, 2H), 2.66 (t, J=7.6 Hz, 2H), 2.42-2.51 (m, 2H), 1.39 (t, J=7.2 Hz, 3H).

### (3) Step C: Preparation of ethyl 2,2-difluoro-5-hydroxypentanoate

The 5-ethoxy-4,4-difluoro-5-oxopentanoic acid (12.0 g, 61 mmol) prepared in Step B and a borane dimethylsulfide tetrahydrofuran solution (2 M 36.6 ml. 73 mmol) were dissolved in 300 ml of tetrahydrofuran, followed by stirring at 50°C for 12 hours. After finishing the reaction, water (10 ml) was added to the reaction solution, followed by stirring at 60°C for 1 hour. After finishing the reaction, water was added to the reaction solution, and the resultant was extracted with ethyl acetate. An organic layer was washed with an aqueous sodium chloride solution, dried over anhydrous magnesium sulfate and filtered. The filtrate was concentrated under a reduced pressure to obtain ethyl 2,2-difluoro-5-hydroxypentanoate (6.5 g, 35.7 mmol, yield 59%). The synthesis of the ethyl 2,2-difluoro-5-hydroxypentanoate thus obtained was confirmed through ¹H NMR analysis.

¹H NMR (500MHz, CDCl₃) δ 4.35 (q, J=7.1 Hz, 2H), 3.74 (t, J=6.3 Hz, 2H), 2.16-2.26 (m, 2H), 1.72-1.81 (m, 2H), 1.38 (t, J=7.2 Hz, 3H).

### (4) Step D: Preparation of ethyl 2,2-difluoro-5-((methanesulfonyl)oxy)pentanoate

The ethyl 2,2-difluoro-5-hydroxypentanoate (9.4 g, 51.6 mmol) obtained in Step C, methanesulfonyl chloride (4.42 ml, 56.8 mmol), diisopropylethylamine (18.0 ml, 103 mmol) and 4-dimethylaminopyridine (315 mg, 2.58 mmol) were dissolved in dichloromethane (200 ml), followed by stirring at room temperature for 3 hours. After finishing the reaction, a N hydrochloric acid solution was added to the reaction solution, followed by extracting with ethyl acetate. An organic layer was washed with an aqueous sodium chloride solution, dried over anhydrous magnesium sulfate and filtered. The filtrate was concentrated under a reduced pressure and purified by column chromatography (ethyl acetate/hexane = 1/3 by a weight ratio) to obtain ethyl 2,2-difluoro-5-((methanesulfonyl)oxy)pentanoate (13.3 g, 51.1 mmol, yield 99%). The synthesis of the ethyl 2,2-difluoro-5-((methanesulfonyl)oxy)pentanoate thus obtained was confirmed through ¹H NMR analysis.

¹H NMR (500MHz, CDCl₃) δ 4.37 (q, J=7.1 Hz, 2H), 4.31 (t, J=6.3 Hz, 2H), 3.06 (s, 3H), 2.20-2.29 (m, 2H), 1.99-2.05 (m, 2H), 1.38-1.40 (m, 3H).

### (5) Step E: Preparation of ethyl 5-cyano-2,2-difluoropentanoate

The ethyl 2,2-difluoro-5-((methanesulfonyl)oxy)pentanoate (13.3 g, 51.1 mmol) prepared in Step D, and potassium cyanate (10,0 g, 153.3 mmol) were dissolved in a mixture solution of 150 ml of tetrahydrofuran and 150 ml of dimethylformamide, followed by stirring at 70°C for 18 hours. After finishing the reaction, the reaction solution was concentrated under a reduced pressure. To the concentrate, an aqueous saturated ammonium chloride solution was added, and extraction with ethyl acetate was performed. An organic layer was washed with aqueous sodium chloride solution, dried over anhydrous magnesium sulfate and filtered. The filtrate was concentrated under a reduced pressure and purified by column chromatography (ethyl acetate/hexane = 1/1 by a weight ratio) to obtain ethyl 5-cyano-2,2-difluoropentanoate (7.1 g, 37.1 mmol, yield 73%). The synthesis of the ethyl 5-cyano-2,2-difluoropentanoate thus obtained was confirmed through ¹H NMR analysis.

¹H NMR (400MHz, CDCl₃) δ 4.27-4.35 (m, 2H), 2.45 (t, J=7.1 Hz, 2H), 2.15-2.29 (m, 2H), 1.91 (t, 7.1 Hz, 2H), 1.31-1.37 (m, 3H).

### (6) Step F: Preparation of diethyl 2,2-difluorohexanedioate

The ethyl 5-cyano-2,2-difluoropentanoate (7.1 g, 37.7 mmol) prepared in Step E and acetyl chloride (30 ml) were dissolved in 60 ml of ethanol, followed by refluxing for 48 hours. After finishing the reaction, the resultant was concentrated under a reduced pressure. To the concentrate, an aqueous saturated sodium bicarbonate solution was added, and extraction with ethyl acetate was performed. An organic layer was washed with aqueous sodium chloride solution, dried over anhydrous magnesium sulfate and filtered. The filtrate was concentrated under a reduced pressure to obtain diethyl 2,2-difluorohexanedioate (6.5 g, 27.3 mmol, yield 74%). The synthesis of the diethyl 2,2-difluorohexanedioate thus obtained was confirmed through ¹H NMR analysis.

¹H NMR (500MHz, CDCl₃) δ 4.35 (q. J=7.1 Hz, 2H), 4.16 (q, J=7.1 Hz, 2H), 2.40 (t, J=7.3 Hz, 2H), 2.07-2.20 (m, 2H), 1.86 (m, 2H), 1.38 (t, J=7.2 Hz, 3H), 1.28 (t, J=7.2 Hz, 3H).

### (7) Step G: Preparation of ethyl 3,3-difluoro-2-oxocyclopentan-1-carboxylate

The diethyl 2,2-difluorohexanedioate (51.57 g, 216 mmol) prepared in Step F was dissolved in tetrahydrofuran (1000 ml), and potassium tert-butoxide (26.7 g, 238 mmol) was slowly added thereto, followed by stirring at room temperature for 4 hours. After finishing the reaction, 240 ml of a 1 N hydrochloric acid solution was slowly added to the reaction solution at 0°C, followed by stirring for about 10 minutes. The reaction solution was diluted in ethyl acetate, and an organic layer was washed with an aqueous sodium chloride solution. An aqueous solution was extracted with ethyl acetate, and an organic layer collected was dried over anhydrous magnesium sulfate and filtered. The filtrate was concentrated under a reduced pressure and sufficiently dried under a reduced pressure state to obtain ethyl 3,3-difluoro-2-oxocyclopentan-1-carboxylate (41.6 g, 216 mmol, yield 99%), and this product was used in the next reaction without separate purification.

### (8) Step H: Preparation of 7,7-difluoro-2-(methylthio)-6,7-dihydro-5H-cyclopenta[d]pyrimidin-4-ol

The ethyl 3,3-difluoro-2-oxocyclopentan-1-carboxylate (540 mg, 2.41 mmol) prepared in Step G, methylisothioura hemisulfate (672 mg, 4.82 mmol) and sodium carbonate (767 mg, 7.24 mmol) were dissolved in 50 ml of water, followed by stirring at room temperature for 12 hours. After finishing the reaction, an excessive amount of water was added to the reaction solution, and extraction with ethyl acetate was performed. An organic layer was washed with an aqueous sodium chloride solution, dried over anhydrous magnesium sulfate and filtered. The filtrate was concentrated under a reduced pressure and purified by column chromatography (ethyl acetate/hexane = 1/1 by a weight ratio) to obtain 7,7-difluoro-2-(methylthio)-6,7-dihydro-5H-cyclopenta[d]pyrimidin-4-ol (250 mg, 1.15 mmol, yield 48%). The synthesis of the 7,7-difluoro-2-(methylthio)-6,7-dihydro-5H-cyclopenta[d]pyrimidin-4-ol thus obtained was confirmed through ¹H NMR analysis.

¹H NMR (500MHz, DMSO-D₆) δ 1.92-1.97 (m, 2H), 1.81 (s, 3H), 1.71-1.79 (m, 2H).

### (9) Step I: Preparation of 4-chloro-7,7-difluoro-2-(methylthio)-6,7-dihydro-5H-cyclopenta[d]pyrimidine

The 7,7-difluoro-2-(methylthio)-6,7-dihydro-5H-cyclopenta[d]pyrimidin-4-ol (5.2 g, 23.83 mmol) prepared in Step H, phosphoryl trichloride (22.21 ml, 238 mmol) and benzyltriethylammonium chloride (5.43 g, 23.93 mmol) were dissolved in 150 ml of dichloroethane, followed by stirring at 80°C for 3 hours. After finishing the reaction, the reaction solution was distilled under a reduced pressure. To the concentrate, a saturated sodium bicarbonate solution was added, and extraction with ethyl acetate was performed. An organic layer was washed with an aqueous sodium chloride solution, dried over anhydrous magnesium sulfate and filtered. The filtrate was concentrated under a reduced pressure and purified by column chromatography (ethyl acetate/hexane = 1/10 by a weight ratio) to obtain 4-chloro-7,7-difluoro-2-(methylthio)-6,7-dihydro-5H-cyclopenta[d]pyrimidine (5.00 g, 23.83 mmol, yield 89%). The synthesis of the 4-chloro-7,7-difluoro-2-(methylthio)-6,7-dihydro-5H-cyclopenta[d]pyrimidine thus obtained was confirmed through ¹H NMR analysis.

¹H NMR (500MHz, CDCl₃) δ 2.99-3.03 (m, 2H), 2.62-2.71 (m, 5H).

### Preparation Example 12

The 4-chloro-7,7-difluoro-2-(methylthio)-6,7-dihydro-5H-cyclopenta[d]pyrimidine (26.84 g, 113 mmol) prepared in Preparation Example 11, and 3-chloroperbenzoic acid (61.5 g, 250 mmol) were dissolved in 500 ml of dichloromethane, followed by stirring at room temperature for 18 hours. To the reaction solution after finishing the reaction, sodium thiosulfate (3.6 g, 22.68 mmol) was added, and water and a saturated sodium carbonate solution were added in sequence, followed by stirring. An organic layer extracted with dichloromethane was dried over anhydrous magnesium sulfate and filtered. The filtrate was concentrated under a reduced pressure to obtain 4-chloro-7,7-difluoro-2-(methylsulfonyl)-6,7-dihydro-5H-cyclopenta[d]pyrimidine of Formula 1 (30.5 g, yield >99%), and this product was used in the next reaction without separate purification. The synthesis of the 4-chloro-7,7-difluoro-2-(methylsulfonyl)-6,7-dihydro-5H-cyclopenta[d]pyrimidine thus obtained was confirmed through ¹H NMR analysis.

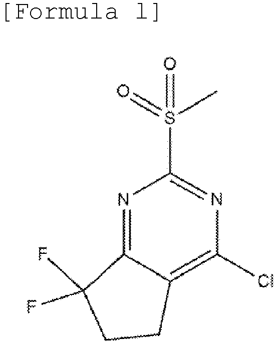

¹H NMR (400MHz, CDCl₃) δ 2.80 (m, 2H), 2.65 (s, 3H), 2.49-2.60 (m, 2H).

### Preparation Example 13

Tert-butyl piperazin-1-carboxylate (6.00 g, 32.21 mmol) and diisopropylethylamine (8.32 g, 64.37 mmol) were dissolved in 200 ml of dichloromethane, and the temperature was reduced to -78°C. Bromoacetyl bromide (7.15 g, 35.43 mmol) was slowly added thereto, followed by stirring for 1 hour, while slowly raising the temperature to room temperature. After finishing the reaction, the reaction solution was concentrated under a reduced pressure. A 1 N aqueous hydrochloric acid solution was added thereto, and the resultant was extracted with ethyl acetate. An organic layer was dried over anhydrous magnesium sulfate and filtered. The filtrate was concentrated under a reduced pressure and purified by column chromatography (ethyl acetate/hexane = 1/1 by a weight ratio) to obtain tert-butyl 4(2-bromoacetyl)piperazin-1-carboxylate of Formula m (6.62 g, 21.5 mmol, yield 66%). The synthesis of the tert-butyl 4(2-bromoacetyl)piperazin-1-carboxylate thus obtained was confirmed through ¹H NMR analysis.

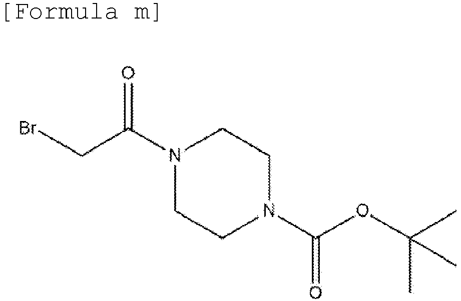

¹H NMR (400MHz, CDCl₃) δ 3.87 (s, 2H), 3.77-3.55 (m, 2H), 3.51 (dd, J=16.2, 5.8 Hz, 4H), 3.47-3.32 (m, 2H), 1.49 (d, J=18.3 Hz, 9H).

### Preparation Example 14

Tert-butyl 1,4-diazepane-1-carboxylate (3.00 g, 14.97 mmol) and diisopropylethylamine (3.86 g, 29.94 mmol) were dissolved in 100 ml of dichloromethane, and the temperature was reduced to -78°C. Bromoacetyl bromide (3.32 g, 16.47 mmol) was slowly added thereto, followed by stirring for 1 hour, while raising the temperature to room temperature. After finishing the reaction, the reaction solution was concentrated under a reduced pressure. A 1 N aqueous hydrochloric acid solution was added thereto, and the resultant was extracted with ethyl acetate. An organic layer was dried over anhydrous magnesium sulfate and filtered. The filtrate was concentrated under a reduced pressure and purified by column chromatography (ethyl acetate/hexane = 1/1 by a weight ratio) to obtain tert-butyl 4-(2-bromoacetyl)-1,4-diazepane-1-carboxylate of Formula n (2.21 g, 6.88 mmol, yield 45%). The synthesis of the tert-butyl 4-(2-bromoacetyl)-1,4-diazepane-1-carboxylate thus obtained was confirmed through 1H NMR analysis.

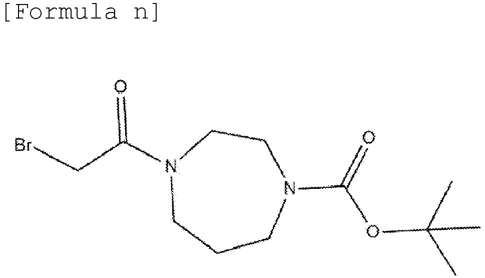

¹H NMR (400MHz, CDCl₃) δ 3.87 (d, J=8.8 Hz, 2H), 3.72-3.34 (m, 8H), 2.01-1.77 (m, 2H), 1.45 (d, J=8.8 Hz, 9H).

### Preparation Example 15

Glycolic acid (5.00 g, 65.74 mmol), N-[(dimethylamino)-1H-1,2,3-triazol-[4-5-b]pyridin-1-ylmethylene]-N-methylmethanaminium hexafluorophosphate N-oxide (37.47 g, 98.54 mmol), tert-butyl piperazin-1-carboxylate (12.24 g, 65.71 mmol) and triethylamine (13.29 g, 131.33 mmol) were dissolved in 200 ml of dimethylformamide, followed by stirring at room temperature for 18 hours. After finishing the reaction, the reaction solution was concentrated under a reduced pressure. Water was added thereto, and extraction with ethyl acetate was performed. An organic layer was dried over anhydrous magnesium sulfate and filtered. The filtrate was concentrated under a reduced pressure and purified by column chromatography (ethyl acetate/hexane = 1/1 by a weight ratio) to obtain tert-butyl 4-(2-hydroxyacetyl)piperazin-1-carboxylate of Formula o (4.81 g, 19.68 mmol, yield 29%). The tert-butyl 4-(2-hydroxyacetyl)piperazin-1-carboxylate thus obtained was confirmed through ¹H NMR analysis.

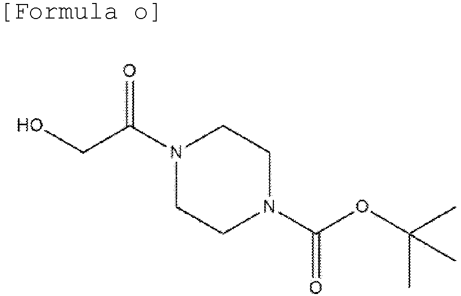

¹H NMR (400MHz, CDCl₃) δ 4.19 (s, 2H), 3.66 (br, s, 2H), 3.59 (s, 1H), 3.48 (br, s, 4H), 2.27 (br s, 2H), 1.49 (s, 9H) .

### Preparation Example 16

By performing Steps A and B in sequence, tert-butyl 4-(2-(5-(4,4,5,5-tetramethyl-1,3,2-dioxaborolan-2-yl)pyridin-2-yl)acetyl)piperazin-1-carboxylate of Formula p was prepared.

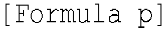

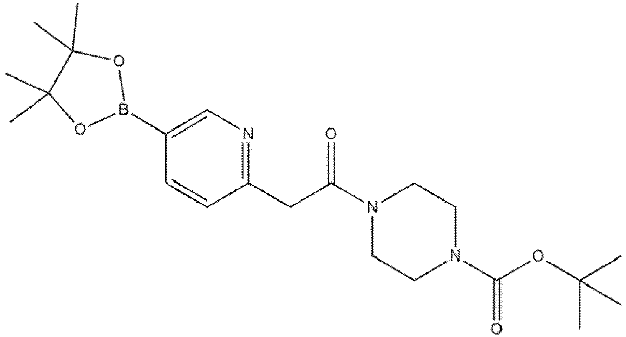

### (1) Step A: Preparation of tert-butyl 4-(2-(5-bromopyridin-2-yl)acetyl)piperazin-1-carboxylate

2-(5-Bromopyridin-2-yl)acetic acid (1 g, 4.63 mmol), tert-butyl-piperazin-1-carboxylate (862 mg, 4.63 mmol), and 1-[bis(dimethylamino)methylene]-1H-1,2,3-triazolo[4,5-b]pyridinium 3-oxide hexafluorophosphate (1.2 g, 3.16 mmol) were dissolved in 20 ml of dimethylformamide, and diisopropylethylamine (2.7 ml, 15.47 mmol) was added thereto, followed by stirring at room temperature for 18 hours. After finishing the reaction, the reaction solution was concentrated under a reduced pressure. An organic layer obtained by adding water and extracting with ethyl acetate was dried over anhydrous magnesium sulfate and filtered. The filtrate was concentrated under a reduced pressure and purified by column chromatography (ethyl acetate/hexane = 1/1 by a weight ratio) to obtain tert-butyl 4-(2-(5-bromopyridin-2-yl)acetyl)piperazin-1-carboxylate (884.8 mg, 2.303 mmol, yield 50%). The synthesis of the tert-butyl 4-(2-(5-bromopyridin-2-yl)acetyl)piperazin-1-carboxylate thus obtained was confirmed through ¹H NMR analysis.

¹H NMR (400MHz, CDCl₃) δ 8.57 (d, J=2.3 Hz, 1H), 7.76 (dd, J=8.2, 2.3 Hz, 1H), 7.25 (d, J=8.0 Hz, 1H), 3.87 (s, 2H), 3.57 (dd, J=10.7, 7.1 Hz, 4H), 3.35 (dt, J=13.1, 5.1 Hz, 4H), 1.44 (d, J=7.8 Hz, 9H).

### (2) Step B: Preparation of tert-butyl 4-(2-(5-(4,4,5,5-tetramethyl-1,3,2-dioxaborolan-2-yl)pyridin-2-yl)acetyl)piperazin-1-carboxylate

The tert-butyl 4-(2-(5-bromopyridin-2-yl)acetyl)piperazin-1-carboxylate (1.18 g, 3.07 mmol) prepared in Step A, 4,4,4',4'5,5,5',5'-octamethyl-2,2-bi(1,3,2-dioxaborolane) (1.17 g, 4.61 mmol), and potassium acetate (904 mg, 9.21 mmol) were dissolved in 30 ml of 1,4-dioxane, and a [1,1'-bis(diphenylphosphino)ferrocene]dichloropalladium dichloromethane complex (251 mg, 0.307 mmol) was added thereto, followed by stirring at 100°C for 18 hours. After finishing the reaction, the reaction solution was cooled to room temperature and extracted with ethyl acetate. An organic layer was dried over anhydrous magnesium sulfate and filtered. The filtrate was concentrated under a reduced pressure to obtain tert-butyl 4-(2-(5-(4,4,5,5-tetramethyl-1,3,2-dioxaborolan-2-yl)pyridin-2-yl)acetyl)piperazin-1-carboxylate (1.74 g, 4.03 mmol, crude). The synthesis of the tert-butyl 4-(2-(5-(4,4,5,5-tetramethyl-1,3,2-dioxaborolan-2-yl)pyridin-2-yl)acetyl)piperazin-1-carboxylate thus obtained was confirmed through ¹H NMR analysis.

¹H NMR (400MHz, CDCl₃) δ 8.33 (s, 1H), 8.01 (dd, J=7.5, 1.6 Hz, 1H), 7.33 (d, J=7.8 Hz, 1H). 3.94 (s, 2H), 3.53-3.59 (m, 4H), 3.34 (t, J=5.0 Hz, 2H), 3.24 (t, J=5.3 Hz, 2H), 1.43 (s, 9H), 1.34 (s, 12H).

### Preparation Example 17

Step A and B were performed in sequence to prepare tert-butyl 4-(2-((5-(4,4,5,5-tetramethyl-1,3,2-dioxaborolan-2-yl)pyridin-2-yl)oxy)acetyl)piperazin-1-carboxylate of Formula q.

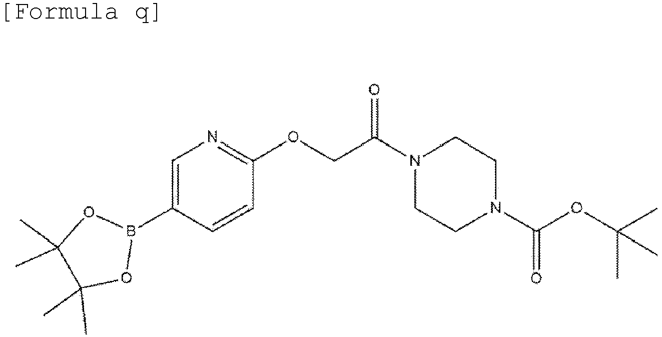

### (1) Step A: Preparation of tert-butyl 4-(2-((5-bromopyridin-2-yl)oxy)acetyl)piperazin-1-carboxylate

Tert-butyl 4(2-hydroxyacetyl)piperazin-1-carboxylate (2,3 g, 9.35 mmol) and sodium hydride (60%, 374 mg, 9.35 mmol) were dissolved in 30 ml of dimethylformamide, followed by stirring at 0°C for 30 minutes. Then, 5-bromo-2-chloropyridine (1.5 g, 7.79 mmol) was added thereto, followed by stirring at 50°C for 2 hours. After finishing the reaction, the reaction solution was concentrated under a reduced pressure. An organic layer obtained by adding a 1 N hydrochloric acid solution and extracting with ethyl acetate was dried over anhydrous magnesium sulfate and filtered. The filtrate was concentrated under a reduced pressure and purified by column chromatography (ethyl acetate/hexane = 1/1 by a weight ratio) to obtain tert-butyl 4(2-((5-bromopyridin-2-yl)oxy)acetyl)piperazin-1-carboxylate (1.58 g, 3.95 mmol, yield 51%). The synthesis of the tert-butyl 4(2-((5-bromopyridin-2-yl)oxy)acetyl)piperazin-1-carboxylate thus obtained was confirmed through ¹H NMR analysis.

¹H NMR (400MHz, CDCl₃) δ 8.15 (s, 1H), 7.70 (d, 1H), 6.83 (d, 1H), 5.00 (s, 2H), 3.62 (br, s, 2H), 3.50 (br s, 4H), 3.45 (br s, 2H), 1.49 (s, 9H).

### (2) Step B: Preparation of tert-butyl 4-(2-((5-(4,4,5,5-tetramethyl-1,3,2-dioxaborolan-2-yl)pyridin-2-yl)oxy)acetyl)piperazin-1-carboxylate

The tert-butyl 4(2-((5-bromopyridin-2-yl)oxy)acetyl)piperazin-1-carboxylate (6 g, 14.99 mmol) prepared in Step A, bis(pinacolato)diboron (4.19 g, 16.49 mmol), and potassium acetate (3.68 g, 37.5 mmol) were dissolved in 20 ml of 1,4-dioxane, followed by degassing for 5 minutes. A 1,1'-bis(diphenylphosphino)ferrocene-palladium(II) dichloridedichloromethane complex (612 mg, 0.749 mmol) was added thereto, followed by stirring at 80°C for 18 hours. After finishing the reaction, ethyl acetate was added to the reaction solution, and the resultant was washed with water and a saturated aqueous sodium chloride solution, dried over anhydrous magnesium sulfate and filtered. The filtrate was concentrated under a reduced pressure to obtain tert-butyl 4-(2-((5-(4,4,5,5-tetramethyl-1,3,2-dioxaborolan-2-yl)pyridin-2-yl)oxy)acetyl)piperazin-1-carboxylate (6.71 g, 23.96 mmol, crude). The synthesis of the tert-butyl 4-(2-((5-(4,4,5,5-tetramethyl-1,3,2-dioxaborolan-2-yl)pyridin-2-yl)oxy)acetyl)piperazin-1-carboxylate was confirmed by mass spectrometry (MS) measurement.
MS 448.3, 470.2(Na⁺)

### Preparation Example 18

Step A and B were performed in sequence to prepare tert-butyl 4-(2-((3-chloro-5-(4,4,5,5-tetramethyl-1,3,2-dioxaborolan-2-yl)pyridin-2-yl)oxy)acetyl)piperazin-1-carboxylate of Formula r.

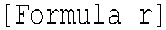

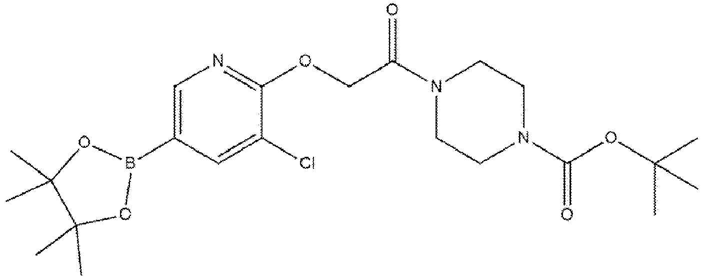

### (1) Step A: Preparation of tert-butyl 4-(2-((5-bromo-3-chloropyridin-2-yl)oxy)acetyl)piperazin-1-carboxylate

Tert-butyl 4(2-hydroxyacetyl)piperazin-1-carboxylate (2.5 g, 10.1 mmol) and sodium hydride (60%, 402 mg, 10.1 mmol) were dissolved in 30 ml of dimethylformamide, followed by stirring at 0°C for 30 minutes. 5-Bromo-2,3-dichloropyridine (1.5 g, 7.79 mmol) was added thereto, followed by stirring at 50°C for 2 hours. After finishing the reaction, the reaction solution was concentrated under a reduced pressure. An organic layer obtained by adding a 1 N hydrochloric acid solution and extracting with ethyl acetate was dried over anhydrous magnesium sulfate and filtered. The filtrate was concentrated under a reduced pressure and purified by column chromatography (ethyl acetate/hexane = 1/1 by a weight ratio) to obtain tert-butyl 4-(2-((5-bromo-3-chloropyridin-2-yl)oxy)acetyl)piperazin-1-carboxylate (2.1 g, 4.83 mmol, yield 58%). The synthesis of the tert-butyl 4-(2-((5-bromo-3-chloropyridin-2-yl)oxy)acetyl)piperazin-1-carboxylate thus obtained was confirmed through ¹H NMR analysis.

¹H NMR (400MHz, CDCl₃) δ 8.05 (s, 1H), 7.80 (d, J=5.5 Hz, 1H), 5.08 (d, J=11.6 Hz, 2H), 3.60 (s, 2H), 3.47 (d, J=30.5 Hz, 6H), 1.48 (s, 9H).

### (2) Step B: Preparation of tert-butyl 4-(2-((3-chloro-5-(4,4,5,5-tetramethyl-1,3,2-dioxaborolan-2-yl)pyridin-2-yl)oxy)acetyl)piperazin-1-carboxylate

The tert-butyl 4-(2-((5-bromo-3-chloropyridin-2-yl)oxy)acetyl)piperazin-1-carboxylate (700 mg, 1.61 mmol) prepared in Step A, bis(pinacolato)diboron (450 mg, 1.77 mmol), and potassium acetate (395 mg, 4.03 mmol) were dissolved in 20 ml of 1,4-dioxane, followed by degassing for 5 minutes. A 1,1'-bis(diphenylphosphino)ferrocene-palladium(II) dichloridedichloromethane complex (66 mg, 0.08 mmol) was added thereto, followed by stirring at 80°C for 18 hours. After finishing the reaction, ethyl acetate was added to the reaction solution, and the resultant was washed with water and a saturated aqueous sodium chloride solution, dried over anhydrous magnesium sulfate and filtered. The filtrate was concentrated under a reduced pressure to obtain tert-butyl 4-(2-((3-chloro-5-(4,4,5,5-tetramethyl-1,3,2-dioxaborolan-2-yl)pyridin-2-yl)oxy)acetyl)piperazin-1-carboxylate. The synthesis of the tert-butyl 4-(2-((3-chloro-5-(4,4,5,5-tetramethyl-1,3,2-dioxaborolan-2-yl)pyridin-2-yl)oxy)acetyl)piperazin-1-carboxylate was confirmed through mass spectrometry (MS) measurement.

### Example 29: Preparation of ((S)-2-(4-(7,7-difluoro-2-(2-methylazetidin-1-yl)-6,7-dihydro-5H-cyclopenta[d]pyrimidin-4-yl)phenyl)acetic acid

As in Reaction 23, the processes of Steps A to D were performed in sequence to prepare the title compound of Formula 1-29.

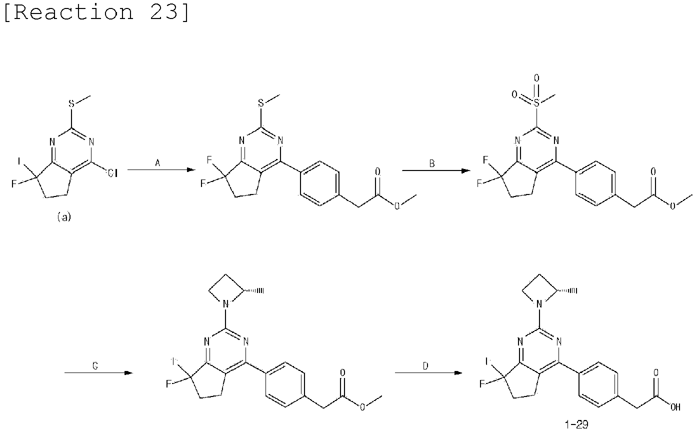

### (1) Step A: Preparation of methyl 2-(4-(7,7-difluoro-2-(methylthio)-6,7-dihydro-5H-cyclopenta[d]pyrimidin-4-yl)phenyl)acetate

The 4-chloro-7,7-difluoro-2-(methylthio)-6,7-dihydro-cyclopenta[d]pyrimidine of Formula (a) (200 mg, 0.845 mmol) prepared in Preparation Example 11, methyl 2-(4-(4,4,5,5-tetramethyl-1,3,2-dioxaborolan-2-yl)phenyl)acetate (350 mg, 1.268 mmol), tetrakis(triphenylphosphine)palladium(O) (49 mg, 0.04 mmol) and a sodium bicarbonate solution (2 M, 27 ml, 2.54 mmol) were dissolved in 20 ml of 1,4-dioxane, followed by degassing for 5 minutes. Then, the resultant was stirred at 80°C for 2 hours. After finishing the reaction, the reaction solution was washed with water, ethyl acetate was added thereto, and the resultant was washed with water and a saturated aqueous sodium chloride solution, dried over anhydrous magnesium sulfate and filtered. The filtrate was concentrated under a reduced pressure and purified by column chromatography (ethyl acetate/hexane = 1/3 by a weight ratio) to obtain methyl 2-(4-(7,7-difluoro-2-(methylthio)-6,7-dihydro-5H-cyclopenta[d]pyrimidin-4-yl)phenyl)acetate (200 mg, 0.57 mmol, yield 98%). The synthesis of the methyl 2-(4-(7,7-difluoro-2-(methylthio)-6,7-dihydro-5H-cyclopenta[d]pyrimidin-4-yl)phenyl)acetate thus obtained was confirmed through ¹H NMR analysis.

¹H NMR (500MHz, CDCl₃) δ 7.93 (d, 2H), 7.44 (d, 2H), 3.71 (s, 3H), 3.70 (s, 2H), 3.21 (m, 2H), 2.64 (s, 3H), 2.62 (m, 2H).

### (2) Step B: Preparation of methyl 2-(4-(7,7-difluoro-2-(methylsulfonyl)-6,7-dihydro-5H-cyclopenta[d]pyrimidin-4-yl)phenyl)acetate

The methyl 2-(4-(7,7-difluoro-2-(methylthio)-6,7-dihydro-5H-cyclopenta[d]pyrimidin-4-yl)phenyl)acetate (200 mg, 0.57 mmol) prepared in Step A and 3-chloroperbenzoic acid (70%, 352 mg, 1.43 mmol) were dissolved in 20 ml of dichloromethane, followed by stirring at room temperature for 10 hours. After finishing the reaction, a saturated sodium thiosulfate solution and a saturated sodium bicarbonate solution were added to the reaction solution in sequence, and the resultant was extracted with ethyl acetate. An organic layer was washed with a saturated aqueous sodium chloride solution, dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain methyl 2-(4-(7,7-difluoro-2-(methylsulfonyl)-6,7-dihydro-5H-cyclopenta[d]pyrimidin-4-yl)phenyl)acetate (150 mg, 0.39 mmol, yield 68%). The synthesis of the methyl 2-(4-(7,7-difluoro-2-(methylsulfonyl)-6,7-dihydro-5H-cyclopenta[d]pyrimidin-4-yl)phenyl)acetate thus obtained was confirmed through ¹H NMR analysis.

¹H NMR (500MHz, CDCl₃) δ 8.03 (d, 2H), 7.49 (d, 2H), 3.73 (s, 3H), 3.72 (s, 2H), 3.45 (s, 3H), 3.38 (m, 2H), 2.74 (m, 2H).

### (3) Step C: Preparation of methyl (S)-2-(4-(7,7-difluoro-2-(2-methylazetidin-1-yl)-6,7-dihydro-5H-cyclopenta[d]pyrimidin-4-yl)phenyl)acetate

The methyl 2-(4-(7,7-difluoro-2-(methylsulfonyl)-6,7-dihydro-5H-cyclopenta[d]pyrimidin-4-yl)phenyl)acetate (130 mg, 0.34 mmol) prepared in Step B, (2S)-2-methylazetidine[(1R,4S)-7,7-dimethyl-2-oxo-norbornan-1-yl]methanesulfonate (239 mg, 0.51 mmol) and potassium carbonate (141 mg, 1.02 mmol) were dissolved in 20 ml of acetonitrile, followed by stirring at 80°C for 2 hours. After finishing the reaction, water was added to the reaction solution, and the resultant was extracted with ethyl acetate. An organic layer was washed with a saturated aqueous sodium chloride solution, dried over anhydrous magnesium sulfate and filtered. The filtrate was concentrated under a reduced pressure, and the concentrate under a reduced pressure was purified by column chromatography(ethyl acetate/hexane = 1/1 by a weight ratio) to obtain methyl (S)-2-(4-(7,7-difluoro-2-(2-methylazetidin-1-yl)-6,7-dihydro-5H-cyclopenta[d]pyrimidin-4-yl)phenyl)acetate (110 mg, 0.30 mmol, yield 88%). The synthesis of the methyl (S)-2-(4-(7,7-difluoro-2-(2-methylazetidin-1-yl)-6,7-dihydro-5H-cyclopenta[d]pyrimidin-4-yl)phenyl)acetate thus obtained was confirmed through ¹H NMR analysis.

¹H NMR (500MHz, CDCl₃) δ 7.87 (d, 2H), 7.39 (d, 2H), 4.59 (m, 1H), 4.15 (m, 1H), 4.08 (m, 1H), 3.70 (s, 3H), 3.68 (s, 2H), 3.08 (m, 2H), 2.53 (m, 2H), 2.47 (m, 1H), 2.01 (m, 1H), 1.59 (d, 3H).

### (4) Step D: Preparation of (S)-2-(4-(7,7-difluoro-2-(2-methylazetidin-1-yl)-6,7-dihydro-5H-cyclopenta[d]pyrimidin-4-yl)phenyl)acetic acid

The methyl (S)-2-(4-(7,7-difluoro-2-(2-methylazetidin-1-yl)-6,7-dihydro-5H-cyclopenta[d]pyrimidin-4-yl)phenyl)acetate (110 mg, 0.30 mmol) prepared in Step C and 1 ml of a 1 N aqueous sodium hydroxide solution were dissolved in 3 ml of tetrahydrofuran, followed by stirring at room temperature for 10 hours. After finishing the reaction, a 1 N hydrochloric acid solution was added to the reaction solution, and the resultant was extracted with ethyl acetate. An organic layer was washed with a saturated aqueous sodium chloride solution, dried over anhydrous magnesium sulfate and filtered. The filtrate was concentrated under a reduced pressure, and the concentrate under a reduced pressure was purified by column chromatography(methanol/dichloromethane = 1/9 by a weight ratio) to obtain (S)-2-(4-(7,7-difluoro-2-(2-methylazetidin-1-yl)-6,7-dihydro-5H-cyclopenta[d]pyrimidin-4-yl)phenyl) acetic acid (38 mg, 0.11 mmol, yield 38%) . The synthesis of the (S)-2-(4-(7,7-difluoro-2-(2-methylazetidin-1-yl)-6,7-dihydro-5H-cyclopenta[d]pyrimidin-4-yl)phenyl)acetic acid thus obtained was confirmed through ¹H NMR analysis.

¹H NMR (500MHz, CDCl₃) δ 7.87 (d, 2H), 7.40 (d, 2H), 4.59 (m, 1H), 4.16 (m, 1H), 4.06 (m, 1H), 3.70 (s, 3H), 3.07 (s, 2H), 2.55 (m, 2H), 2.47 (m, 1H), 2.01 (m, 1H), 1.58 (d, 3H) .

### Example 30: Preparation of (S)-2-(4-(7,7-difluoro-2-(2-methylazetidin-1-yl)-6,7-dihydro-5H-cyclopenta[d]pyrimidin-4-yl)phenyl)-1-(piperazin-1-yl)ethan-1-one

As in Reaction 24, the processes of Steps A and B were performed in sequence to prepare the title compound of Formula 1-30.

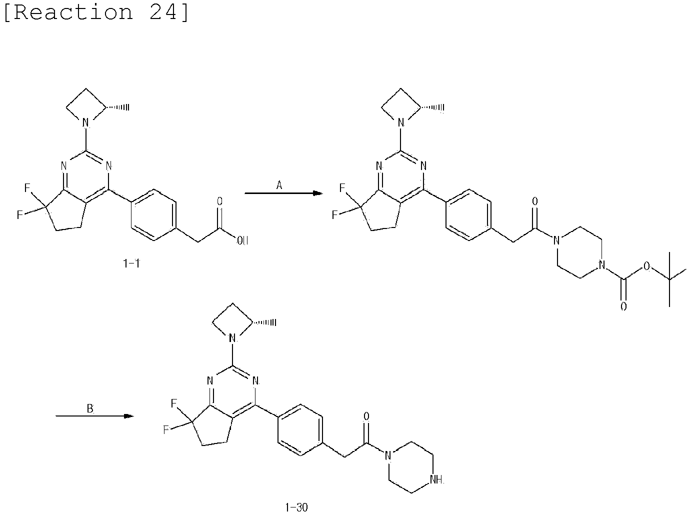

### (1) Step A: Preparation of tert-butyl (S)-4-(2-(4-(7,7-difluoro-2-(2-methylazetidin-1-yl)-6,7-dihydro-5H-cyclopenta[d]pyrimidin-4-yl)phenyl)acetyl)piperazin-1-carboxylate

The (S)-2-(4-(7,7-difluoro-2-(2-methylazetidin-1-yl)-6,7-dihydro-5H-cyclopenta[d]pyrimidin-4-yl)phenyl)acetic acid of Formula 1-29 (38 mg, 0.11 mmol) prepared in Example 29, 2,4,6-tripropyl-1,3,5,2,4,6-trioxatriphosphinane-2,4,6-trioxide (40.4 mg, 0.13 mmol), tert-butyl piperazin-1-carboxylate (24 mg, 0.13 mmol) and triethylamine (0.04 ml, 0.32 mmol) were dissolved in 5 ml of dichloromethane, followed by stirring at room temperature for 2 hours. After finishing the reaction, a 1 N hydrochloric acid solution was added, and extraction with ethyl acetate was performed. An organic layer was dried over anhydrous magnesium sulfate and filtered. The filtrate was concentrated under a reduced pressure and purified by column chromatography (dichloromethane/methanol=9/1 by a weight ratio) to obtain tert-butyl (S)-4-(2-(4-(7,7-difluoro-2-(2-methylazetidin-1-yl)-6,7-dihydro-5H-cyclopenta[d]pyrimidin-4-yl)phenyl)acetyl)piperazin-1-carboxylate (55 mg, 0.10 mmol, yield 99%). The tert-butyl (S)-4-(2-(4-(7,7-difluoro-2-(2-methylazetidin-1-yl)-6,7-dihydro-5H-cyclopenta[d]pyrimidin-4-yl)phenyl)acetyl)piperazin-1-carboxylate thus obtained was confirmed through ¹H NMR analysis.

¹H NMR (500MHz, CDCl₃) δ 7.88 (d, 2H), 7.34 (d, 2H), 4.57 (m, 1H), 4.10 (m, 1H), 3.78 (s, 2H), 3.60 (s, 2H), 3.40 (m, 4H), 3.20 (m, 2H), 3.07 (m, 2H), 2.52 (m, 2H), 2.49 (m, 1H), 2.00 (m, 1H), 1.57 (d, 23H), 1.43 (s, 9H).

### (2) Step B: Preparation of (S)-2-(4-(7,7-difluoro-2-(2-methylazetidin-1-yl)-6,7-dihydro-5H-cyclopenta[d]pyrimidin-4-yl)phenyl)-1-(piperazin-1-yl)ethan-1-one

The tert-butyl (S)-4-(2-(4-(7,7-difluoro-2-(2-methylazetidin-1-yl)-6,7-dihydro-5H-cyclopenta[d]pyrimidin-4-yl)phenyl)acetyl) piperazin-1-carboxylate (55 mg, 0.10 mmol) prepared in Step A and 1 ml of trifluoroacetic acid were dissolved in 4 ml of dichloromethane, followed by stirring at room temperature for 8 hours. After finishing the reaction, the resultant was concentrated under a reduced pressure. To the concentrate, a saturated aqueous ammonium hydroxide solution was added, and the resultant was extracted with ethyl acetate. An organic layer was dried over anhydrous magnesium sulfate and filtered. The filtrate was concentrated under a reduced pressure, and the concentrate under a reduced pressure was purified by column chromatography (dichloromethane/methanol=9/1 by a weight ratio) to obtain (S)-2-(4-(7,7-difluoro-2-(2-methylazetidin-1-yl)-6,7-dihydro-5H-cyclopenta[d]pyrimidin-4-yl)phenyl)-1-(piperazin-1-yl)ethan-1-one (32 mg, 0.075 mmol, yield 75%). The synthesis of the (S)-2-(4-(7,7-difluoro-2-(2-methylazetidin-1-yl)-6,7-dihydro-5H-cyclopenta[d]pyrimidin-4-yl)phenyl)-1-(piperazin-1-yl)ethan-1-one thus obtained was confirmed through ¹H NMR analysis.

¹H NMR (500MHz, CDCl₃) δ 7.87 (d, 2H), 7.34 (d, 2H), 4.59 (m, 1H), 4.15 (m, 1H), 4.06 (m, 1H), 3.76 (s, 2H), 3.61 (m, 2H), 3.43 (m, 2H), 3.08 (m, 2H), 2.83 (m, 2H), 2.72 (m, 2H), 3.60 (m, 2H), 3.50 (m, 1H), 2.00 (m, 1H), 1.57 (d, 3H).

### Example 31: Preparation of ((S)-2-(5-(2-(2-methylazetidin-1-yl)-6-(trifluoromethyl)pyrimidin-4-yl)pyridin-2-yl)-1-(piperazin-1-yl)ethan-1-one

As in Reaction 25, the processes of Steps A to C were performed in sequence to prepare the title compound of Formula 1-31.

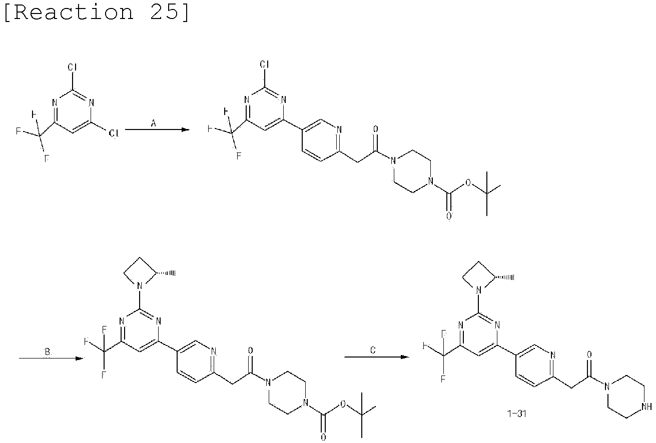

### (1) Step A: Preparation of tert-butyl 4-(2-(5-(2-chloro-6-(trifluoromethyl)pyrimidin-4-yl)pyridin-2-yl)acetyl)piperazin-1-carboxylate

The 2,4-dichloro-6-(trifluoromethyl)pyrimidine (98 mg, 0.450 mmol), the tert-butyl 4-(2-(5-(4,4,5,5-tetramethyl-1,3,2-dioxaborolan-2-yl)pyridin-2-yl)acetyl)piperazin-1-carboxylate of Formula p (388 mg, 0.900 mmol) prepared in Preparation Example 16, a sodium carbonate solution (2 M, 0.675 ml, 1.349 mmol) and tetrakis(triphenylphosphine)palladium (52 mg, 0.045 mmol) were dissolved in 10 ml of 1,4-dioxane, followed by stirring at 80°C for 18 hours. After finishing the reaction, water was added, and the resultant was extracted with ethyl acetate. An organic layer was dried over anhydrous magnesium sulfate and filtered. The filtrate was concentrated under a reduced pressure and purified by column chromatography to obtain tert-butyl 4-(2-(5-(2-chloro-6-(trifluoromethyl)pyrimidin-4-yl)pyridin-2-yl)acetyl)piperazin-1-carboxylate (140 mg, yield 64%). The synthesis of the tert-butyl 4-(2-(5-(2-chloro-6-(trifluoromethyl)pyrimidin-4-yl)pyridin-2-yl)acetyl)piperazin-1-carboxylate thus obtained was confirmed through ¹H NMR analysis.

¹H NMR (400MHz, CDCl₃) δ 9.23 (d, J=1.8 Hz, 1H), 8.42 (dd, J=8.2, 2.3 Hz, 1H), 7.95 (s, 1H), 4.03 (s, 2H), 3.61 (t, J=5.0 Hz, 4H), 3.35-3.40 (m, 4H), 1.45 (s, 9H).

### (2) Step B: Preparation of tert-butyl (S)-4-(2-(5-(2-methylazetidin-1-yl)-6-(trifluoromethyl)pyrimidin-4-yl)pyridin-2-yl)acetyl)piperazin-1-carboxylate

The tert-butyl 4-(2-(5-(2-chloro-6-(trifluoromethyl)pyrimidin-4-yl)pyridin-2-yl)acetyl)piperazin-1-carboxylate (140 mg, 0.29 mmol) prepared in Step A, (2S)-2-methylazetidine[(1R,4S)-7,7-dimethyl-2-oxo-norbornan-1-yl]methanesulfonate (105 mg, 0.35 mmol) and potassium carbonate (120 mg, 0.87 mmol) were dissolved in 20 ml of acetonitrile, followed by stirring at 80°C for 3 hours. After finishing the reaction, water was added, and the resultant was extracted with ethyl acetate. An organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl (S)-4-(2-(5-(2-methylazetidin-1-yl)-5-(trifluoromethyl)pyrimidin-4-yl)pyridin-2-yl)acetyl)piperazin-1-carboxylate (135 mg, yield 90%) . The synthesis of the tert-butyl (S)-4-(2-(5-(2-methylazetidin-1-yl)-6-(trifluoromethyl)pyrimidin-4-yl)pyridin-2-yl)acetyl)piperazin-1-carboxylate thus obtained was confirmed through ¹H NMR analysis.

¹H NMR (400MHz, CDCl₃) δ 9.17 (d, J=2.3 Hz, 1H), 8.27 (dd, J=8.2, 2.3 Hz, 1H), 7.53 (dd, J=7.5, 1.6 Hz, 1H), 7.16 (s, 1H), 4.61 (dd, J=14.4, 6.2 Hz, 1H), 4.06-4.20 (m, 2H), 3.99 (s, 2H), 3.60 (t, J=5.3 Hz, 4H), 3.37 (dd, J=13.3, 5.5 Hz, 4H), 2.47-2.55 (m, 1H), 2.00-2.08 (m, 1H), 1.58 (d, J=6.4 Hz, 3H), 1.44 (s, 9H).

### (3) Step C: Preparation of (S)-2-(5-(2-(2-methylazetidin-1-yl)-6-(trifluoromethyl)pyrimidin-4-yl)pyridin-2-yl)-1-(piperazin-1-yl)ethan-1-one

The tert-butyl (S)-4-(2-(5-(2-methylazetidin-1-yl)-6-(trifluoromethyl)pyrimidin-4-yl)pyridin-2-yl)acetyl)piperazin-1-carboxylate (135 mg, 0.259 mmol) prepared in Step B and 1 ml of trifluoroacetic acid were dissolved in 4 ml of dichloromethane, followed by stirring at room temperature for 4 hours. After finishing the reaction, the resultant was concentrated under a reduced pressure. The concentrate under a reduced pressure was neutralized using a saturated aqueous sodium bicarbonate solution, and then, extracted with dichloromethane. An organic layer was dried over anhydrous magnesium sulfate and filtered. The filtrate was concentrated under a reduced pressure, and the concentrate under a reduced pressure was purified by column chromatography (methanol/dichloromethane = 1/9 by a weight ratio) to obtain (S)-2-(5-(2-(2-methylazetidin-1-yl)-6-(trifluoromethyl)pyrimidin-4-yl)pyridin-2-yl)-1-(piperazin-1-yl)ethan-1-one (78 mg, yield 72%). The synthesis of the (S)-2-(5-(2-(2-methylazetidin-1-yl)-6-(trifluoromethyl)pyrimidin-4-yl)pyridin-2-yl)-1-(piperazin-1-yl)ethan-1-one thus obtained was confirmed through ¹H NMR analysis.

¹H NMR (400MHz, CDCl₃) δ 9.16 (d, J=1.8 Hz, 1H), 8.27 (dd, J=8.2, 2.3 Hz, 1H), 7.46 (d, J=8.2 Hz, 1H), 7.16 (s, 1H), 4.60 (dd, J=14.0, 6.2 Hz, 1H), 4.06-4.19 (m, 2H), 3.98 (s, 2H), 3.59 (td, J=9.6, 4.7 Hz, 4H), 2.79 (dt, J=22.0, 5.0 Hz, 4H), 2.46-2.55 (m, 1H), 1.99-2.08 (m, 1H), 1.58 (d, J=6.4 Hz, 3H) .

### Example 32: Preparation of (S)-2-(4-(7,7-difluoro-2-(2-methylazetidin-1-yl)-6,7-dihydro-5H-cyclopenta[d]pyrimidin-4-yl)phenoxy)-1-(piperazin-1-yl)ethan-1-one)

As in Reaction 26, the processes of Steps A to E were performed in sequence to prepare the title compound of Formula 1-32.

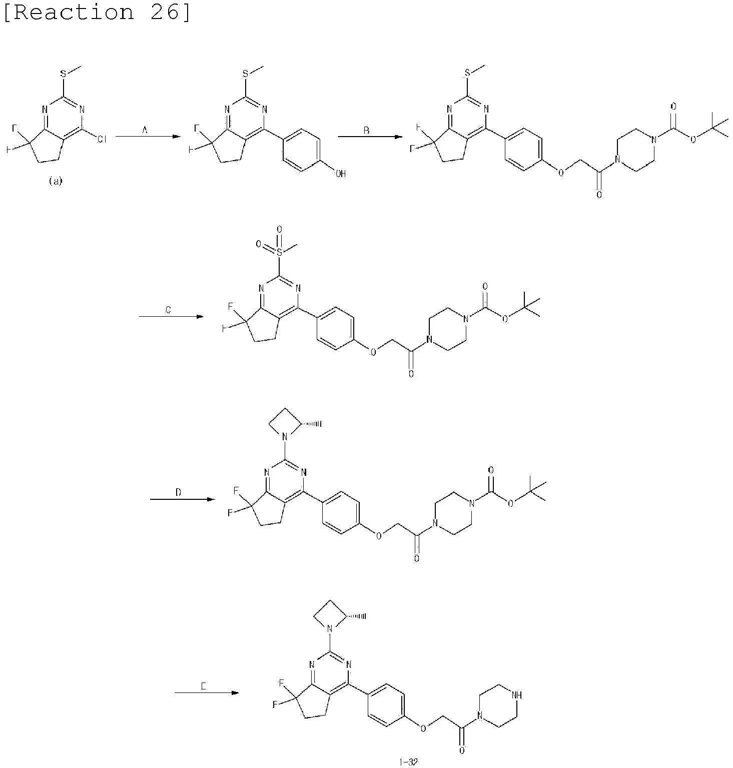

### (1) Step A: Preparation of 4-(7,7-difluoro-2-(methylthio)-6,7-dihydro-5H-cyclopenta[d]pyrimidin-4-yl)phenol

The 4-chloro-7,7-difluoro-2-(methylthio)-6,7-dihydro-cyclopenta[d]pyrimidine of Formula k (200 mg, 0.845 mmol) prepared in Preparation Example 11, (4-hydroxyphenyl)boronic acid (175 mg, 1.268 mmol), tetrakis(triphenylphosphine)palladium(O) (49 mg, 0.042 mmol) and a sodium carbonate solution (2 M, 1.27 ml, 2.54 mmol) were dissolved in 20 ml of 1,4-dioxane, followed by stirring at 80°C for 2 hours. After finishing the reaction, water was added to the reaction solution, and the resultant was extracted with ethyl acetate. An organic layer was dried over anhydrous magnesium sulfate and filtered. The filtrate was concentrated under a reduced pressure and purified by column chromatography (ethyl acetate/hexane = 1/3 by a weight ratio) to obtain 4-(7,7-difluoro-2-(methylthio)-6,7-dihydro-5H-cyclopenta[d]pyrimidin-4-yl)phenol (130 mg, 0.44 mmol, yield 52%). The synthesis of the 4-(7,7-difluoro-2-(methylthio)-6,7-dihydro-5H-cyclopenta[d]pyrimidin-4-yl)phenol thus obtained was confirmed through ¹H NMR analysis.

¹H NMR (500MHz, CDCl₃) δ 7.69 (d, 2H), 6.70 (d, 2H), 3.24 (m, m, 2H), 2.69 (s, 3H), 2.65 (m, 2H).

### (2) Step B: Preparation of tert-butyl 4-(2-(4-(7,7-difluoro-2-(methylthio)-6,7-dihydro-5H-cyclopenta[d]pyrimidin-4-yl)phenoxy)acetyl)piperazin-1-carboxylate

The 4-(7,7-difluoro-2-(methylthio)-6,7-dihydro-5H-cyclopenta[d]pyrimidin-4-yl)phenol (130 mg, 0.44 mmol) prepared in Step A, the tert-butyl 4-(2-bromoacetyl)piperazin-1-carboxylate of Formula c (203 mg, 0.66 mmol) prepared in Preparation Example 3 and potassium carbonate (183 mg, 1.33 mmol) were dissolved in 10 ml of dimethylformamide, followed by stirring at room temperature for 2 hours. After finishing the reaction, the reaction solution was concentrated under a reduced pressure. The resultant was dissolved in ethyl acetate and then, washed with a saturated sodium chloride solution. Then, an organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl 4-(2-(4-(7,7-difluoro-2-(methylthio)-6,7-dihydro-5H-cyclopenta[d]pyrimidin-4-yl)phenoxy)acetyl)piperazin-1-carboxylate (160 mg, 0.31 mmol, yield 70%). The synthesis of the tert-butyl 4-(2-(4-(7,7-difluoro-2-(methylthio)-6,7-dihydro-5H-cyclopenta[d]pyrimidin-4-yl)phenoxy)acetyl)piperazin-1-carboxylate thus obtained was confirmed through ¹H NMR analysis.

¹H NMR (400MHz, CDCl₃) δ 7.97 (dd, J=6.9, 2.3 Hz, 2H), 7.07 (dd, J=6.9, 1.8 Hz, 2H), 4.78 (s, 2H), 3.57 (q, J=5.6 Hz, 4H), 3.39-3.44 (m, 4H), 3.18-3.22 (m, 2H), 2.66 (s, 3H), 2.63 (m, 2H), 1.46 (s, 9H).

### (3) Step C: Preparation of tert-butyl 4-(2-(4-(7,7-difluoro-2-(methylsulfonyl)-6,7-dihydro-5H-cyclopenta[d]pyrimidin-4-yl)phenoxy)acetyl)piperazin-1-carboxylate

The tert-butyl 4-(2-(4-(7,7-difluoro-2-(methylthio)-6,7-dihydro-5H-cyclopenta[d]pyrimidin-4-yl)phenoxy)acetyl)piperazin-1-carboxylate (160 mg, 0.31 mmol) prepared in Step B and 3-chloroperbenzoic acid (70%, 189 mg, 0.77 mmol) were dissolved in 20 ml of dichloromethane, followed by stirring at room temperature for 10 hours. After finishing the reaction, a saturated sodium thiosulfate solution and a saturated sodium bicarbonate solution were added to the reaction solution in sequence, and the resultant was extracted with ethyl acetate. An organic layer was washed with an aqueous saturated sodium chloride solution, dried over anhydrous magnesium sulfate and filtered. The filtrate was concentrated under a reduced pressure, and the concentrate under a reduced pressure was purified by column chromatography (methanol/dichloromethane = 1/9 by a weight ratio) to obtain tert-butyl 4-(2-(4-(7,7-difluoro-2-(methylsulfonyl)-6,7-dihydro-5H-cyclopenta[d]pyrimidin-4-yl)phenoxy)acetyl)piperazin-1-carboxylate (141 mg, 0.26 mmol, yield 84%). The synthesis of the tert-butyl 4-(2-(4-(7,7-difluoro-2-(methylsulfonyl)-6,7-dihydro-5H-cyclopenta[d]pyrimidin-4-yl)phenoxy)acetyl)piperazin-1-carboxylate thus obtained was confirmed through mass spectrometry (MS) measurement.
MS 552.6, 574.6 (Na⁺)

### (4) Step D: Preparation of tert-butyl (S)-4-(2-(4-(7,7-difluoro-2-(2-methylazetidin-1-yl)-6,7-dihydro-5H-cyclopenta[d]pyrimidin-4-yl)phenoxy)acetyl)piperazin-1-carboxylate

The tert-butyl 4-(2-(4-(7,7-difluoro-2-(methylsulfonyl)-6,7-dihydro-5H-cyclopenta[d]pyrimidin-4-yl)phenoxy)acetyl)piperazin-1-carboxylate (141 mg, 0.26 mmol) prepared in Step C, (2S)-2-methylazetidine[(1R,4S)-7,7-dimethyl-2-oxo-norbornan-1-yl]methanesulfonate (173 mg, 0.37 mmol) and potassium carbonate (127 mg, 0.92 mmol) were dissolved in 20 ml of acetonitrile, followed by stirring at 80°C for 2 hours. After finishing the reaction, an excessive amount of water was added to the reaction solution, and the resultant was extracted with ethyl acetate. An organic layer was washed with a saturated aqueous sodium chloride solution, dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl (S)-4-(2-(4-(7,7-difluoro-2-(2-methylazetidin-1-yl)-6,7-dihydro-5H-cyclopenta[d]pyrimidin-4-yl)phenoxy)acetyl)piperazin-1-carboxylate (95 mg, 0.18 mmol, yield 58%). The synthesis of the tert-butyl (S)-4-(2-(4-(7,7-difluoro-2-(2-methylazetidin-1-yl)-6,7-dihydro-5H-cyclopenta[d]pyrimidin-4-yl)phenoxy)acetyl)piperazin-1-carboxylate thus obtained was confirmed through ¹H NMR analysis.

¹H NMR (500MHz, CDCl₃) δ 7.90 (d, 2H), 7.02 (d, 2H), 4.75 (s, 2H), 4.55 (m, 1H), 4.13 (m, 1H), 4.05 (m, 1H), 3.57 (m, 2H), 3.40 (m, 4H), 3.05 (m, 2H), 2.50 (m, 3H), 2.00 (m, 1H), 1.56 (d, 3H), 1.43 (s, 9H).

### (5) Step E: Preparation of (S)-4-(2-(4-(7,7-difluoro-2-(2-methylazetidin-1-yl)-6,7-dihydro-5H-cyclopenta[d]pyrimidin-4-yl)phenoxy)-1-(piperazin-1-yl)ethan-1-one

The tert-butyl (S)-4-(2-(4-(7,7-difluoro-2-(2-methylazetidin-1-yl)-6,7-dihydro-5H-cyclopenta[d]pyrimidin-4-yl)phenoxy)acetyl)piperazin-1-carboxylate (95 mg, 0.18 mmol) prepared in Step D and 1 ml of trifluoroacetic acid were dissolved in 3 ml of dichloromethane, followed by stirring at room temperature for 10 hours. After finishing the reaction, a saturated sodium bicarbonate solution was added to the reaction solution, and the resultant was extracted with ethyl acetate. An organic layer was washed with a saturated aqueous sodium chloride solution, dried over anhydrous magnesium sulfate and filtered. The filtrate was concentrated under a reduced pressure, and the concentrate under a reduced pressure was purified by column chromatography (7 N ammonia methanol solution/dichloromethane = 1/9 by a weight ratio) to obtain (S)-4-(2-(4-(7,7-difluoro-2-(2-methylazetidin-1-yl)-6,7-dihydro-5H-cyclopenta[d]pyrimidin-4-yl)phenoxy)-1-(piperazin-1-yl)ethan-1-one (55 mg, 0.12 mmol, yield 69%). The synthesis of the (S)-4-(2-(4-(7,7-difluoro-2-(2-methylazetidin-1-yl)-6,7-dihydro-5H-cyclopenta[d]pyrimidin-4-yl)phenoxy)-1-(piperazin-1-yl)ethan-1-one thus obtained was confirmed through ¹H NMR analysis.

¹H NMR (500MHz, CDCl₃) δ 7.88 (d, 2H), 7.01 (d, 2H), 4.71 (s, 2H), 4.54 (m, 1H), 4.13 (m, 1H), 4.03 (m, 1H), 3.56 (m, 2H), 3.51 (m, 2H), 3.05 (m, 2H), 2.81 (m, 4H), 2.52 (m, 2H), 2.48 (m, 1H), 1.97 (m, 1H), 1.55 (d, 3H).

### Example 33: Preparation of (S)-2-(4-(7,7-difluoro-2-(2-methylazetidin-1-yl)-6,7-dihydro-5H-cyclopenta[d]pyrimidin-4-yl)-2-fluorophenoxy)-1-(piperazin-1-yl)ethan-1-one)

As in Reaction 27, the processes of Steps A to E were performed in sequence to prepare the title compound of Formula 1-33.

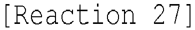

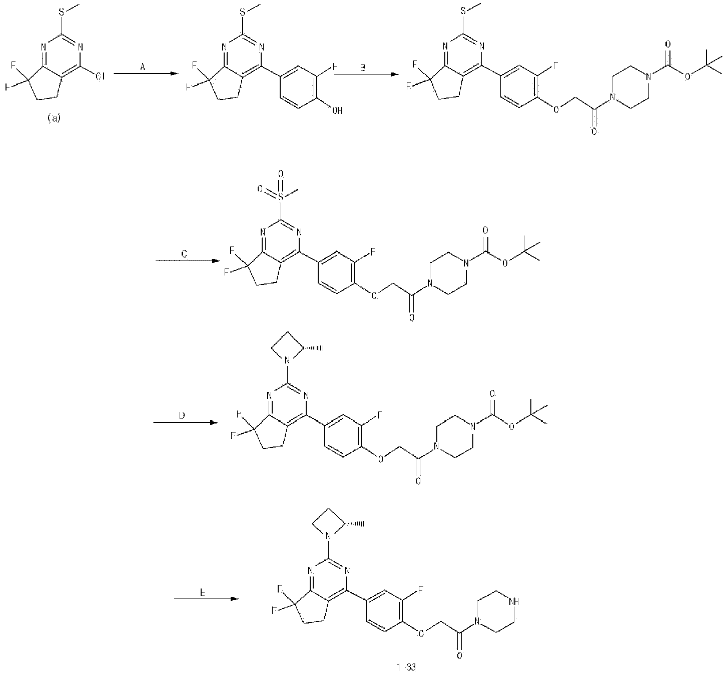

### (1) Step A: Preparation of 4-(7,7-difluoro-2-(methylthio)-6,7-dihydro-5H-cyclopenta[d]pyrimidin-4-yl)-2-fluorophenol

The 4-chloro-7,7-difluoro-2-(methylthio)-6,7-dihydro-cyclopenta[d]pyrimidine of Formula k (200 mg, 0.845 mmol) prepared in Preparation Example 11, (3-fluoro-4-hydroxyphenyl)boronic acid (198 mg, 1.27 mmol), tetrakis(triphenylphosphine)palladium(O) (49 mg, 0.042 mmol) and a sodium carbonate solution (2 M, 1.27 ml, 2.54 mmol) were dissolved in 20 ml of 1,4-dioxane, followed by stirring at 80°C for 2 hours. After finishing the reaction, water was added to the reaction solution, and the resultant was extracted with ethyl acetate. An organic layer was dried over anhydrous magnesium sulfate and filtered. The filtrate was concentrated under a reduced pressure and purified by column chromatography (ethyl acetate/hexane = 1/3 by a weight ratio) to obtain 4-(7,7-difluoro-2-(methylthio)-6,7-dihydro-5H-cyclopenta[d]pyrimidin-4-yl)-2-fluorophenol (260 mg, 0.83 mmol, yield 98%). The synthesis of the 4-(7,7-difluoro-2-(methylthio)-6,7-dihydro-5H-cyclopenta[d]pyrimidin-4-yl)-2-fluorophenol thus obtained was confirmed through ¹H NMR analysis.

¹H NMR (500MHz, CDCl₃) δ 7.8 (d, 1H), 7.72 (2H, 1H), 7.13 (t, 1H), 3.24 (m, 2H), 2.67 (s, 3H), 2.66 (m, 2H).

### (2) Step B: Preparation of tert-butyl 4-(2-(4-(7,7-difluoro-2-(methylthio)-6,7-dihydro-5H-cyclopenta[d]pyrimidin-4-yl)-2-fluorophenoxy)acetyl)piperazin-1-carboxylate

The 4-(7,7-difluoro-2-(methylthio)-6,7-dihydro-5H-cyclopenta[d]pyrimidin-4-yl)-2-fluorophenol (260 mg, 0.83 mmol) prepared in Step A, the tert-butyl 4-(2-bromoacetyl)piperazin-1-carboxylate of Formula c (307 mg, 1.00 mmol) prepared in Preparation Example 3 and potassium carbonate (345 mg, 345 mmol) were dissolved in 10 ml of dimethylformamide, followed by stirring at room temperature for 2 hours. After finishing the reaction, the reaction solution was concentrated under a reduced pressure. The resultant was dissolved in ethyl acetate and washed with a saturated sodium chloride solution. Then, an organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl 4-(2-(4-(7,7-difluoro-2-(methylthio)-6,7-dihydro-5H-cyclopenta[d]pyrimidin-4-yl)-2-fluorophenoxy)acetyl)piperazin-1-carboxylate (447 mg, 0.83 mmol, yield 100%). The synthesis of the tert-butyl 4-(2-(4-(7,7-difluoro-2-(methylthio)-6,7-dihydro-5H-cyclopenta[d]pyrimidin-4-yl)-2-fluorophenoxy)acetyl)piperazin-1-carboxylate thus obtained was confirmed by mass spectrometry (MS) measurement.
MS 538.6, 560.6 (Na⁺)

### (3) Step C: Preparation of tert-butyl 4-(2-(4-(7,7-difluoro-2-(methylsulfonyl)-6,7-dihydro-5H-cyclopenta[d]pyrimidin-4-yl)-2-fluorophenoxy)acetyl)piperazin-1-carboxylate

The tert-butyl 4-(2-(4-(7,7-difluoro-2-(methylthio)-6,7-dihydro-5H-cyclopenta[d]pyrimidin-4-yl)-2-fluorophenoxy)acetyl)piperazin-1-carboxylate (447 mg, 0.83 mmol) prepared in Step B and 3-chloroperbenzoic acid (70%, 512 mg, 2.08 mmol) were dissolved in 20 ml of dichloromethane, followed by stirring at room temperature for 10 hours. After finishing the reaction, a saturated sodium thiosulfate solution and a saturated sodium bicarbonate solution were added in sequence to the reaction solution, and the resultant was extracted with ethyl acetate. An organic layer was washed with an aqueous saturated sodium chloride solution, dried over anhydrous magnesium sulfate and filtered. The filtrate was concentrated under a reduced pressure, and the concentrate under a reduced pressure was purified by column chromatography (methanol/dichloromethane = 1/9 by a weight ratio) to obtain tert-butyl 4-(2-(4-(7,7-difluoro-2-(methylsulfonyl)-6,7-dihydro-5H-cyclopenta[d]pyrimidin-4-yl)-2-fluorophenoxy)acetyl)piperazin-1-carboxylate (420 mg, 0.74 mmol, yield 89%). The synthesis of the tert-butyl 4-(2-(4-(7,7-difluoro-2-(methylsulfonyl)-6,7-dihydro-5H-cyclopenta[d]pyrimidin-4-yl)-2-fluorophenoxy)acetyl)piperazin-1-carboxylate thus obtained was confirmed through mass spectrometry (MS) measurement.
MS 570.6, 592.6 (Na⁺)

### (4) Step D: Preparation of tert-butyl (S)-4-(2-(4-(7,7-difluoro-2-(2-methylazetidin-1-yl)-6,7-dihydro-5H-cyclopenta[d]pyrimidin-4-yl)-2-fluorophenoxy)acetyl)piperazin-1-carboxylate

The tert-butyl 4-(2-(4-(7,7-difluoro-2-(methylsulfonyl)-6,7-dihydro-5H-cyclopenta[d]pyrimidin-4-yl)-2-fluorophenoxy)acetyl)piperazin-1-carboxylate (420 mg, 0.74mmol) prepared in Step C, (2S)-2-methylazetidine[(1R,4S)-7,7-dimethyl-2-oxo-norbornan-1-yl]methanesulfonate (481 mg, 1.02 mmol) and potassium carbonate (127 mg, 0.92 mmol) were dissolved in 20 ml of acetonitrile, followed by stirring at 80°C for 2 hours. After finishing the reaction, an excessive amount of water was added to the reaction solution, and the resultant was extracted with ethyl acetate. An organic layer was washed with a saturated aqueous sodium chloride solution, dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl (S)-4-(2-(4-(7,7-difluoro-2-(2-methylazetidin-1-yl)-6,7-dihydro-5H-cyclopenta[d]pyrimidin-4-yl)-2-fluorophenoxy)acetyl)piperazin-1-carboxylate (320 mg, 0.57 mmol, yield 77%). The synthesis of the tert-butyl (S)-4-(2-(4-(7,7-difluoro-2-(2-methylazetidin-1-yl)-6,7-dihydro-5H-cyclopenta[d]pyrimidin-4-yl)-2-fluorophenoxy)acetyl)piperazin-1-carboxylate thus obtained was confirmed through ¹H NMR analysis.

¹H NMR (500MHz, CDCl₃) δ 7.79 (d, 1H), 7.66 (d, 1H). 7.13 (t, 1H), 4.83 (s, 2H), 4.58 (m, 1H), 4.16 (m, 1H), 4.08 (m, 1H), 3.59 (m, 4H), 3.50 (m, 2H), 3.40 (m, 2H), 2.59-2.40 (m, 3H), 2.03 (m, 1H), 1.58 (d, 3H), 1.45 (s, 9H).

### (5) Step E: Preparation of (S)-4-(2-(4-(7,7-difluoro-2-(2-methylazetidin-1-yl)-6,7-dihydro-5H-cyclopenta[d]pyrimidin-4-yl)-2-fluorophenoxy)-1-(piperazin-1-yl)ethan-1-one

The tert-butyl (S)-4-(2-(4-(7,7-difluoro-2-(2-methylazetidin-1-yl)-6,7-dihydro-5H-cyclopenta[d]pyrimidin-4-yl)-2-fluorophenoxy)acetyl)piperazin-1-carboxylate (320 mg, 0.57 mmol) prepared in Step D and 1 ml of trifluoroacetic acid were dissolved in 3 ml of dichloromethane, followed by stirring at room temperature for 10 hours. After finishing the reaction, a saturated sodium bicarbonate solution was added to the reaction solution, and the resultant was extracted with ethyl acetate. An organic layer was washed with a saturated aqueous sodium chloride solution, dried over anhydrous magnesium sulfate and filtered. The filtrate was concentrated under a reduced pressure, and the concentrate under a reduced pressure was purified by column chromatography (7 N ammonia methanol solution/dichloromethane = 1/9 by a weight ratio) to obtain (S)-4-(2-(4-(7,7-difluoro-2-(2-methylazetidin-1-yl)-6,7-dihydro-5H-cyclopenta[d]pyrimidin-4-yl)-2-fluorophenoxy)-1-(piperazin-1-yl)ethan-1-one (150 mg, 0.33 mmol, yield 58%). The synthesis of the (S)-4-(2-(4-(7,7-difluoro-2-(2-methylazetidin-1-yl)-6,7-dihydro-5H-cyclopenta[d]pyrimidin-4-yl)-2-fluorophenoxy)-1-(piperazin-1-yl)ethan-1-one thus obtained was confirmed through ¹H NMR analysis.

¹H NMR (500MHz, CDCl₃) δ 7.73 (dd, 1H), 7.62 (d, 1H), 7.08 (t, 1H), 4.80 (s, 2H), 4.53 (m, 1H), 4.12 (m, 1H), 4.04 (m, 1H), 3.55 (m, 4H), 3.04 (m, 2H), 2.85 (m, 4H), 2.51 (m, 2H), 2.48 (m, 1H), 1.99 (m, 1H), 1.54 (d, 3H).

### Example 34: Preparation of (S)-2-(4-(7,7-difluoro-2-(2-methylazetidin-1-yl)-6,7-dihydro-5H-cyclopenta[d]pyrimidin-4-yl)-2,6-difluorophenoxy)-1-(piperazin-1-yl)ethan-1-one)

As in Reaction 28, the processes of Steps A to E were performed in sequence to prepare the title compound of Formula 1-34.

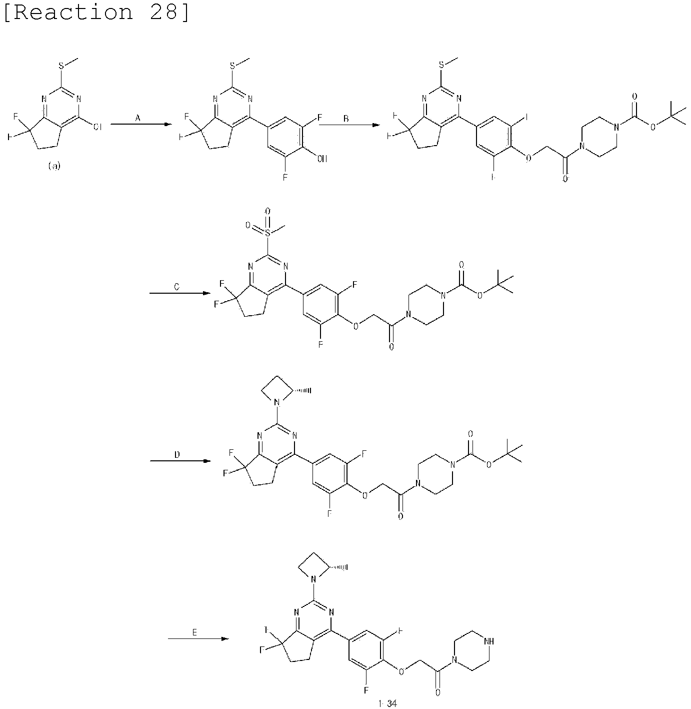

### (1) Step A: Preparation of 4-(7,7-difluoro-2-(methylthio)-6,7-dihydro-5H-cyclopenta[d]pyrimidin-4-yl)-2,6-difluorophenol

The 4-chloro-7,7-difluoro-2-(methylthio)-6,7-dihydro-cyclopenta[d]pyrimidine of Formula k (200 mg, 0.845 mmol) prepared in Preparation Example 11, (3,5-difluoro-4-hydroxyphenyl)boronic acid (176 mg, 1.01 mmol), tetrakis(triphenylphosphine)palladium(O) (49 mg, 0.042 mmol) and a sodium carbonate solution (2 M, 1.27 ml, 2.54 mmol) were dissolved in 20 ml of 1,4-dioxane, followed by stirring at 80°C for 2 hours. After finishing the reaction, water was added to the reaction solution, and the resultant was extracted with ethyl acetate. An organic layer was dried over anhydrous magnesium sulfate and filtered. The filtrate was concentrated under a reduced pressure and purified by column chromatography (ethyl acetate/hexane = 1/3 by a weight ratio) to obtain 4-(7,7-difluoro-2-(methylthio)-6,7-dihydro-5H-cyclopenta[d]pyrimidin-4-yl)-2,6-fluorophenol (214 mg, 0.647 mmol, yield 76%). The synthesis of the 4-(7,7-difluoro-2-(methylthio)-6,7-dihydro-5H-cyclopenta[d]pyrimidin-4-yl)-2,6-difluorophenol thus obtained was confirmed through ¹H NMR analysis.

¹H NMR (500MHz, CDCl₃) δ 7.66 (dd, J=16.3, 7.8 Hz, 2H), 3.73 (s, 1H), 3.32-3.18 (m, 2H), 2.07 (s, 1H), 1.58 (s, 3H), 1.28 (t, J=7.0 Hz, 1H).

### (2) Step B: Preparation of tert-butyl 4-(2-(4-(7,7-difluoro-2-(methylthio)-6,7-dihydro-5H-cyclopenta[d]pyrimidin-4-yl)-2,6-difluorophenoxy)acetyl)piperazin-1-carboxylate

The 4-(7,7-difluoro-2-(methylthio)-6,7-dihydro-5H-cyclopenta[d]pyrimidin-4-yl)-2,6-difluorophenol (214 mg, 0.647 mmol) prepared in Step A, the tert-butyl 4-(2-bromoacetyl)piperazin-1-carboxylate of Formula c (238 mg, 0.77 mmol) prepared in Preparation Example 3, and potassium carbonate (282 mg, 2.04 mmol) were dissolved in 10 ml of dimethylformamide, followed by stirring at room temperature for 2 hours. After finishing the reaction, the reaction solution was concentrated under a reduced pressure. The resultant was dissolved in ethyl acetate and washed with a saturated sodium chloride solution. Then, an organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl 4-(2-(4-(7,7-difluoro-2-(methylthio)-6,7-dihydro-5H-cyclopenta[d]pyrimidin-4-yl)-2,6-difluorophenoxy)acetyl)piperazin-1-carboxylate (310 mg, 0.556 mmol, yield 85%). The synthesis of the tert-butyl 4-(2-(4-(7,7-difluoro-2-(methylthio)-6,7-dihydro-5H-cyclopenta[d]pyrimidin-4-yl)-2,6-difluorophenoxy)acetyl)piperazin-1-carboxylate thus obtained was confirmed through ¹H NMR analysis.

¹H NMR (400MHz, CDCl₃) δ 7.63 (d, J=9.2 Hz, 2H), 5.01-4.91 (m, 3H), 3.63 (d, J=4.0 Hz, 2H), 3.52 (d, J=35.4 Hz, 10H), 3.24 (t, J=6.6 Hz, 2H), 1.50 (d, J=3.1 Hz, 9H).

### (3) Step C: Preparation of tert-butyl 4-(2-(4-(7,7-difluoro-2-(methylsulfonyl)-6,7-dihydro-5H-cyclopenta[d]pyrimidin-4-yl)-2,6-difluorophenoxy)acetyl)piperazin-1-carboxylate

The tert-butyl 4-(2-(4-(7,7-difluoro-2-(methylthio)-6,7-dihydro-5H-cyclopenta[d]pyrimidin-4-yl)-2,6-difluorophenoxy)acetyl)piperazin-1-carboxylate (310 mg, 0.556 mmol) prepared in Step B and 3-chloroperbenzoic acid (70%, 288 mg, 1.67 mmol) were dissolved in 20 ml of dichloromethane, followed by stirring at room temperature for 10 hours. After finishing the reaction, a saturated sodium thiosulfate solution and a saturated sodium bicarbonate solution were added in sequence to the reaction solution, and the resultant was extracted with ethyl acetate. An organic layer was washed with an aqueous saturated sodium chloride solution, dried over anhydrous magnesium sulfate and filtered. The filtrate was concentrated under a reduced pressure, and the concentrate under a reduced pressure was purified by column chromatography (methanol/dichloromethane = 1/9 by a weight ratio) to obtain tert-butyl 4-(2-(4-(7,7-difluoro-2-(methylsulfonyl)-6,7-dihydro-5H-cyclopenta[d]pyrimidin-4-yl)-2,6-difluorophenoxy)acetyl)piperazin-1-carboxylate(244 mg, 0.414 mmol, yield 74%) . The synthesis of the tert-butyl 4-(2-(4-(7,7-difluoro-2-(methylsulfonyl)-6,7-dihydro-5H-cyclopenta[d]pyrimidin-4-yl)-2,6-difluorophenoxy)acetyl)piperazin-1-carboxylate thus obtained was confirmed through ¹H NMR analysis.

¹H NMR (500MHz, CDCl₃) δ 7.81-7.67 (m, 2H), 5.04 (s, 2H), 3.63 (s, 2H), 3.54 (d, J=8.5 Hz, 4H), 3.49 (s, 3H), 3.42 (t, J=6.6 Hz, 2H), 2.90-2.71 (m, 2H), 1.51 (s, 9H).

### (4) Step D: Preparation of tert-butyl (S)-4-(2-(4-(7,7-difluoro-2-(2-methylazetidin-1-yl)-6,7-dihydro-5H-cyclopenta[d]pyrimidin-4-yl)-2,6-difluorophenoxy)acetyl)piperazin-1-carboxylate

The tert-butyl 4-(2-(4-(7,7-difluoro-2-(methylsulfonyl)-6,7-dihydro-5H-cyclopenta[d]pyrimidin-4-yl)-2,6-difluorophenoxy)acetyl)piperazin-1-carboxylate (244 mg, 0.41 mmol) prepared in Step C, (2S)-2-methylazetidine[(1R,2S)-7,7-dimethyl-2-oxo-norbornan-1-yl]methanesulfonate (150 mg, 0.49 mmol) and potassium carbonate (171 mg, 1.24 mmol) were dissolved in 20 ml of acetonitrile, followed by stirring at 80°C for 2 hours. After finishing the reaction, an excessive amount of water was added to the reaction solution and extracted with ethyl acetate. An organic layer was washed with a saturated aqueous sodium chloride solution, dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl (S)-4-(2-(4-(7,7-difluoro-2-(2-methylazetidin-1-yl)-6,7-dihydro-5H-cyclopenta[d]pyrimidin-4-yl)-2,6-difluorophenoxy)acetyl)piperazin-1-carboxylate (228 mg, 0.39 mmol, yield 94%). The synthesis of the tert-butyl (S)-4-(2-(4-(7,7-difluoro-2-(2-methylazetidin-1-yl)-6,7-dihydro-5H-cyclopenta[d]pyrimidin-4-yl)-2,6-difluorophenoxy)acetyl)piperazin-1-carboxylate thus obtained was confirmed through ¹H NMR analysis.

¹H NMR (500MHz, CDCl₃) δ 7.62-7.48 (m, 2H), 4.91 (s, 2H), 4.58 (q, J=6.7 Hz, 1H), 4.25-4.01 (m, 2H), 3.69-3.39 (m, 8H), 3.09 (d. J=3.7 Hz, 2H), 2.67-2.42 (m, 3H), 1.62-1.55 (m, 3H), 1.47 (d, J=12.8 Hz, 10H), 1.26 (t, J=7.2 Hz, 1H).

### (5) Step E: Preparation of (S)-4-(2-(4-(7,7-difluoro-2-(2-methylazetidin-1-yl)-6,7-dihydro-5H-cyclopenta[d]pyrimidin-4-yl)-2,6-difluorophenoxy)-1-(piperazin-1-yl)ethan-1-one

The tert-butyl (S)-4-(2-(4-(7,7-difluoro-2-(2-methylazetidin-1-yl)-6,7-dihydro-5H-cyclopenta[d]pyrimidin-4-yl)-2,6-difluorophenoxy)acetyl)piperazin-1-carboxylate (0.18 mmol) prepared in Step D and 1 ml of trifluoroacetic acid were dissolved in 3 ml of dichloromethane, followed by stirring at room temperature for 10 hours. After finishing the reaction, a saturated sodium bicarbonate solution was added to the reaction solution, and the resultant was extracted with ethyl acetate. An organic layer was washed with a saturated aqueous sodium chloride solution, dried over anhydrous magnesium sulfate and filtered. The filtrate was concentrated under a reduced pressure, and the concentrate under a reduced pressure was purified by column chromatography (7 N ammonia methanol solution/dichloromethane = 1/9 by a weight ratio) to obtain (S)-4-(2-(4-(7,7-difluoro-2-(2-methylazetidin-1-yl)-6,7-dihydro-5H-cyclopenta[d]pyrimidin-4-yl)-2,6-difluorophenoxy)-1-(piperazin-1-yl)ethan-1-one (139 mg, 0.2828 mmol, yield 73%). The synthesis of the (S)-4-(2-(4-(7,7-difluoro-2-(2-methylazetidin-1-yl)-6,7-dihydro-5H-cyclopenta[d]pyrimidin-4-yl)-2,6-difluorophenoxy)-1-(piperazin-1-yl)ethan-1-one thus obtained was confirmed through ¹H NMR analysis.

¹H NMR (500MHz, MeOD) δ 7.55 (dd, J=15.3, 5.5 Hz, 2H), 5.00 (s, 2H), 4.60-4.45 (m, 1H), 4.17-3.93 (m, 2H), 3.64-3.44 (m, 4H), 3.15-2.99 (m, 2H), 2.95-2.75 (m, 4H), 2.64-2.40 (m, 3H), 2.11-1.89 (m, 1H), 1.61-1.47 (m, 3H).

### Example 35: Preparation of (S)-2-(2-chloro-4-(7,7-difluoro-2-(2-methylazetidin-1-yl)-6,7-dihydro-5H-cyclopenta[d]pyrimidin-4-yl)phenoxy)-1-(piperazin-1-yl)ethan-1-one)

As in Reaction 29, the processes of Steps A to E were performed in sequence to prepare the title compound of Formula 1-35.

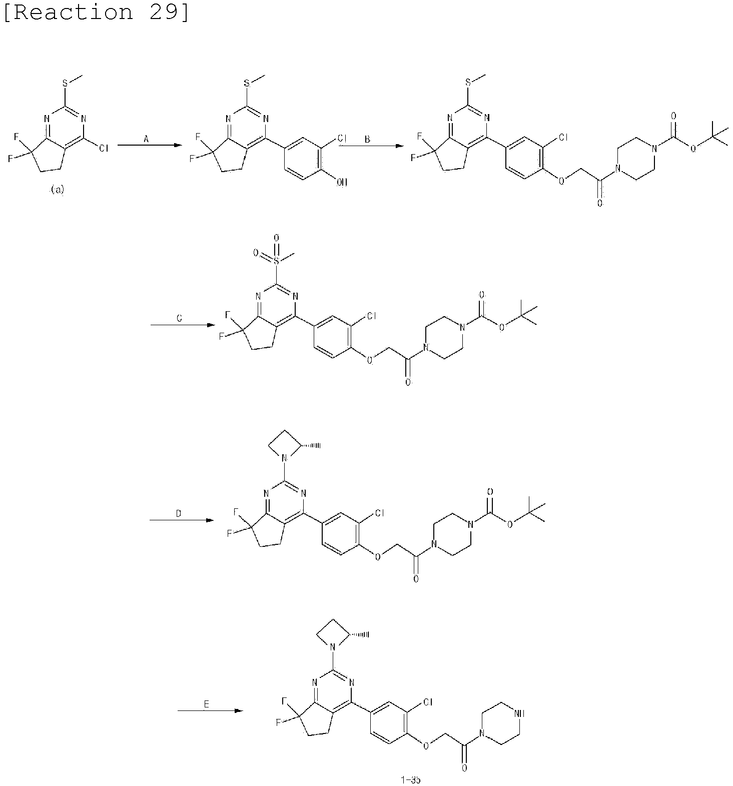

### (1) Step A: Preparation of 2-chloro-4-(7,7-difluoro-2-(methylthio)-6,7-dihydro-5H-cyclopenta[d]pyrimidin-4-yl)phenol

The 4-chloro-7,7-difluoro-2-(methylthio)-6,7-dihydro-cyclopenta[d]pyrimidine of Formula k (200 mg, 0.845 mmol) prepared in Preparation Example 11, (3-chloro-4-hydroxyphenyl)boronic acid (174 mg, 1.01 mmol), tetrakis(triphenylphosphine)palladium(O) (49 mg, 0.042 mmol) and a sodium carbonate solution (2 M, 1.27 ml, 2.54 mmol) were dissolved in 20 ml of 1,4-dioxane, followed by stirring at 80°C for 2 hours. After finishing the reaction, water was added to the reaction solution, and the resultant was extracted with ethyl acetate. An organic layer was dried over anhydrous magnesium sulfate and filtered. The filtrate was concentrated under a reduced pressure and purified by column chromatography (ethyl acetate/hexane = 1/3 by a weight ratio) to obtain 2-chloro-4-(7,7-difluoro-2-(methylthio)-6,7-dihydro-5H-cyclopenta[d]pyrimidin-4-yl)phenol (224 mg, 0.68 mmol, yield 80%). The synthesis of the 2-chloro-4-(7,7-difluoro-2-(methylthio)-6,7-dihydro-5H-cyclopenta[d]pyrimidin-4-yl)phenol thus obtained was confirmed through ¹H NMR analysis.

¹H NMR (500MHz, CDCl₃) δ 8.07 (d, J=1.7 Hz, 1H), 7.84 (dd, J=8.5, 2.1 Hz, 1H), 7.22-7.02 (m, 1H), 3.22 (t, J=6.6 Hz, 2H), 2.75-2.55 (m, 5H).

### (2) Step B: Preparation of tert-butyl 4-(2-(2-chloro-4-(7,7-difluoro-2-(methylthio)-6,7-dihydro-5H-cyclopenta[d]pyrimidin-4-yl)phenoxy)acetyl)piperazin-1-carboxylate

The 4-(7,7-difluoro-2-(methylthio)-6,7-dihydro-5H-cyclopenta[d]pyrimidin-4-yl)-2,6-difluorophenol (224 mg, 0.681 mmol) prepared in Step A, the tert-butyl 4-(2-bromoacetyl)piperazin-1-carboxylate of Formula c (251 mg, 0.81 mmol) prepared in Preparation Example 3 and potassium carbonate (282 mg, 2.04 mmol) were dissolved in 10 ml of dimethylformamide, followed by stirring at room temperature for 2 hours. After finishing the reaction, the reaction solution was concentrated under a reduced pressure. The resultant was dissolved in ethyl acetate and washed with a saturated sodium chloride solution. Then, an organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl 4-(2-(2-chloro-4-(7,7-difluoro-2-(methylthio)-6,7-dihydro-5H-cyclopenta[d]pyrimidin-4-yl)phenoxy)acetyl)piperazin-1-carboxylate (333 mg, 0.59 mmol, yield 88 %). The synthesis of the tert-butyl 4-(2-(2-chloro-4-(7,7-difluoro-2-(methylthio)-6,7-dihydro-5H-cyclopenta[d]pyrimidin-4-yl)phenoxy)acetyl)piperazin-1-carboxylate thus obtained was confirmed through ¹H NMR analysis.

¹H NMR (400MHz, CDCl₃) δ 8.22 (s, 1H), 8.01 (d, J=7.9 Hz, 1H), 7.21 (d, J=8.8 Hz, 1H), 4.93 (s, 2H), 3.72-3.55 (m, 4H), 3.57-3.46 (m, 5H), 3.46-3.34 (m, 4H), 2.88-2.70 (m, 2H), 1.49 (s, 9H).

### (3) Step C: Preparation of tert-butyl 4-(2-(2-chloro-4-(7,7-difluoro-2-(methylsulfonyl)-6,7-dihydro-5H-cyclopenta[d]pyrimidin-4-yl)phenoxy)acetyl)piperazin-1-carboxylate

The tert-butyl 4-(2-(2-chloro-4-(7,7-difluoro-2-(methylthio)-6,7-dihydro-5H-cyclopenta[d]pyrimidin-4-yl)phenoxy)acetyl)piperazin-1-carboxylate (333 mg, 0.559 mmol) prepared in Step B and 3-chloroperbenzoic acid (70%, 310 mg, 1.779 mmol) were dissolved in 20 ml of dichloromethane, followed by stirring at room temperature for 10 hours. After finishing the reaction, a saturated sodium thiosulfate solution and a saturated sodium bicarbonate solution were added in sequence to the reaction solution, and the resultant was extracted with ethyl acetate. An organic layer was washed with an aqueous saturated sodium chloride solution, dried over anhydrous magnesium sulfate and filtered. The filtrate was concentrated under a reduced pressure, and the concentrate under a reduced pressure was purified by column chromatography (methanol/dichloromethane = 1/9 by a weight ratio) to obtain tert-butyl 4-(2-(2-chloro-4-(7,7-difluoro-2-(methylsulfonyl)-6,7-dihydro-5H-cyclopenta[d]pyrimidin-4-yl)phenoxy)acetyl)piperazin-1-carboxylate (329 mg, 0.566 mmol, yield 93%). The synthesis of the tert-butyl 4-(2-(2-chloro-4-(7,7-difluoro-2-(methylsulfonyl)-6,7-dihydro-5H-cyclopenta[d]pyrimidin-4-yl)phenoxy)acetyl)piperazin-1-carboxylate thus obtained was confirmed through ¹H NMR analysis.

¹H NMR (500MHz, CDCl₃) δ 8.22 (s, 1H), 8.01 (d, J=7.9 Hz, 1H), 7.21 (d, J=8.8 Hz, 1H), 4.93 (s, 2H), 3.72-3.55 (m, 4H), 3.57-3.46 (m, 5H), 3.46-3.34 (m, 4H), 2.88-2.70 (m, 2H), 1.49 (s, 9H).

### (4) Step D: Preparation of tert-butyl 4-(2-(2-chloro-4-(7,7-difluoro-2-(2-methylazetidin-1-yl)-6,7-dihydro-5H-cyclopenta[d]pyrimidin-4-yl)phenoxy)acetyl)piperazin-1-carboxylate

The tert-butyl 4-(2-(2-chloro-4-(7,7-difluoro-2-(methylsulfonyl)-6,7-dihydro-5H-cyclopenta[d]pyrimidin-4-yl)phenoxy)acetyl)piperazin-1-carboxylate (329 mg, 0.56 mmol) prepared in Step C, (2S)-2-methylazetidine[(1R,4S)-7,7-dimethyl-2-oxo-norbornan-1-yl] methanesulfonate (204 mg, 0.67 mmol) and potassium carbonate (232 mg, 1.68 mmol) were dissolved in 20 ml of acetonitrile, followed by stirring at 80°C for 2 hours. After finishing the reaction, an excessive amount of water was added to the reaction solution, and the resultant was extracted with ethyl acetate. An organic layer was washed with a saturated aqueous sodium chloride solution, dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl 4-(2-(2-chloro-4-(7,7-difluoro-2-(2-methylazetidin-1-yl)-6,7-dihydro-5H-cyclopenta[d]pyrimidin-4-yl)phenoxy)acetyl)piperazin-1-carboxylate (299 mg, 0.51 mmol, yield 92%). The synthesis of the tert-butyl 4-(2-(2-chloro-4-(7,7-difluoro-2-(2-methylazetidin-1-yl)-6,7-dihydro-5H-cyclopenta[d]pyrimidin-4-yl)phenoxy)acetyl)piperazin-1-carboxylate thus obtained was confirmed through ¹H NMR analysis.

¹H NMR (500MHz, CDCl₃) δ 8.03 (d, J=1.8 Hz, 1H), 7.81 (d, J=8.5 Hz, 1H), 7.11 (d, J=8.5 Hz, 1H), 4.86 (s, 2H), 4.59 (dt, J=20.0, 6.3 Hz, 1H), 4.26-4.01 (m, 3H), 3.72-3.51 (m, 4H), 3.44 (d, J=35.1 Hz, 4H), 3.20-2.99 (m, 2H), 2.66-2.39 (m, 3H), 2.12-1.94 (m, 1H), 1.61-1.57 (m, 3H), 1.52-1.38 (m, 9H).

### (5) Step E: Preparation of (S)-2-(2-chloro-4-(7,7-difluoro-2-(2-methylazetidin-1-yl)-6,7-dihydro-5H-cyclopenta[d]pyrimidin-4-yl)phenoxy)-1-(piperazin-1-yl)ethan-1-one

The tert-butyl 4-(2-(2-chloro-4-(7,7-difluoro-2-(2-methylazetidin-1-yl)-6,7-dihydro-5H-cyclopenta[d]pyrimidin-4-yl)phenoxy)acetyl)piperazin-1-carboxylate (299 mg, 0.51 mmol) prepared in Step D and 1 ml of trifluoroacetic acid were dissolved in 3 ml of dichloromethane, followed by stirring at room temperature for 10 hours. After finishing the reaction, a saturated sodium bicarbonate solution was added to the reaction solution, and the resultant was extracted with ethyl acetate. An organic layer was washed with a saturated aqueous sodium chloride solution, dried over anhydrous magnesium sulfate and filtered. The filtrate was concentrated under a reduced pressure, and the concentrate under a reduced pressure was purified by column chromatography (7 N ammonia methanol solution/dichloromethane = 1/9 by a weight ratio) to obtain (S)-2-(2-chloro-4-(7,7-difluoro-2-(2-methylazetidin-1-yl)-6,7-dihydro-5H-cyclopenta[d]pyrimidin-4-yl)phenoxy)-1-(piperazin-1-yl) ethan-1-one (179 mg, 0.37 mmol, yield 72%) . The synthesis of the (S)-2-(2-chloro-4-(7,7-difluoro-2-(2-methylazetidin-1-yl)-6,7-dihydro-5H-cyclopenta[d]pyrimidin-4-yl)phenoxy)-1-(piperazin-1-yl)ethan-1-one thus obtained was confirmed through ¹H NMR analysis.

¹H NMR (500MHz, MeOD) δ 7.97 (d, J=1.8 Hz, 1H), 7.85-7.66 (m, 1H), 7.08-6.91 (m, 1H), 5.12-4.87 (m, 2H), 4.49 (dt, J=19.8, 6.3 Hz, 1H), 4.17-3.88 (m, 2H), 3.71-3.40 (m, 4H), 3.14-2.93 (m, 2H), 2.93-2.60 (m, 4H), 2.65-2.35 (m, 3H), 2.10-1.89 (m, 1H), 1.72-1.18 (m, 3H).

### Example 36: Preparation of (S)-2-(2-(chloro-4-(7,7-difluoro-2-(2-methylazetidin-1-yl)-6,7-dihydro-5H-cyclopenta[d]pyrimidin-4-yl)phenoxy)-1-(1,4-diazepan-1-yl)ethan-1-one)

As in Reaction 30, the processes of Steps A to E were performed in sequence to prepare the title compound of Formula 1-36.

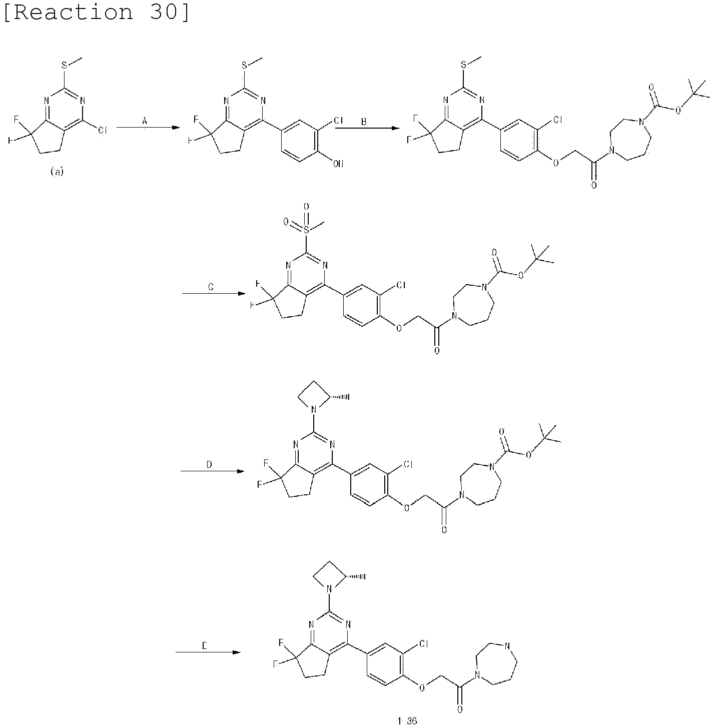

### (1) Step A: Preparation of 2-chloro-4-(7,7-difluoro-2-(methylthio)-6,7-dihydro-5H-cyclopenta[d]pyrimidin-4-yl)phenol

The same procedure in (1) of Example 35 was performed to prepare 2-chloro-4-(7,7-difluoro-2-(methylthio)-6,7-dihydro-5H-cyclopenta[d]pyrimidin-4-yl)phenol.

### (2) Step B: Preparation of tert-butyl 4-(2-(2-chloro-4-(7,7-difluoro-2-(methylthio)-6,7-dihydro-5H-cyclopenta[d]pyrimidin-4-yl)phenoxy)acetyl)-1,4-diazepan-1-carboxylate

The 2-chloro-4-(7,7-difluoro-2-(methylthio)-6,7-dihydro-5H-cyclopenta[d]pyrimidin-4-yl)phenol (205 mg, 0.62 mmol) prepared in Step A, the tert-butyl 4-(2-bromoacetyl)-1,4-diazepan-1-carboxylate of Formula d (240 mg, 0.74 mmol) prepared in Preparation Example 4, and potassium carbonate (258 mg, 1.87 mmol) were dissolved in 10 ml of dimethylformamide, followed by stirring at room temperature for 2 hours. After finishing the reaction, the reaction solution was concentrated under a reduced pressure. The resultant was dissolved in ethyl acetate and washed with a saturated sodium chloride solution. Then, an organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl 4-(2-(2-chloro-4-(7,7-difluoro-2-(methylthio)-6,7-dihydro-5H-cyclopenta[d]pyrimidin-4-yl)phenoxy)acetyl)-1,4-diazepan-1-carboxylate (303 mg, 0.53 mmol, yield 85%) . The synthesis of the tert-butyl 4-(2-(2-chloro-4-(7,7-difluoro-2-(methylthio)-6,7-dihydro-5H-cyclopenta[d]pyrimidin-4-yl)phenoxy)acetyl)-1,4-diazepan-1-carboxylate thus obtained was confirmed through ¹H NMR analysis.

¹H NMR (400MHz, CDCl₃) δ 8.11 (s, 1H), 7.88 (d, J=8.5 Hz, 1H), 7.14 (d, J=8.8 Hz, 1H), 4.90 (d, J=7.9 Hz, 2H), 3.78-3.11 (m, 12H), 2.76-2.57 (m, 5H), 2.03-1.81 (m, 2H), 1.60 (d, J=11.0 Hz, 6H), 1.48 (s, 6H), 1.46 (s, 4H).

### (3) Step C: Preparation of tert-butyl 4-(2-(2-chloro-4-(7,7-difluoro-2-(methylsulfonyl)-6,7-dihydro-5H-cyclopenta[d]pyrimidin-4-yl)phenoxy)acetyl)-1,4-azepan-1-carboxylate

The tert-butyl 4-(2-(2-chloro-4-(7,7-difluoro-2-(methylthio)-6,7-dihydro-5H-cyclopenta[d]pyrimidin-4-yl)phenoxy)acetyl)-1,4-diazepan-1-carboxylate (303 mg. 0.532 mmol) prepared in Step B and 3-chloroperbenzoic acid (70%, 189 mg, 0.77 mmol) were dissolved in 20 ml of dichloromethane, followed by stirring at room temperature for 10 hours. After finishing the reaction, a saturated sodium thiosulfate solution and a saturated sodium bicarbonate solution were added in sequence to the reaction solution, and the resultant was extracted with ethyl acetate. An organic layer was washed with an aqueous saturated sodium chloride solution, dried over anhydrous magnesium sulfate and filtered. The filtrate was concentrated under a reduced pressure, and the concentrate under a reduced pressure was purified by column chromatography (methanol/dichloromethane = 1/9 by a weight ratio) to obtain tert-butyl 4-(2-(2-chloro-4-(7,7-difluoro-2-(methylsulfonyl)-6,7-dihydro-5H-cyclopenta[d]pyrimidin-4-yl)phenoxy)acetyl)-1,4-diazepan-1-carboxylate (281 mg, 0.467 mmol, yield 87%) . The synthesis of the tert-butyl 4-(2- (2-chloro-4-(7,7-difluoro-2-(methylsulfonyl)-6,7-dihydro-5H-cyclopenta[d]pyrimidin-4-yl)phenoxy)acetyl)-1,4-diazepan-1-carboxylate thus obtained was confirmed through ¹H NMR analysis.

¹H NMR (500MHz, CDCl₃) δ 8.22 (d, J=22.3 Hz, 1H), 8.06-7.93 (m, 1H), 7.17 (d, J=8.8 Hz, 1H), 4.93 (d, J=6.7 Hz, 2H), 3.79-3.29 (m, 12H), 2.88-2.69 (m, 2H), 2.05-1.81 (m, 2H), 1.47 (d, J=10.1 Hz, 9H).

### (4) Step D: Preparation of (S)-tert-butyl 4-(2-(2-chloro-4-(7,7-difluoro-2-(2-methylazetidin-1-yl)-6,7-dihydro-5H-cyclopenta[d]pyrimidin-4-yl)phenoxy)acetyl)-1,4-diazepan-1-carboxylate

The tert-butyl 4-(2-(2-chloro-4-(7,7-difluoro-2-(methylsulfonyl)-6,7-dihydro-5H-cyclopenta[d]pyrimidin-4-yl)phenoxy)acetyl)-1,4-diazepan-1-carboxylate (281 mg, 0.24 mmol) prepared in Step C, (2S)-2-methylazetidine[(1R,4S)-7,7-dimethyl-2-oxo-norbornan-1-yl]heptan-methanesulfonate (170 mg, 0.56 mmol) and potassium carbonate (193 mg, 1.40 mmol) were dissolved in 20 ml of acetonitrile, followed by stirring at 80°C for 2 hours. After finishing the reaction, an excessive amount of water was added to the reaction solution, and the resultant was extracted with ethyl acetate. An organic layer was washed with a saturated aqueous sodium chloride solution, dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain (S)-tert-butyl 4-(2-(2-chloro-4-(7,7-difluoro-2-(2-methylazetidin-1-yl)-6,7-dihydro-5H-cyclopenta[d]pyrimidin-4-yl)phenoxy)acetyl)-1,4-diazepan-1-carboxylate (255 mg, 0.43 mmol, yield 92%) . The synthesis of the (S)-tert-butyl 4-(2-(2-chloro-4-(7,7-difluoro-2-(2-methylazetidin-1-yl)-6,7-dihydro-5H-cyclopenta[d]pyrimidin-4-yl)phenoxy)acetyl)-1,4-diazepan-1-carboxylate thus obtained was confirmed through ¹H NMR analysis.

¹H NMR (500MHz, CDCl₃) δ 8.04 (s, 1H), 7.82 (d, J=7.9 Hz, 1H), 7.13 (s, 1H), 4.61 (s, 1H), 4.26-4.04 (m, 4H), 3.72 (s, 3H), 3.66-3.42 (m, 1H), 3.44-3.27 (m, 2H), 3.12 (s, 3H), 2.67-2.48 (m, 2H), 2.02 (d, J=49.4 Hz, 4H), 1.61 (d, J=6.4 Hz, 5H), 1.47 (d, J=9.5 Hz, 12H).

### (5) Step E: Preparation of (S)-2-(2-chloro-4-(7,7-difluoro-2-(2-methylazetidin-1-yl)-6,7-dihydro-5H-cyclopenta[d]pyrimidin-4-yl)phenoxy)-1-(1,4-diazepan-1-yl)ethan-1-one

The (S)-tert-butyl 4-(2-(2-chloro-4-(7,7-difluoro-2-(2-methylazetidin-1-yl)-6,7-dihydro-5H-cyclopenta[d]pyrimidin-4-yl)phenoxy)acetyl)-1,4-diazepan-1-carboxylate (255 mg, 0.43 mmol) prepared in Step D and 1 ml of trifluoroacetic acid were dissolved in 3 ml of dichloromethane, followed by stirring at room temperature for 10 hours. After finishing the reaction, a saturated sodium bicarbonate solution was added to the reaction solution, and the resultant was extracted with ethyl acetate. An organic layer was washed with a saturated aqueous sodium chloride solution, dried over anhydrous magnesium sulfate and filtered. The filtrate was concentrated under a reduced pressure, and the concentrate under a reduced pressure was purified by column chromatography (7 N ammonia methanol solution/dichloromethane = 1/9 by a weight ratio) to obtain (S)-2-(2-chloro-4-(7,7-difluoro-2-(2-methylazetidin-1-yl)-6,7-dihydro-5H-cyclopenta[d]pyrimidin-4-yl)phenoxy)-1-(piperazin-1-yl)ethan-1-one (190 mg, 0.368 mmol, yield 90%). The synthesis of the (S)-2-(2-chloro-4-(7,7-difluoro-2-(2-methylazetidin-1-yl)-6,7-dihydro-5H-cyclopenta[d]pyrimidin-4-yl)phenoxy)-1-(1,4-diazepan-1-yl)ethan-1-one thus obtained was confirmed through ¹H NMR analysis.

¹H NMR (500MHz, MeOD) δ 8.07 (s, 1H), 7.88 (d, J=8.8 Hz, 1H), 7.16 (t, J=8.2 Hz, 1H), 5.02 (d, J=9.5 Hz, 2H), 4.57 (q, J=6.6 Hz, 1H), 4.19-3.98 (m, 2H), 3.75-3.57 (m, 4H), 3.14 (t, J=6.4 Hz, 2H), 3.03 (t, J=5.3 Hz, 1H), 2.97-2.79 (m, 3H), 2.66-2.43 (m, 3H), 2.05 (d, J=9.5 Hz, 1H), 1.94 (d, J=11.3 Hz, 1H), 1.80 (t, J=5.8 Hz, 1H), 1.57 (d, J=6.4 Hz, 3H).

### Example 37: Preparation of (S)-2-((5-(7,7-difluoro-2-(2-methylazetidin-1-yl)-6,7-dihydro-5H-cyclopenta[d]pyrimidin-4-yl)pyridin-2-yl)oxy)-1-(piperazin-1-yl)ethan-1-one)

As in Reaction 31, the processes of Steps A to C were performed in sequence to prepare the title compound of Formula 1-37.

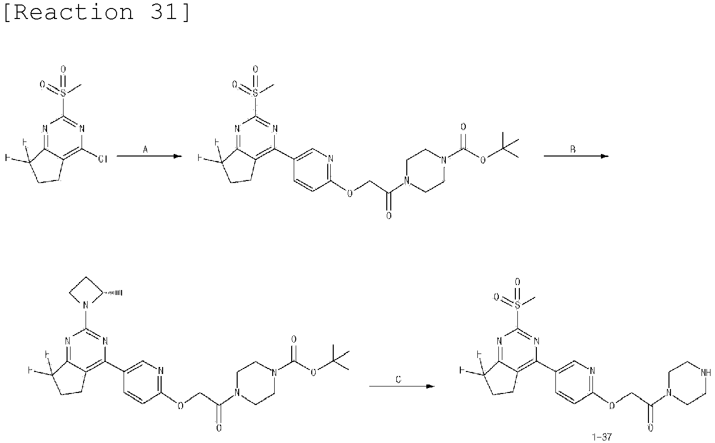

### (1) Step A: Preparation of tert-butyl 4-(2-((5-(7,7-difluoro-2-(methylsulfonyl)-6,7-dihydro-5H-cyclopenta[d]pyrimidin-4-yl)pyridin-2-yl)oxy)acetyl)piperazin-1-carboxylate

4-Chloro-7,7-difluoro-2-(methylsulfonyl)-6,7-dihydro-5H-cyclopenta[d]pyrimidine (317 mg, 1.18 mmol), the tert-butyl 4-(2-((5-(4,4,5,5-tetramethyl-1,3,2-dioxaborolan-2-yl)pyridin-2-yl)oxy)acetyl)piperazin-1-carboxylate (792 mg, 1.77 mmol) prepared in Preparation Example 17, and potassium carbonate (489 mg, 3.54 mmol) were dissolved in 20 ml of 1,2-dimethoxyethane/water (4/1 by a weight ratio), followed by degassing for 5 minutes. A 1,1'-bis(diphenylphosphino)ferrocene-palladium (II) dichloridedichloromethane complex (48 mg, 0.059 mmol) was added thereto, followed by stirring at 60°C for 1 hour. After finishing the reaction, the reaction solution was diluted in ethyl acetate and washed with water and a saturated aqueous sodium chloride solution. An organic layer was dried over anhydrous magnesium sulfate and filtered. The filtrate was concentrated under a reduced pressure, and the concentrate under a reduced pressure was purified by column chromatography (dichloromethane/methanol = 19/1 by a weight ratio) to obtain tert-butyl 4-(2-((5-(7,7-difluoro-2-(methylsulfonyl)-6,7-dihydro-5H-cyclopenta[d]pyrimidin-4-yl)pyridin-2-yl)oxy)acetyl)piperazin-1-carboxylate (367 mg, 0.66 mmol, yield 56%). The synthesis of the tert-butyl 4-(2-((5-(7,7-difluoro-2-(methylsulfonyl)-6,7-dihydro-5H-cyclopenta[d]pyrimidin-4-yl)pyridin-2-yl)oxy)acetyl)piperazin-1-carboxylate thus obtained was confirmed through ¹H NMR analysis.

¹H NMR (400MHz, DMSO-d₆) δ 8.87 (d, J=2.1 Hz, 1H), 8.48 (dd, J=8.7, 2.3 Hz, 1H), 7.16 (d, J=8.8 Hz, 1H), 5.23 (s, 2H), 3.54 (s, 3H), 3.45 (d, J=24.1 Hz, 8H), 3.29-3.23 (m, 2H), 2.87-2.67 (m, 2H), 1.41 (d, J=12.8 Hz, 9H).

### (2) Step B: Preparation of tert-butyl (S)-4-(2-((5-(7,7-difluoro-2-(2-methylazetidin-1-yl)-6,7-dihydro-5H-cyclopenta[d]pyrimidin-4-yl)pyridin-2-yl)oxy)acetyl)piperazin-1-carboxylate

The tert-butyl 4-(2-((5-(7,7-difluoro-2-(methylsulfonyl)-6,7-dihydro-5H-cyclopenta[d]pyrimidin-4-yl)pyridin-2-yl)oxy)acetyl)piperazin-1-carboxylate (367 mg, 0.66 mmol) prepared in Step A, (2S)-2-methylazetidine[(1R,4S)-7,7-dimethyl-2-oxo-norbornan-1-yl]methanesulfonate (241 mg, 0.79 mmol), and potassium carbonate (274 mg, 1.98 mmol) were dissolved in 20 ml of acetonitrile, followed by stirring at 80°C for 1 hour. After finishing the reaction, the reaction solution was concentrated under a reduced pressure. To the concentrate under a reduced pressure, ethyl acetate was added, and the resultant was washed with a saturated aqueous sodium chloride solution. An organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl (S)-4-(2-((5-(7,7-difluoro-2-(2-methylazetidin-1-yl)-6,7-dihydro-5H-cyclopenta[d]pyrimidin-4-yl)pyridin-2-yl)oxy)acetyl)piperazin-1-carboxylate (332 mg, 0.60 mmol, yield 91%). The synthesis of the tert-butyl (S)-4-(2-((5-(7,7-difluoro-2-(2-methylazetidin-1-yl)-6,7-dihydro-5H-cyclopenta[d]pyrimidin-4-yl)pyridin-2-yl)oxy)acetyl)piperazin-1-carboxylate thus obtained was confirmed through ¹H NMR analysis.

¹H NMR (500MHz, CDCl₃) δ 8.88 (d, J=2.1 Hz, 1H), 8.25 (dd, J=8.7, 2.3 Hz, 1H), 7.00 (d, J=8.5 Hz, 1H), 5.10 (s, 2H), 4.71-4.47 (m, 1H), 4.10 (dd, J=21.8, 2.1 Hz, 3H), 3.62 (s, 2H), 3.48 (d, J=30.5 Hz, 6H), 3.09 (d, J=6.7 Hz, 2H), 2.57 (s, 2H), 2.04-1.99 (m, 1H), 1.58 (d, J=6.1 Hz, 3H), 1.48 (s, 9H).

### (3) Step C: Preparation of (S)-2-((5-(7,7-difluoro-2-(2-methylazetidin-1-yl)-6,7-dihydro-5H-cyclopenta[d]pyrimidin-4-yl)pyridin-2-yl)oxy)-1-(piperazin-1-yl)ethan-1-one

The tert-butyl (S)-4-(2-((5-(7,7-difluoro-2-(2-methylazetidin-1-yl)-6,7-dihydro-5H-cyclopenta[d]pyrimidin-4-yl)pyridin-2-yl)oxy)acetyl)piperazin-1-carboxylate (332 mg, 0.60 mmol) prepared in Step B and 2 ml of trifluoroacetic acid were dissolved in 20 ml of dichloromethane, followed by stirring at room temperature for 18 hours. After finishing the reaction, the reaction solution was concentrated under a reduced pressure. To the concentrate under a reduced pressure, a saturated aqueous ammonium solution was added, and the resultant was extracted with ethyl acetate. An organic layer was dried over anhydrous magnesium sulfate and filtered. The filtrate was concentrated under a reduced pressure, and the concentrate under a reduced pressure was purified by column chromatography (7 N ammonia methanol solution/dichloromethane = 1/10 by a weight ratio) to obtain (S)-2-((5-(7,7-difluoro-2-(2-methylazetidin-1-yl)-6,7-dihydro-5H-cyclopenta[d]pyrimidin-4-yl)pyridin-2-yl)oxy)-1-(piperazin-1-yl)ethan-1-one (230 mg, 0.51 mmol, yield 84%). The synthesis of the (S)-2-((5-(7,7-difluoro-2-(2-methylazetidin-1-yl)-6,7-dihydro-5H-cyclopenta[d]pyrimidin-4-yl)pyridin-2-yl)oxy)-1-(piperazin-1-yl)ethan-1-one thus obtained was confirmed through ¹H NMR analysis.

¹H NMR (500MHz, MeOD) δ 8.72 (s, 1H), 8.35 (d, J=8.5 Hz, 1H), 7.02 (d, J=8.8 Hz, 1H), 5.17 (s, 2H), 4.57 (q, J=7.0 Hz, 1H), 4.19-3.98 (m, 2H), 3.55 (d. J=4.9 Hz, 4H), 3.13 (d, J=4.6 Hz, 2H), 2.96-2.75 (m, 4H), 2.66-2.44 (m, 3H), 2.05 (d, J=9.8 Hz, 1H), 1.57 (d, J=6.1 Hz, 3H).

### Example 38: Preparation of (S)-2-((3-chloro-5-(7,7-difluoro-2-(2-methylazetidin-1-yl)-6,7-dihydro-5H-cyclopenta[d]pyrimidin-4-yl)pyridin-2-yl)oxy)-1-(piperazin-1-yl)ethan-1-one)

As in Reaction 32, the processes of Steps A to C were performed in sequence to prepare the title compound of Formula 1-38.

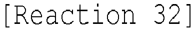

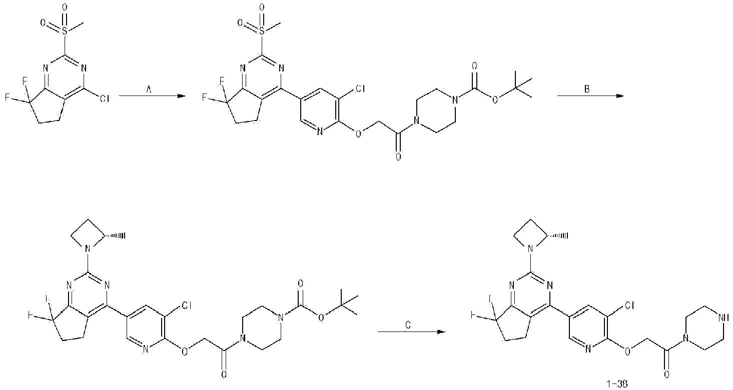

### (1) Step A: Preparation of tert-butyl 4-(2-((3-chloro-5-(7,7-difluoro-2-(methylsulfonyl)-6,7-dihydro-5H-cyclopenta[d]pyrimidin-4-yl)pyridin-2-yl)oxy)acetyl)piperazin-1-carboxylate

4-Chloro-7,7-difluoro-2-(methylsulfonyl)-6,7-dihydro-5H-cyclopenta[d]pyrimidine (260 mg, 0.97 mmol), the tert-butyl 4-(2-((3-chloro-5-(4,4,5,5-tetramethyl-1,3,2-dioxaborolan-2-yl)pyridin-2-yl)oxy)acetyl)piperazin-1-carboxylate (776 mg, 1.61 mmol) prepared in Preparation Example 18, and potassium carbonate (416 mg, 3.00 mmol) were dissolved in 20 ml of 1,2-dimethoxyethane/water (4/1 by a weight ratio), followed by degassing for 5 minutes. A 1,1'-bis(diphenylphosphino)ferrocene-palladium(II) dichloridedichloromethane complex (41 mg, 0.05 mmol) was added thereto, followed by stirring at 60°C for 1 hour. After finishing the reaction, the reaction solution was diluted with ethyl acetate, and the resultant was washed with water and a saturated aqueous sodium chloride solution. An organic layer was dried over anhydrous magnesium sulfate and filtered. The filtrate was concentrated under a reduced pressure, and the concentrate under a reduced pressure was purified by column chromatography (dichloromethane/methanol = 19/1 by a weight ratio) to obtain tert-butyl 4-(2-((3-chloro-5-(7,7-difluoro-2-(methylsulfonyl)-6,7-dihydro-5H-cyclopenta[d]pyrimidin-4-yl)pyridin-2-yl)oxy)acetyl)piperazin-1-carboxylate (560 mg, 0.95 mmol, yield 98%). The synthesis of the tert-butyl 4-(2-((3-chloro-5-(7,7-difluoro-2-(methylsulfonyl)-6,7-dihydro-5H-cyclopenta[d]pyrimidin-4-yl)pyridin-2-yl)oxy)acetyl)piperazin-1-carboxylate thus obtained was confirmed through mass spectrometry (MS) measurement.
MS 588.0, 610.0 (Na⁺)

### (2) Step B: Preparation of tert-butyl (S)-4-(2-((3-chloro-5-(7,7-difluoro-2-(2-methylazetidin-1-yl)-6,7-dihydro-5H-cyclopenta[d]pyrimidin-4-yl)pyridin-2-yl)oxy)acetyl)piperazin-1-carboxylate

The tert-butyl 4-(2-((3-chloro-5-(7,7-difluoro-2-(methylsulfonyl)-6,7-dihydro-5H-cyclopenta[d]pyrimidin-4-yl)pyridin-2-yl)oxy)acetyl)piperazin-1-carboxylate (560 mg, 0.95 mmol) prepared in Step A, (2S)-2-methylazetidine[(1R,4S)-7,7-dimethyl-2-oxo-norbornan-1-yl]methanesulfonate (490 mg, 1.62 mmol), and potassium carbonate (525 mg, 3.80 mmol) were dissolved in 20 ml of acetonitrile, followed by stirring at 80°C for 1 hour. After finishing the reaction, the reaction solution was concentrated under a reduced pressure. To the concentrate under a reduced pressure, ethyl acetate was added, and the resultant was washed with a saturated aqueous sodium chloride solution. An organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl (S)-4-(2-((3-chloro-5-(7,7-difluoro-2-(2-methylazetidin-1-yl)-6,7-dihydro-5H-cyclopenta[d]pyrimidin-4-yl)pyridin-2-yl)oxy)acetyl)piperazin-1-carboxylate (299 mg, 0.51 mmol, yield 54%). The synthesis of the tert-butyl (S)-4-(2-((3-chloro-5-(7,7-difluoro-2-(2-methylazetidin-1-yl)-6,7-dihydro-5H-cyclopenta[d]pyrimidin-4-yl)pyridin-2-yl)oxy)acetyl)piperazin-1-carboxylate thus obtained was confirmed through ¹H NMR analysis.

¹H NMR (500MHz, CDCl₃) δ 8.55 (s, 1H), 8.32 (s, 1H), 5.17 (s, 2H), 4.59 (td, J=13.4, 6.7 Hz, 1H), 4.18-4.00 (m, 2H), 3.70-3.37 (m, 8H), 3.09 (d, J=3.1 Hz, 2H), 2.68-2.40 (m, 3H), 1.53-1.41 (m, 9H).

### (3) Step C: Preparation of (S)-2-((3-chloro-5- (7,7-difluoro-2-(2-methylazetidin-1-yl)-6,7-dihydro-5H-cyclopenta[d]pyrimidin-4-yl)pyridin-2-yl)oxy)-1-(piperazin-1-yl)ethan-1-one

The tert-butyl (S)-4-(2-((3-chloro-5-(7,7-difluoro-2-(2-methylazetidin-1-yl)-6,7-dihydro-5H-cyclopenta[d]pyrimidin-4-yl)pyridin-2-yl)oxy)acetyl)piperazin-1-carboxylate (299 mg, 0.52 mmol) prepared in Step B and 2 ml of trifluoroacetic acid were dissolved in 20 ml of dichloromethane, followed by stirring at room temperature for 18 hours. After finishing the reaction, the reaction solution was concentrated under a reduced pressure. To the concentrate under a reduced pressure, a saturated aqueous ammonium solution was added, and the resultant was extracted with ethyl acetate. An organic layer was dried over anhydrous magnesium sulfate and filtered. The filtrate was concentrated under a reduced pressure, and the concentrate under a reduced pressure was purified by column chromatography (7 N ammonia methanol solution/dichloromethane = 1/10 by a weight ratio) to obtain (S)-2-((3-chloro-5-(7,7-difluoro-2-(2-methylazetidin-1-yl)-6,7-dihydro-5H-cyclopenta[d]pyrimidin-4-yl)pyridin-2-yl)oxy)-1-(piperazin-1-yl)ethan-1-one (214 mg, 0.44 mmol, yield 86%). The synthesis of the (S)-2-((3-chloro-5-(7,7-difluoro-2-(2-methylazetidin-1-yl)-6,7-dihydro-5H-cyclopenta[d]pyrimidin-4-yl)pyridin-2-yl)oxy)-1-(piperazin-1-yl)ethan-1-one thus obtained was confirmed through ¹H NMR analysis.

¹H NMR (500MHz, MeOD) δ 8.62 (s, 1H), 8.41 (d, J=1.8 Hz, 1H), 5.30-5.19 (m, 2H), 4.58 (q, J=6.7 Hz, 1H), 4.21 - 3.98 (m, 2H), 3.56 (d, J=4.3 Hz, 4H), 3.14 (d, J=3.1 Hz, 2H), 2.97-2.74 (m, 4H), 2.68-2.44 (m, 3H), 2.20-1.98 (m, 1H), 1.57 (d, J=6.1 Hz, 3H).

### Example 39: Preparation of (S)-2-((5-(2-(2-methylazetidin-1-yl)-6-(trifluoromethyl)pyrimidin-4-yl)pyridin-2-yl)oxy)-1-(piperazin-1-yl)ethan-1-one

As in Reaction 33, the processes of Steps A to C were performed in sequence to prepare the title compound of Formula 1-39.

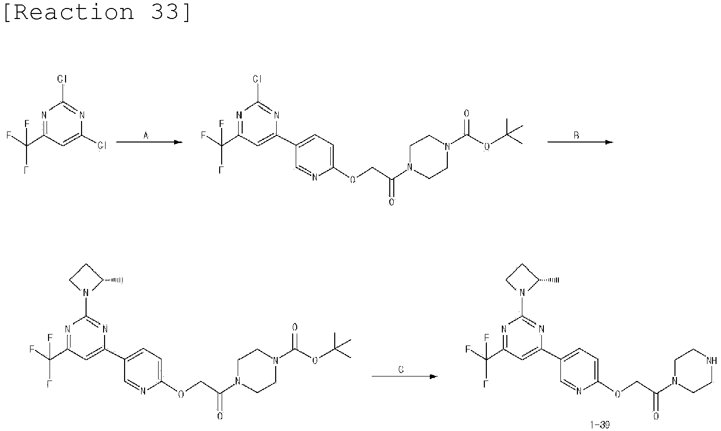

### (1) Step A: Preparation of tert-butyl 4-(2-((5-(2-chloro-6-(trifluoromethyl)pyrimidin-4-yl)pyridin-2-yl)oxy)acetyl)piperazin-1-carboxylate

2,4-Dichloro-6-(trifluoromethyl)pyrimidine (456 mg, 2.10 mmol), the tert-butyl 4-(2-((5- (4,4,5,5-tetramethyl-1,3,2-dioxaborolan-2-yl)pyridin-2-yl)oxy)acetyl)piperazin-1-carboxylate (1128 mg, 2.52 mmol) prepared in Preparation Example 17, and sodium carbonate (2 M, 3.15 ml, 6.30 mmol) were dissolved in 20 ml of 1,4-dioxane, followed by degassing for 5 minutes. 1,1'-bis(diphenylphosphino)ferrocene-palladium(II) dichloride (44 mg, 0.063 mmol) was added thereto, followed by stirring at 60°C for 1 hour. After finishing the reaction, the reaction solution was diluted in ethyl acetate, and the resultant was washed with water and a saturated aqueous sodium chloride solution. An organic layer was dried over anhydrous magnesium sulfate and filtered. The filtrate was concentrated under a reduced pressure to obtain tert-butyl 4-(2-((5-(2-chloro-6-(trifluoromethyl)pyrimidin-4-yl)pyridin-2-yl)oxy)acetyl)piperazin-1-carboxylate (976 mg, 1.945 mmol, crude), and the next reaction was performed without a separate purification process.

### (2) Step B: Preparation of tert-butyl (S)-4-(2-((5-(2-(2-methylazetidin-1-yl)-6-(trifluoromethyl)pyrimidin-4-yl)pyridin-2-yl)oxy)acetyl)piperazin-1-carboxylate

The tert-butyl 4-(2-((5-(2-chloro-6-(trifluoromethyl)pyrimidin-4-yl)pyridin-2-yl)oxy)acetyl)piperazin-1-carboxylate (976 mg, 1.945 mmol) prepared in Step A, (2S)-2-methylazetidine[(1R,4S)-7,7-dimethyl-2-oxo-norbornan-1-yl]methanesulfonate (708 mg, 2.334 mmol), and potassium carbonate (274 mg, 1.98 mmol) were dissolved in 20 ml of acetonitrile, followed by stirring at 80°C for 1 hour. After finishing the reaction, the reaction solution was concentrated under a reduced pressure. To the concentrate under a reduced pressure, ethyl acetate was added and washed with a saturated aqueous sodium chloride solution. An organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl (S)-4-(2-((5-(2-(2-methylazetidin-1-yl)-6-(trifluoromethyl)pyrimidin-4-yl)pyridin-2-yl)oxy)acetyl)piperazin-1-carboxylate (558 mg, 1.041 mmol, yield 53%). The synthesis of the tert-butyl (S)-4-(2-((5-(2-(2-methylazetidin-1-yl)-6-(trifluoromethyl)pyrimidin-4-yl)pyridin-2-yl)oxy)acetyl)piperazin-1-carboxylate thus obtained was confirmed through ¹H NMR analysis.

¹H NMR (500MHz, CDCl₃) δ 8.84 (s, 1H), 8.31 (d, J=8.8 Hz, 1H), 7.13 (s, 1H), 7.01 (d, J=8.5 Hz, 1H), 5.12 (s, 2H), 4.62 (q, J=6.6 Hz, 1H), 4.10-4.19 (m, 2H), 3.47-3.64 (m, 8H), 2.52 (m, 1H), 2.06 (m, 1H), 1.60 (d, J=7.6 Hz, 3H), 1.49 (s, 9H) .

### (3) Step C: Preparation of (S)-2-((5-2-(2-methylazetidin-1-yl)-6-(trifluoromethyl) pyrimidin-4-yl)pyridin-2-yl)oxy)-1-(piperazin-1-yl)ethan-1-one

The tert-butyl (S)-4-(2-((5-(2-(2-methylazetidin-1-yl)-6-(trifluoromethyl)pyrimidin-4-yl)pyridin-2-yl)oxy)acetyl)piperazin-1-carboxylate (558 mg, 1.040 mmol) prepared in Step B and 2 ml of trifluoroacetic acid were dissolved in 20 ml of dichloromethane, followed by stirring at room temperature for 18 hours. After finishing the reaction, the reaction solution was concentrated under a reduced pressure. To the concentrate under a reduced pressure, a saturated aqueous sodium bicarbonate solution was added, and the resultant was extracted with ethyl acetate. An organic layer was dried over anhydrous magnesium sulfate and filtered. The filtrate was concentrated under a reduced pressure, and the concentrate under a reduced pressure was purified by column chromatography (7 N ammonia methanol solution/dichloromethane = 1/10 by a weight ratio) to obtain (S)-2-((5-2-(2-methylazetidin-1-yl)-6-(trifluoromethyl)pyrimidin-4-yl)pyridin-2-yl)oxy)-1-(piperazin-1-yl)ethan-1-one (230 mg, 0.528 mmol, yield 50%). The synthesis of the (S)-2-((5-2-(2-methylazetidin-1-yl)-6-(trifluoromethyl)pyrimidin-4-yl)pyridin-2-yl)oxy)-1-(piperazin-1-yl)ethan-1-one thus obtained was confirmed through ¹H NMR analysis.

¹H NMR (500MHz, CDCl₃) δ 8.84 (s, 1H), 8.30 (d, J=8.5 Hz, 1H), 7.13 (s, 1H), 7.02 (d, J=8.5 Hz, 1H), 5.12 (s, 2H), 4.62 (q, J=6.6 Hz, 1H), 4.08-4.21 (m, 2H), 3.65 (s, 2H), 3.53 (s, 2H), 2.93 (d, J = 24 . 7 Hz, 4H), 2.53 (dd, J=8.5, 5.5 Hz, 1H), 2.03-2.08 (m, 1H), 1.61 (d, J=5.8 Hz, 3H).

### Example 40: Preparation of (S)-2-((5-(5-methyl-2-(2-methylazetidin-1-yl)-6-(trifluoromethyl)pyrimidin-4-yl)pyridin-2-yl)oxy)-1-(piperazin-1-yl)ethan-1-one

As in Reaction 34, the processes of Steps A to C were performed in sequence to prepare the title compound of Formula 1-40.

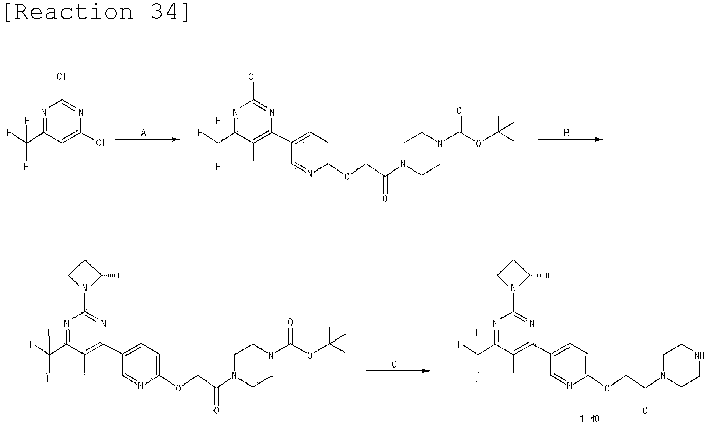

### (1) Step A: Preparation of tert-butyl 4-(2-((5-(2-chloro-5-methyl-6-(trifluoromethyl)pyrimidin-4-yl)pyridin-2-yl)oxy)acetyl)piperazin-1-carboxylate

2,4-Dichloro-5-methyl-6-(trifluoromethyl)pyrimidine (253 mg, 1.095 mmol), the tert-butyl 4-(2-((5- (4,4,5,5-tetramethyl-1,3,2-dioxaborolan-2-yl)pyridin-2-yl)oxy)acetyl)piperazin-1-carboxylate (588 mg, 1.314 mmol) prepared in Preparation Example 17, and sodium carbonate (2 M, 1.64 ml, 3.29 mmol) were dissolved in 20 ml of 1,4-dioxane, followed by degassing for 5 minutes. Bis(triphenylphosphine) palladium(II) (23 mg, 0.033 mmol) was added thereto, followed by stirring at 60°C for 1 hour. After finishing the reaction, the reaction solution was diluted in ethyl acetate, and the resultant was washed with water and a saturated aqueous sodium chloride solution. An organic layer was dried over anhydrous magnesium sulfate and filtered. The filtrate was concentrated under a reduced pressure, and the concentrate under a reduced pressure was purified by column chromatography (dichloromethane/methanol=2/1 by a weight ratio) to obtain tert-butyl 4-(2-((5-(2-chloro-5-methyl-6-(trifluoromethyl)pyrimidin-4-yl)pyridin-2-yl)oxy)acetyl)piperazin-1-carboxylate (275 mg, 0.533 mmol, yield 48%). The synthesis of the tert-butyl 4-(2-((5-(2-chloro-5-methyl-6-(trifluoromethyl)pyrimidin-4-yl)pyridin-2-yl)oxy)acetyl)piperazin-1-carboxylate thus obtained was confirmed through ¹H NMR analysis.

¹H NMR (400MHz, CDCl₃) δ 8.39 (d, J=1.8 HZ, 1H), 7.91 (dd, J=8.7, 2.3 Hz, 1H), 7.03 (d, J=8.7 Hz, 1H), 5.09 (s, 2H), 3.44-3.60 (m, 8H), 2.50 (d, J=1.8 Hz, 3H), 1.46 (s, 9H).

### (2) Step B: Preparation of tert-butyl (S)-4-(2-((5-(5-methyl-2-(2-methylazetidin-1-yl)-6-(trifluoromethyl)pyrimidin-4-yl)pyridin-2-yl)oxy)acetyl)piperazin-1-carboxylate

The tert-butyl 4-(2-((5-(2-chloro-5-methyl-6-(trifluoromethyl)pyrimidin-4-yl)pyridin-2-yl)oxy)acetyl)piperazin-1-carboxylate (0.533 mmol) prepared in Step A, (2S)-2-methylazetidine[(1R,4S)-7,7-dimethyl-2-oxo-norbornan--1-yl]methanesulfonate (0.64 mmol), and potassium carbonate (221 mg, 1.60 mmol) were dissolved in 20 ml of acetonitrile, followed by stirring at 80°C for 1 hour. After finishing the reaction, the reaction solution was concentrated under a reduced pressure. To the concentrate under a reduced pressure, ethyl acetate was added, and the resultant was washed with a saturated aqueous sodium chloride solution. An organic layer was dried over anhydrous magnesium sulfate and filtered. The filtrate was concentrated under a reduced pressure, and the concentrate under a reduced pressure was purified by column chromatography (ethyl acetate/hexane = 1/1 by a weight ratio) to obtain tert-butyl (S)-4-(2-((5-(5-methyl-2-(2-methylazetidin-1-yl)-6-(trifluoromethyl)pyrimidin-4-yl)pyridin-2-yl)oxy)acetyl)piperazin-1-carboxylate (232 mg, 0.42 mmol, yield 79%). The synthesis of the tert-butyl (S)-4-(2-((5-(5-methyl-2-(2-methylazetidin-1-yl)-6-(trifluoromethyl)pyrimidin-4-yl)pyridin-2-yl)oxy)acetyl)piperazin-1-carboxylate thus obtained was confirmed through ¹H NMR analysis.

¹H NMR (400MHz, CDCl₃) δ 8.32 (d, J=2.7 Hz, 1H), 7.82 (dd, J=8.5, 2.5 Hz, 1H), 6.95-6.98 (m, 1H), 5.07 (s, 2H), 4.51 (m, 1H), 3.98-4.12 (m, 2H), 3.56 (m, 8H), 2.50-2.34 (1H), 2.02 (m, 2H), 1.47 (s, 9H).

### (3) Step C: Preparation of (S)-2-((5-(5-methyl-2-(2-methylazetidin-1-yl)-6-(trifluoromethyl)pyrimidin-4-yl)pyridin-2-yl)oxy)-1-(piperazin-1-yl)ethan-1-one

The tert-butyl (S)-4-(2-((5-(5-methyl-2-(2-methylazetidin-1-yl)-6-(trifluoromethyl)pyrimidin-4-yl)pyridin-2-yl)oxy)acetyl)piperazin-1-carboxylate (0.60 mmol) prepared in Step B and 2 ml of trifluoroacetic acid were dissolved in 20 ml of dichloromethane, followed by stirring at room temperature for 18 hours. After finishing the reaction, the reaction solution was concentrated under a reduced pressure. To the concentrate under a reduced pressure, a saturated aqueous ammonium solution was added, and the resultant was extracted with ethyl acetate. An organic layer was dried over anhydrous magnesium sulfate and filtered. The filtrate was concentrated under a reduced pressure, and the concentrate under a reduced pressure was purified by column chromatography (7 N ammonia methanol solution/dichloromethane = 1/10 by a weight ratio) to obtain (S)-2-((5-(5-methyl-2-(2-methylazetidin-1-yl)-6-(trifluoromethyl)pyrimidin-4-yl)pyridin-2-yl)oxy)-1-(piperazin-1-yl)ethan-1-one (95.4 mg, 0.212 mmol, yield 50%). The synthesis of the (S)-2-((5-(5-methyl-2-(2-methylazetidin-1-yl)-6-(trifluoromethyl)pyrimidin-4-yl)pyridin-2-yl)oxy)-1-(piperazin-1-yl)ethan-1-one thus obtained was confirmed through ¹H NMR analysis.

¹H NMR (400MHz, CDCl₃) δ 8.32 (d, J=2.3 Hz, 1H), 7.81 (dd, J=8.7, 2.3 Hz, 1H), 6.97 (d, J=8.2 Hz, 1H), 5.07 (s, 2H), 4.50 (q, J=6.9 Hz, 1H), 3.96-4.10 (m, 2H), 3.55 (m, 4H), 2.89 (m, 4H), 2.39-2.48 (m, 1H), 2.25 (d, J=1.8 Hz, 3H), 1.96-2.06 (m, 1H), 1.52 (d, J=6.4 Hz, 3H).

### Experimental Example: measurement of activity in vitro of ketohexokinase inhibitor

Ketohexokinase (KHK) was expressed in E. Coli and purified using His tag. In order to measure the activity of the purified KHK, Transcreener ADP2 TR-FRET Red Assay kit of BellBrook Labs was used. A method of measuring the amount of ADP produced was used using TR-FRET after reacting a solution including a suitable concentration of KHK protein and ATP, and fructose for 15 minutes. In order to see the activity of a ketohexokinase (KHK) inhibitor, ketohexokinase (KHK) and a suitable concentration of an inhibitor were reacted first for 30 minutes, and then, reacted with a solution including the substrate of ketohexokinase (KHK) for 15 minutes. Then, reaction for TR-FRET was performed for 1 hour, and fluorescence was measured using Envision equipment of PerkinElmer. According to the optimization procedure of TR-FRET published by BellBrook Lab, the set value of the Envision equipment was determined.

The result values by concentration of the ketohexokinase (KHK) inhibitor were determined as the ratio of a wavelength of 665 nm and a wavelength of 615nm. The 665/615 ratio value by concentration of each inhibitor was converted to ADP concentration based on ADP standard curve, and inhibition activity Ki value was obtained using Morrison equation by statistical software (Prizm). Meanwhile, the Ki value is a value representing the binding affinity of the inhibitor and an enzyme, and a lower value means better inhibitory activity.

**[Table 1]**

| Divi sion | Example | | | | | | | | | | | | | | | | |
|---|---|---|---|---|---|---|---|---|---|---|---|---|---|---|---|---|---|
| | 1 | 2 | 3 | | 4 | 5 | 6 | 7 | 8 | | 9 | | 10 | 11 | 12 | 13 | 14 |
| Ki | C | D | D | | C | C | B | D | D | | D | | D | D | C | D | C |

| Divi sion | Example | | | | | | | | | | | | | | | | |
|---|---|---|---|---|---|---|---|---|---|---|---|---|---|---|---|---|---|
| | 15 | 16 | 17 | | 18 | 19 | 20 | 21 | 22 | | 23 | | 24 | 25 | 26 | 27 | 28 |
| Ki | D | D | D | | D | D | D | D | B | | C | | C | D | D | C | C |

| Divi sion | Example | | | | | | | | | | | | | | | | |
|---|---|---|---|---|---|---|---|---|---|---|---|---|---|---|---|---|---|
| | 29 | 30 | | 31 | | 32 | 33 | 34 | | 35 | | 36 | | 37 | 38 | 39 | 40 |
| Ki | C | D | | D | | C | C | D | | D | | D | | D | D | D | D |

In Table 1, A ≥ 1 µM, B = 1-0.1 µM, C = 0.1-0.01 µM, and D ≤ 0.01 µM. Through the results of Table 1, it could be confirmed that the compounds of Example 1 to Example 40 according to the present invention have excellent inhibitory activity with respect to ketohexokinase.

## Claims

1. A compound of the following Formula 1, a pharmaceutically acceptable salt thereof, or an isomer thereof: 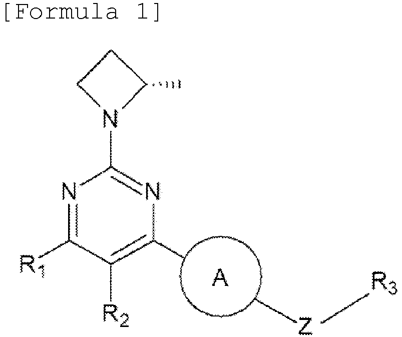 in Formula 1,
A is a C₃-C₈ heterocycloalkyl group, unsubstituted or substituted with a C₁-C₃ alkyl group or a carbonyl group, or 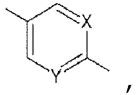
X and Y are each independently N or C-Rₐ,
Rₐ is hydrogen or a halogen group,
Z is a direct linkage, a C₁-C₃ alkylene group, -O-, or - C(O)-,
R₁ and R₂ are each independently hydrogen, a halogen group, or a C₁-C₃ alkyl group, unsubstituted or substituted with 1 to 5 halogen groups, or R₁ and R₂ are connected with each other to form a C₃-C₆ aliphatic cyclic group, unsubstituted or substituted with 1 to 3 halogen groups, or a C₃-C₆ heterocycloalkyl group, unsubstituted or substituted with 1 to 3 halogen groups,
R₃ is -(CR₄R₅)ₙ-B-R₆,
R₄ and R₅ are each independently hydrogen or a C₁-C₃ alkyl group,
n is an integer of 0 to 3,
B is a direct linkage, -C(O)-, or -S(O)₂-, and
R₆ is a hydroxyl group, or a C₃-C₇ heterocycloalkyl group containing N, unsubstituted or substituted with a C₁-C₃ alkyl group, a carbonyl group or a C₁-C₃ alkylcarbonyl group.

2. The compound, the pharmaceutically acceptable salt thereof, or the isomer thereof according to claim 1, wherein, in the Formula 1, in case where A is the heterocycloalkyl group, A is a substituent represented by the following Formula 1a or Formula 1b: 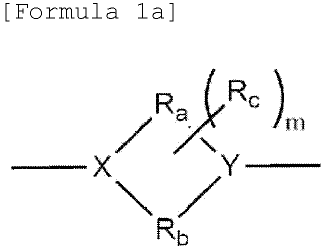 in Formula 1a,
X and Y are each independently CH or N,
Rₐ and R_{b} are each independently a C₁-C₃ alkylene group,
R_{c} is a C₁-C₃ alkyl group or a carbonyl group, and
m is 0 or 1, 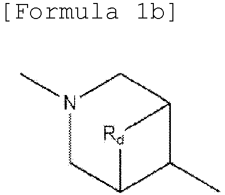 in Formula 1b,
R_{d} is a direct linkage or CH₂.

3. The compound, the pharmaceutically acceptable salt thereof, or the isomer thereof according to claim 1, wherein, in the Formula 1, in case where A is the 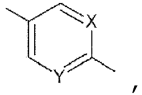
X and Y are each independently N or C-Rₐ,
Rₐ is hydrogen or a halogen group,
Z is a C₁-C₃ alkylene group or -O-,
R₁ and R₂ are connected with each other to form a C₃-C₆ aliphatic cyclic group, unsubstituted or substituted with 1 to 3 halogen group,
R₃ is - (CR₄R₅)ₙ-A-R₆,
R₄ and R₅ are hydrogen,
n is an integer of 0 to 3,
A is -C(O)-, and
R₆ is a hydroxyl group, or a C₄-C₆ heterocycloalkyl group containing N, unsubstituted or substituted with a C₁-C₃ alkyl group.

4. The compound, the pharmaceutically acceptable salt thereof, or the isomer thereof according to claim 1, wherein, in the Formula 1, in case where A is the 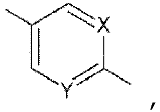
X is N,
Y is C-Rₐ, where Rₐ is hydrogen,
Z is a C₁-C₃ alkylene group or -O-,
R₁ is a C₁-C₃ alkyl group substituted with 1 to 3 halogen groups,
R₂ is hydrogen or a C₁-C₃ alkyl group,
R₃ is -(CR₄R₅)ₙ-A-R₆,
R₄ and R₅ are hydrogen,
n is an integer of 0 to 3,
A is -C(O)-, and
R₆ is a C₄-C₆ heterocycloalkyl group containing N, unsubstituted or substituted with a C₁-C₃ alkyl group.

5. The compound, the pharmaceutically acceptable salt thereof, or the isomer thereof according to claim 1, wherein the halogen group is F or Cl.

6. The compound, the pharmaceutically acceptable salt thereof, or the isomer thereof according to claim 1, wherein the compound of Formula 1 is one or more selected from the group consisting of the following group:
(S)-2-((1-(2-(2-methylazetidin-2-yl)-6-(trifluoromethyl)pyrimidin-4-yl)azetidin-3-yl)oxy)-1-(piperazin-1-yl)ethan-1-one;
(S)-2-((1-(5-chloro-2-(2-methylazetidin-1-yl)-6-(trifluoromethyl)pyrimidin-4-yl)azetidin-3-yl)oxy)-1-(piperazin-1-yl)ethyn-1-one;
(S)-2-((1-(7,7-difluoro-2-(2-(methylazetidin-1-yl)-6,7-dihydro-5H-cyclopenta[d]pyrimidin-4-yl)azetidin-3-yl)oxy)-1-(piperazin-1-yl)ethan-1-one;
(S)-2-((1-(8,8-difluoro-2-(2-methylazetidin-1-yl)-5,6,7,8-tetrahydroquinazolin-4-yl)azetidin-3-yl)oxy)-1-(piperazin-1-yl)ethan-1-one;
2-(((2S,3R)-1-(7,7-difluoro-2-((S)-2-methylazetidin-1-yl)-6,7-dihydro-5H-cyclopenta[d]pyrimidin-4-yl)-2-methylazetidin-3-yl)oxy)-1-(piperazin-1-yl)ethan-1-one;
2-(((S)-1-(2-((S)-2-methylazetidin-1-yl)-6-(trifluoromethyl)pyrimidin-4-yl)pyrrolidin-3-yl)oxy)-1-(piperazin-1-yl)ethan-1-one;
2-(((S)-1-(7,7-difluoro-2-((S)-2-methylazetidin-1-yl)-6,7-dihydro-5H-cyclopenta[d]pyrimidin-4-yl)pyrrolidin-3-yl)oxy)-1-(piperazin-1-yl)ethan-1one;
2-(((R)-1-(7,7-difluoro-2-((S)-2-methylazetidin-1-yl)-6,7-dihydro-5H-cyclopenta[d]pyrimidin-4-yl)pyrrolidin-3-yl)oxy)-1-(piperazin-1-yl)ethan-1one;
2-((R)-1-(7,7-difluoro-2-((S)-2-methylazetidin-1-yl)-6,7-dihydro-5H-cyclopenta[d]pyrimidin-4-yl)pyrrolidin-3-yl)-1-(piperazin-1-yl)ethan-1one;
(S)-2-((1-(7,7-difluoro-2-(2-methylazetidin-1-yl)-6,7-dihydro-5H-cyclopenta[d]pyrimidin-4-yl)piperizin-4-yl)oxy)-1-(piperazin-1-yl)ethan-1-one;
(S)-2-(4-(2-(2-methylazetidin-1-yl)-6-(trifluoromethyl)pyrimidin-4-yl)piperazin-1-yl)-1-(piperazin-1-yl)ethan-1-one;
(S)-2-(4-(5-chloro-2-(2-methylazetidin-1-yl)-6-(trifluoromethyl)pyrimidin-4-yl)piperazin-1-yl)-1-(piperazin-1-yl)ethan-1-one;
(S)-2-(4-(2-(2-methylazetidin-1-yl)-6-(trifluoromethyl)pyrimidin-4-yl)-1-(2-oxo-2-(piperazin-1-yl)ethyl)piperazin-2-one;
(S)-2-(4-(7,7-difluoro-2-(2-methylazetidin-1-yl)-6,7-dihydro-5H-cyclopenta[d]pyrimidin-4-yl)piperazin-1-yl)-1-(piperazin-1-yl)ethan-1-one;
(S)-2-(4-(7,7-difluoro-2-(2-methylazetidin-1-yl)-6,7-dihydro-5H-cyclopenta[d]pyrimidin-4-yl)piperazin-1-yl)-1-morpholinoethan-1-one;
(S)-1-(4-(7,7-difluoro-2-(2-methylazetidin-1-yl)-6,7-dihydro-5H-cyclopenta[d]pyrimidin-4-yl)piperazin-1-yl)-3-(piperazin-1-yl)propan-1,3-dione;
(S)-(4-(7,7-difluoro-2-(2-methylazetidin-1-yl)-6,7-dihydro-5H-cyclopenta[d]pyrimidin-4-yl)-1-(2-oxo-2-(piperazin-1-yl)ethyl)piperazin-2-one;
(S)-2(4-(7,7-difluoro-2-(2-methylazetidin-1-yl)-6,7-dihydro-5H-cyclopenta[d]-pyrimidin-4-yl)piperidin-1-yl)-1-(piperazin-1-yl)ethan-1-one;
2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)-1-(piperazin-1-yl)ethan-1-one;
2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)-1-(4-methylpiperazin-1-yl)ethan-1-one;
4-(2-((1R,5S,6R)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)acetyl)piperazin-2-one;
2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)-1-(piperazin-1-yl)ethan-1-one;
1-(4-acetylpiperazin-1-yl)-2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)ethan-1-one;
2-((1R,5S,6S)-3-(8,8-difluoro-2-((S)-2-methylazetidin-1-yl)-5,6,7,8-tetrahydroquinazol-4-yl)-3-azabicyclo[3.1.1]heptan-6-yl)-1-(piperazin-1-yl)ethan-1-one;
2-((1R,5S,6S)-3-(8,8-difluoro-2-((S)-2-methylazetidin-1-yl)-5,8-dihydro-6H-pyrazol[3,4-d]pyrimidin-4-yl)-3-azabicyclo[3.1.1]heptan-6-yl)-1-(piperazin-1-yl)ethan-1-one;
7,7-difluoro-2-((S)-2-methylazetidin-1-yl)-4-((1R,5S,6S)-6-((piperazin-1-ylsulfonyl)methyl)-3-azabicyclo[3.1.0]hexan-3-yl)-6,7-dihydro-5H-cyclopenta[d]pyrimidine;
(S)-2-((1-(5-methyl-2-(2-methylazetidin-1-yl)-6-(trifluoro)pyrimidin-4-yl)azetidin-3-yl)oxy)-1-(piperazin-1-yl)ethan-1-one;
(S)-2-((1-(2-(2-methylazetidin-1-yl)-6-(trifluoromethyl)pyrimidin-4-yl)piperidin-4-yl)oxy)-1-(piperazin-1-yl)ethan-1-one;
(S)-2-((1-(5-methyl-2-(2-methylazetidin-1-yl)-6-(trifluoro)pyrimidin-4-yl)piperidin-4-yl)oxy)-1-(piperazin-1-yl)ethan-1-one;
(S)-2-(4-(7,7-difluoro-2-(2-methylazetidin-1-yl)-6,7-dihydro-5H-cyclopenta[d]-pyrimidin-4-yl)phenyl)acetic acid;
(S)-2-(4-(7,7-difluoro-2-(2-methylazetidin-1-yl)-6,7-dihydro-5H-cyclopenta[d]-pyrimidin-4-yl)phenyl)-1-(piperazin-1-yl)ethan-1-one;
(S)-2-(5-(2-(2-methylazetidin-1-yl)-6-(trifluoromethyl)pyrimidin-4-yl)pyridin-2-yl)-1-(piperazin-1-yl)ethan-1-one;
(S)-2-(4-(7,7-difluoro-2-(2-methylazetidin-1-yl)-6,7-dihydro-5H-cyclopenta[d]pyrimidin-4-yl)phenoxy)-1-(piperazin-1-yl)ethan-1-one;
(S)-2-(4-(7,7-difluoro-2-(2-methylazetidin-1-yl)-6,7-dihydro-5H-cyclopenta[d]pyrimidin-4-yl)-2-fluorophenoxy)-1-(piperazin-1-yl)ethan-1-one;
(S)-2-(4-(7,7-difluoro-2-(2-methylazetidin-1-yl)-6,7-dihydro-5H-cyclopenta[d]pyrimidin-4-yl)-2,6-difluorophenoxy)-1-(piperazin-1-yl)ethan-1-one;
(S)-2-(2-chloro-4-(7,7-difluoro-2-(2-methylazetidin-1-yl)-6,7-dihydro-5H-cyclopenta[d]pyrimidin-4-yl)phenoxy)-1-(piperazin-1-yl)ethan-1-one;
(S)-2-(2-chloro-4-(7,7-difluoro-2-(2-methylazetidin-1-yl)-6,7-dihydro-5H-cyclopenta[d]pyrimidin-4-yl)phenoxy)-1-(1,4-diazepan-1-yl)ethan-1-one;
(S)-2-((5-(7,7-difluoro-2-(2-methylazetidin-1-yl)-6,7-dihydro-5H-cyclopenta[d]pyrimidin-4-yl)pyridin-2-yl)oxy)-1-(piperazin-1-yl)ethan-1-one;
(S)-2-((3-chloro-5-(7,7-difluoro-2-(2-methylazetidin-1-yl)-6,7-dihydro-5H-cyclopenta[d]pyrimidin-4-yl)pyridin-2-yl)oxy)-1-(piperazin-1-yl)ethan-1-one;
(S)-2-((5-(2-(2-methylazetidin-1-yl)-6-(trifluoromethyl)pyrimidin-4-yl)pyridin-2-yl)oxy)-1-(piperazin-1-yl)ethan-1-one; and
(S)-2-((5-(5-methyl-2-(2-methylazetidin-1-yl)-6-(trifluoromethyl)pyrimidin-4-yl)pyridin-2-yl)oxy)-1-(piperazin-1-yl)ethan-1-one.

7. The compound, the pharmaceutically acceptable salt thereof, or the isomer thereof according to claim 1, being a ketohexokinase (KHK) inhibitor.

8. The compound, the pharmaceutically acceptable salt thereof, or the isomer thereof according to claim 1, being used for preventing or treating metabolic diseases.

9. A pharmaceutical composition for inhibiting ketohexokinase (KHK), the composition comprising the compound, the pharmaceutically acceptable salt thereof, or the isomer thereof according to claim 1, as an active ingredient.

10. A pharmaceutical composition for preventing or treating metabolic diseases, the composition comprising the compound, the pharmaceutically acceptable salt thereof, or the isomer thereof according to claim 1, as an active ingredient.

11. The pharmaceutical composition for preventing or treating metabolic diseases according to claim 10, wherein the metabolic disease is diabetes, complications of diabetes, obesity, nonalcoholic steatohepatitis, or fatty hepatitis.

12. The pharmaceutical composition for preventing or treating metabolic diseases according to claim 10, wherein the metabolic disease is nonalcoholic steatohepatitis.

13. A method for preparing a pharmaceutical composition for preventing or treating diabetes, complications of diabetes, obesity, nonalcoholic steatohepatitis, or fatty hepatitis, the method comprising a step of mixing the compound, the pharmaceutically acceptable salt thereof, or the isomer thereof according to claim 1, as an active ingredient with a pharmaceutically acceptable carrier.

14. A method for treating a metabolic disease comprising nonalcoholic steatohepatitis in a mammal, the method comprising administering the compound, the pharmaceutically acceptable salt thereof, or the isomer thereof according to claim 1.
